# EUROPEAN PATENT SPECIFICATION

(11) **EP 1 349 836 B1**
(45) Date of publication and mention of the grant of the patent: **14.06.2006**
(21) Application number: 01987005.4
(22) Date of filing: 06.12.2001
(51) Int. Cl.: C07D 209/80, A61K 31/407, C07D 495/14, A61P 29/00

(54) **TETRACYCLIC DERIVATIVES AS SPLA2 INHIBITORS**
TETRACYCLISCHE DERIVATE ALS SPLA2 INHIBITOREN
NOUVEAUX INHIBITEURS DE SPLA2

(30) Priority: 18.12.2000 US 256395 P
(43) Date of publication of application: 08.10.2003
(73) Proprietor: ELI LILLY AND COMPANY, Indianapolis, Indiana 46285 (US)
(72) Inventor: BEIGHT, Douglas, Wade, Frankfort, IN 46041 (US); JANDZINSKI, John, David, Hopewell Junction, NY 12533 (US); KINNICK, Michael, Dean, Indianapolis, IN 46217 (US); LIN, Ho-Shen, Indianapolis, IN 46217 (US); MORIN, John, Michael, Junior, Brownsburg, IN 46112 (US); RICHETT, Michael, Enrico, Indianapolis, IN 46250 (US); SALL, Daniel, Jon, Greenwood, IN 46143 (US); SAWYER, Jason, Scott, Indianapolis, IN 46220 (US)
(74) Representative: Kingsbury, Oliver William
(86) International application number: PCT/US2001/043186
(87) International publication number: WO 2002/050029

(56) References cited:
- EP-A- 0 950 661
- WO-A-00/07590
- US-A- 5 523 297
- CHEMICAL ABSTRACTS, vol. 124, no. 23, 1996 Columbus, Ohio, US; abstract no. 317028x, page 1205; XP002202619 & J. CHEM. RES., SYNOP., no. 2, 1996, pages 84-5,

## Description

This invention relates to novel tetracyclic compounds useful for Inflammatory Diseases.

The structure and physical properties of human non-pancreatic secretory phospholipase A₂ (hereinafter called, "sPLA₂") has been thoroughly described in two articles, namely, "Cloning and Recombinant Expression of Phospholipase A₂ Present in Rheumatoid Arthritic Synovial Fluid" by Seilhamer, Jeffrey J.; Pruzanski, Waldemar; Vadas Peter; Plant, Shelley; Miller, Judy A.; Kloss, Jean; and Johnson, Lorin K.; The Journal of Biological Chemistry, Vol. 264, No. 10, Issue of April 5, pp. 5335-5338, 1989; and "Structure and Properties of a Human Non-pancreatic Phospholipase A₂" by Kramer, Ruth M.; Hession, Catherine; Johansen, Berit; Hayes, Gretchen; McGray, Paula; Chow, E. Pingchang; Tizard, Richard; and Pepinsky,
R. Blake; The Journal of Biological Chemistry, Vol. 264, No. 10, Issue of April 5, pp. 5768-5775, 1989; the disclosures of which are incorporated herein by reference.

It is believed that sPLA₂ is a rate limiting enzyme in the arachidonic acid cascade which hydrolyzes membrane phospholipids. Thus, it is important to develop compounds which inhibit sPLA₂ mediated release of fatty acids (e.g., arachidonic acid). Such compounds would be of value in the general treatment of conditions induced and/or maintained by overproduction of sPLA₂; such as sepsis or rheumatoid arthritis.

It is desirable to develop new compounds and treatments for sPLA₂ induced diseases.

The present invention relates to a compound of formula (I) and pharmaceutically acceptable salt, solvate or prodrug thereof, useful for the treatment or prevention of inflammatory diseases: 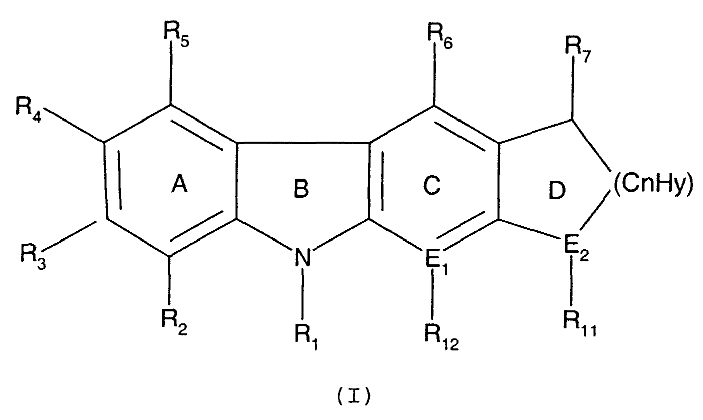 Wherein;
E₁ is C or N and E₂ is C, CH, N, O or S;
n is 1, 2 or 3 and y is an appropriate number of hydrogen atoms based on the value of (n) and also based on ring unsaturation;
the D ring has zero, single or multiple unsaturation as chemically possible based on ring size and conjugational or electronic effects;
R₁ is the group -(L)-R₈₀; where, -(L)- is a divalent linking group of 1 to 12 atoms selected from carbon, hydrogen, oxygen, nitrogen, and sulfur; wherein the combination of atoms in -(L)- are selected from the group consisting of (i) carbon and hydrogen only, (ii) sulfur only, (iii) oxygen only, (iv) nitrogen and hydrogen only, (v) carbon, hydrogen, and sulfur only, and (vi) and carbon, hydrogen, and oxygen only; and where R₈₀ is a substituted or unsubstituted group selected from the group consisting of (C₅₋C₁₄)cycloalkyl, (C₅-C₁₄)cycloalkenyl, phenyl, naphthyl, norbornanyl, bicycloheptadienyl, toluyl, xylenyl, indenyl, stilbenyl, terphenylyl, diphenylethylenyl, phenyl-cyclohexenyl, acenaphthylenyl, anthracenyl, biphenyl, bibenzylyl and related bibenzylyl homologues represented by the formula (a); 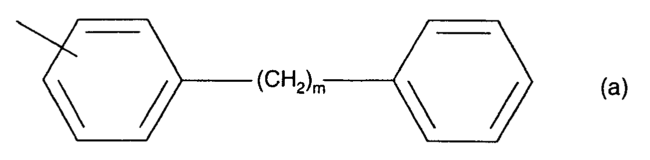 where m is a number from 1 to 8;
R₂ is hydrogen, methyl, ethyl, propyl, isopropyl, cyclopropyl, -F, -CF₃, -Cl, -Br, or -O-CH₃;
R₃, and R₄ are independently selected from the group consisting of hydrogen, (C₁-C₄)alkyl, (C₂₋C₄)alkenyl, -O-((C₁-C₄)alkyl), -S-((C₁-C₃)alkyl), -(C₃₋C₄)cycloalkyl, -CF₃, halo, -NO₂, -CN, and -SO₃;
R₅ is -(L₅)- Z, where -(L₅)- is a divalent linker group selected from a bond, or a divalent group selected from:

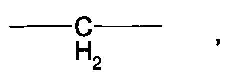

-O- ,

-S- ,

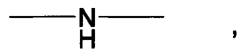

or 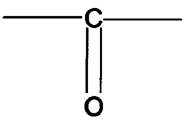 and Z is selected from an amide, thioamide, oxime amide, oxime thioamide, glyoxylamide, hydrazide, ureido or acetamide group represented by the formulae, 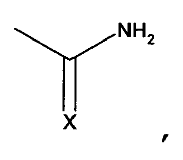 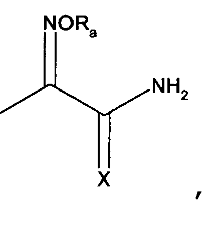 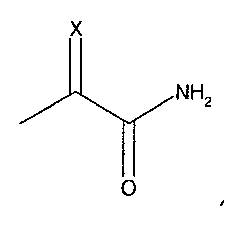 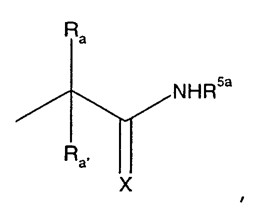 or 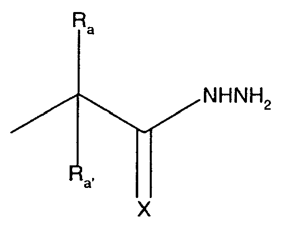 wherein X is oxygen or sulfur, Rₐ and R_{a'} are independently selected from hydrogen, (C₁-C₈)alkyl, aryl, (C₁-C₈)alkaryl, (C₁-C₈)alkoxy, aralkyl and -CN, and R^{5a} is hydrogen, methyl or ethyl;
R₆ is the group, hydrogen, CONH₂, CONHR^{6b}, -(La)-(acidic group), -(Lₕ)-(N-hydroxyfunctional amide group) or -(Lc)-(acylamino acid group)-, wherein;
-(Lₐ)-, -(Lₕ)-, or -(Lc)- is selected from a group represented by the formula; 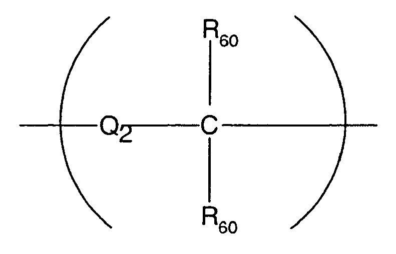 where Q₂ is selected from the group -(CH₂)-, -O-, -NR₆₀-, -C(O)-, and -S-, and each R₆₀ is independently selected from hydrogen, (C₁-C₈)alkyl, aryl, (C₁₋C₈)alkaryl, (C₁-C₈)alkoxy, aralkyl, and halo;
the (acidic group) is selected from CO₂H CO₂Na, CO₂K, COR^{6a}, SO₃H or P(O)(OH)₂;
a (N-hydroxyfunctional amide group) is represented by the formula: 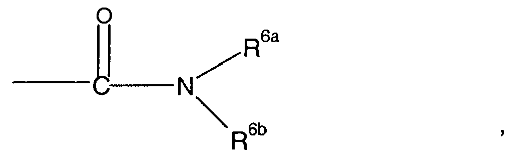 wherein R^{6a} is selected from the group consisting of OH, (C₁-C₆)alkoxy, and aryloxy; and wherein R^{6b} is hydrogen or an organic substituent selected from the group consisting of (C₁-C₈)alkyl, aryl, (C₇-C₁₄)aralkyl, (C₇₋C₁₄)alkaryl, (C₃-C₈)cycloalkyl, (C₁-C₈)alkoxyalkyl and these groups substituted with halogen, -CF₃, -OH, (C₁₋C₈)alkyl, amino, carbonyl, and -CN; and
the (acylamino acid group) is represented by the formula: 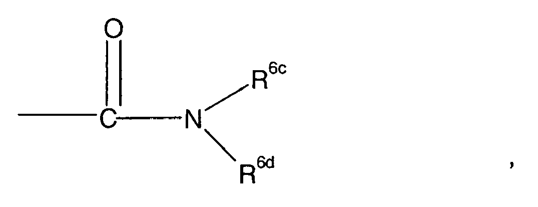 wherein R^{6c} is selected from the group consisting of H, (C₁-C₆)alkyl, (C₁-C₆)alkoxy, heteroaryl, aryl, and -CF₃; and wherein NR^{6d} is an amino acid residue of either a natural or unnatural amino acid with the nitrogen atom being part of the amino group of the amino acid; and wherein the amino acid residue is derived from an amino acid selected from the group comprising isoleucine, valine, phenylalanine, aspartic acid, leucine, glycine, asparagine, cysteine, glutamine, glutamic acid, histidine, lysine, methionine, serine, threonine, tryptophan, tyrosine and derivatives thereof;
R₇ is selected from hydrogen, a non-interfering substituent, the group -(Lₕ)-(N-hydroxyfunctional amide group) as previously defined, or the group -(L_{c})-acylamino acid group) as previously defined, or the group, -(Lₐ)-(acidic group) as previously defined;
and provided that at least one of R₆ or R₇ is the group -(Lₕ)-(N-hydroxyfunctional amide group), or the group -(L_{c})-acylamino acid group), or the group,-(Lₐ)-(acidic group);
R₁₁ and R₁₂ where applicable based on the identity of E₁ and E₂ are independently selected from hydrogen and non-interfering groups;
R₁₁ and R₁₂ are as described previously and are non-existent where the corresponding E₁ or E₂ is independently a nitrogen atom where the valency on nitrogen would be exceeded. When E₂ is oxygen R₁₁ is non-existent.

The present invention provides novel tetracyclic compounds of formula I having potent and selective effectiveness as inhibitors of mammalian sPLA₂.

The present invention also relates to the use of novel tetracyclic compounds of formula I useful in the treatment and/or prevention of Inflammatory Diseases.

This invention also relates to the use of a novel tetracyclic compound of formula I to inhibit mammalian sPLA₂ mediated release of fatty acids.

The present invention provides a pharmaceutical composition containing any of the tetracyclic compounds of the invention.

The present invention also relates to the use of a formulation comprising a compound of formula 1, and a carrier or diluent for the treatment or prevention of sepsis.

The present invention relates to the use of a pharmaceutical composition comprising a therapeutically effective amount of sPLA₂ inhibitor compounds of formula I and mixtures thereof for the manufacture of a medicament for the treatment of Inflammatory Diseases.

### I. Definitions:

The terms, "mammal" and "mammalian" include human and domesticated quadrupeds. The term, "Inflammatory Diseases" refers to diseases such as inflammatory bowel disease, sepsis, septic shock, adult respiratory distress syndrome, pancreatitis, trauma-induced shock, asthma, bronchial asthma, allergic rhinitis, rheumatoid arthritis, cystic fibrosis, stroke, acute bronchitis, chronic bronchitis, acute bronchiolitis, chronic bronchiolitis, osteoarthritis, gout, spondylarthropathris, ankylosing spondylitis, Reiter's syndrome, psoriatic arthropathy, enteropathric spondylitis, Juvenile arthropathy or juvenile ankylosing spondylitis, Reactive arthropathy, infectious or post-infectious arthritis, gonoccocal arthritis, tuberculous arthritis, viral arthritis, fungal arthritis, syphilitic arthritis, Lyme disease, arthritis associated with "vasculitic syndromes", polyarteritis nodosa, hypersensitivity vasculitis, Luegenec's granulomatosis, polymyalgin rheumatica, joint cell arteritis, calcium crystal deposition arthropathris, pseudo gout, non-articular rheumatism, bursitis, tenosynomitis, epicondylitis (tennis elbow), carpal tunnel syndrome, repetitive use injury (typing), miscellaneous forms of arthritis, neuropathic joint disease (charco and joint), hemarthrosis (hemarthrosic), Henoch-Schonlein Purpura, hypertrophic osteoarthropathy, multicentric reticulohistiocytosis, arthritis associated with certain diseases, surcoilosis, hemochromatosis, sickle cell disease and other hemoglobinopathries, hyperlipoproteineimia, hypogammaglobulinemia, hyperparathyroidism, acromegaly, familial Mediterranean fever, Behat's Disease, systemic lupus erythrematosis, or relapsing polychondritis and related diseases.

The term, "tetracyclic", or "tetracyclic nucleus" as used herein refers to a nucleus (having numbered positions) with the structural formula (X): 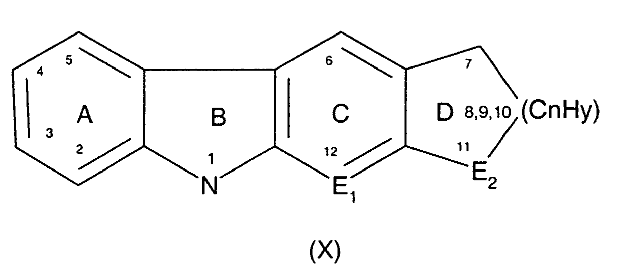 wherein, n is 1, 2 or 3, and y is the appropriate number of hydrogen atoms depending on D ring unsaturation;
wherein E₁ is C or N and E₂ is C, CH, N, O, or S with the appropriate number of hydrogen atoms appended;
and the letters A, B, C and D are ring identifiers; and
wherein, the D ring has zero, single or multiple unsaturation as chemically possible based on ring size and conjugational or electronic effects. The tetracyclic compounds of the invention employ certain defining terms as follows:

The term, "alkyl" by itself or as part of another substituent means, unless otherwise defined, a straight or branched chain monovalent hydrocarbon radical such as methyl, ethyl, n-propyl, isopropyl, n-butyl, tertiary butyl, sec-butyl, n-pentyl, and n-hexyl.

The term, "alkenyl" employed alone or in combination with other terms means a straight chain or branched monovalent hydrocarbon group having the stated number ranges of carbon atoms, and typified by groups such as vinyl, propenyl, crotonyl, isopentenyl, and various butenyl isomers.

The term, "hydrocarbyl" means an organic group containing only carbon and hydrogen.

The term, "halo" means fluoro, chloro, bromo, or iodo.

The terms, "non-interfering substituent", or "non-interfering groups" refer to (C₁-C₈)alkyl, (C₂-C₈)alkenyl, (C₂-C₈)alkynyl, (C₇-C₁₂)aralkyl, (C₇-C₁₂)alkaryl, (C₃-C₈)cycloalkyl, (C₃-C₈)cycloalkenyl, phenyl, toluyl, xylenyl, biphenyl, (C₁-C₈)alkoxy, (C₂-C₈)alkenyloxy, (C₂-C₈)alkynyloxy, (C₂-C₁₂) alkoxyalkyl, (C₂₋C₁₂)alkoxyalkyloxy, (C₂-C₁₂)alkylcarbonyl, and (C₂₋C₁₂)alkylcarbonylamino.

The term, "organic substituent" refers to a monovalent radical consisting of carbon and hydrogen with or without oxygen, nitrogen, sulfur, halogen, or other elements. Illustrative organic substituents are (C₁-C₈)alkyl, aryl, (C₇-C₁₄) aralkyl, (C₇-C₁₄)alkaryl, (C₃-C₈)cycloalkyl, (C₁-C₈)alkoxyalkyl and these groups substituted with halogen, -CF₃, -OH, (C₂-C₈)alkyl, amino, carbonyl, and -CN.

The term "substituted group" is an organic group substituted with one or more non-interfering substituents.

As used herein the terms "group", "radical" or "fragment" are synonymous and are intended to indicate functional groups or fragments of molecules attachable to a bond or other fragments of molecules. For example acetamide group represent the acetamide fragment or radical. Structures of groups, radicals or fragments unattached to the tetracyclic nucleus have been drawn to show the first line as a connecting bond only. Thus, the group 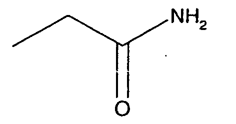 indicates the acetamide radical not the propanamide radical unless otherwise indicated.

The term, "amino acid residue" refers to the portion of the amino acid group coupled at the nitrogen atom of the amino terminus. It is the amino acid less a hydrogen atom from the amino terminus. It is further illustrated as used herein for the amino acid alanine attached at the nitrogen atom as shown below: 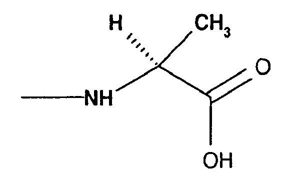 The term, "amine", includes primary, secondary and tertiary amines.

The term, "alkylene chain of 1 or 2 carbon atoms" refers to the divalent radicals, -CH₂-CH₂- and -CH₂-.

The term, "group containing 1 to 10 non-hydrogen atoms" refers to relatively small groups which form substituents at the 2- position of the tetracyclic nucleus, said groups may contain non-hydrogen atoms alone, or non-hydrogen atoms plus hydrogen atoms as required to satisfy the unsubstituted valence of the non-hydrogen atoms, for example; (i) groups absent hydrogen which contain no more than 4 non-hydrogen atoms such as -CF₃, -Cl, -Br, -NO₂, -CN, -SO₃; and (ii) groups having hydrogen atoms which contain less than 4 non-hydrogen atoms such as -CH₃, -C₂H₅, and -CH=CH₂.

The term "oxime amide" means the radical, -C(=NOR)-C(O)NH₂

The term "thio-oxime amide" means the radical -C(=NOR)-C(S)-NH₂.

### II. The Tetracyclic Compounds of the Invention:

The present invention provides a novel class of tetracyclic compounds useful as sPLA₂ inhibitors for the treatment and/or prophylaxis of inflammation attendant to inflammatory diseases. Subclasses of tetracyclic compounds of this invention include tetracyclic oxyacid derivatives, tetracyclic-5-oxime amide oxyacid derivatives, tetracyclic-5-acetamide oxyacid derivatives, tetracyclic-5-glyoxylamide-N-hydroxyfunctional amide derivatives, tetracyclic-5-oxime amide-N-hydroxyfunctional amide derivatives, tetracyclic-5-acetamide hydroxy functional amide derivatives, tetracyclic-5-glyoxylamide acylamino acid derivatives, tetracyclic-5-oxime amide acylamino acid derivatives, tetracyclic-5-acetamide acylamino acid derivatives.

The compounds of the invention are represented by the general formula (I) as defined above and include a pharmaceutically acceptable salt, solvate or prodrug thereof;

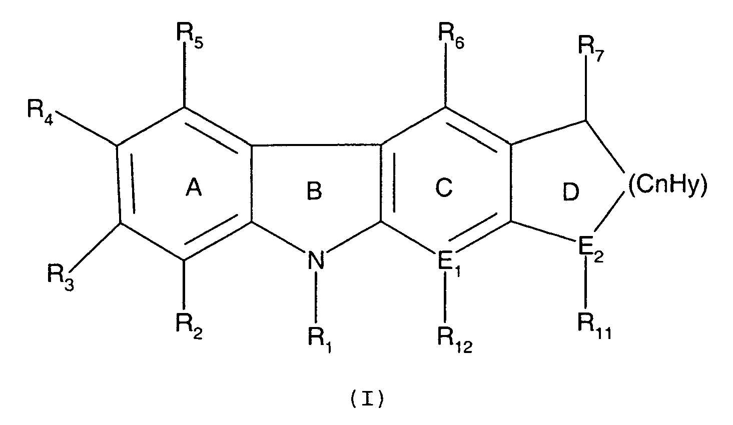

For positions 1 through 12 as shown in structure (X), and in compounds of the invention, it should be understood that where valency is incomplete an extra bond to hydrogen is contemplated by this invention to effect complete valency for the particular carbon, nitrogen or sulfur at that position of the ring i.e. D ring.

### Preferred Subgroups of Compounds of Formula (I):

A preferred subclass of compounds of formula I are those wherein E₁ and E₂ are independently carbon.

Another preferred class of compounds of formula I are those wherein E₁ and E₂ are independently nitrogen.

Also preferred is a subclass of compounds of formula I wherein E₂ is sulfur.

Another preferred subclass are compounds of formula I wherein the D ring is a 5 or 6 membered ring.

Also preferred is a subclass of compounds of formula I wherein the D ring is benzene.

Also preferred is a subclass wherein the C and D rings combine to form a naphthalene ring system.

### Preferred R₁ substituents:

Particularly preferred as the linking group -(L)-of R₁ is an alkylene chain of 1 or 2 carbon atoms, namely, -(CH₂)- or -(CH₂-CH₂)-.

Particularly preferred are compounds wherein for R₁ the combined group -(L)-R₈₀ is selected from the group consisting of 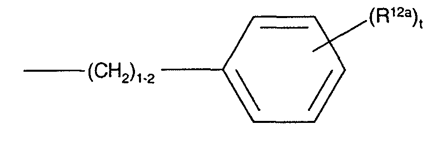 or 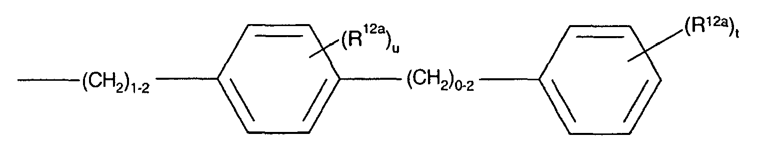 where R^{12a} is a radical independently selected from halo, (C₁-C₈)alkyl, (C₁-C₈)alkoxy, -S-((C₁-C₈)alkyl), -O-((C₁-C₈)alkyl) and (C₁-C₈)haloalkyl where t is a number from 0 to 5 and u is a number from 0 to 4.

### Preferred R₃/R₄ substituents:

Particularly preferred R₃ and R₄ groups are independently selected from hydrogen, methyl, ethyl, propyl, isopropyl, cyclopropyl, -F, -CF₃, -Cl, -Br, or -O-CH₃.

### Preferred R₅ substituents:

A preferred subclass of compounds of formula (I) are those wherein X is oxygen.

Also preferred is a subclass of compounds of formula I wherein Z is a glyoxylamide (glyoxamide) group represented by

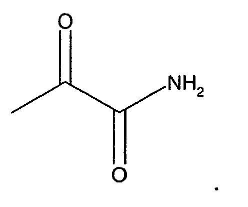

Another preferred subclass of compounds of formula (I) are those wherein Z is an amide group

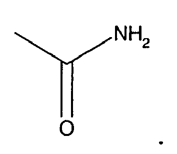

Another preferred subclass of compounds of formula (I) are those wherein Z is an oxime amide group.

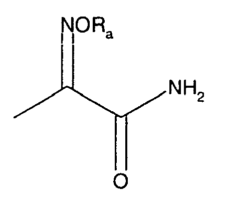

Also preferred are compounds of formula (I) wherein Z is an acetamide group represented by the formula: 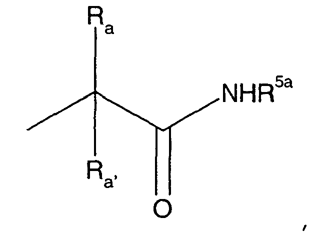 wherein Rₐ and R_{a'} are independently selected from hydrogen, (C₁-C₈)alkyl, aryl, (C₁-C₈)alkaryl, (C₁₋C₈)alkoxy, aralkyl and -CN, and R^{5a} is hydrogen, methyl or ethyl. For the group R₅ it is most preferred that the linking group -(L₅)- be a bond.

### Preferred R₆ substituents:

A preferred subclass of compounds of formula I are those wherein R₆ is a substituent selected from hydrogen, CONH₂, CONHR^{6b}, or the group, -(Lₐ)-(acidic group); wherein -(Lₐ)- is selected from the group consisting of; 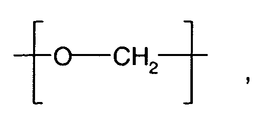 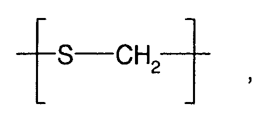 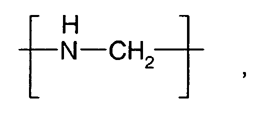 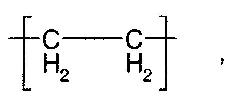 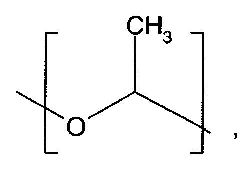 and 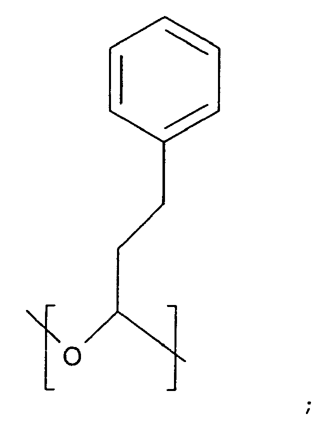 and
wherein the (acidic group) on the group -(Lₐ)-(acidic group) of R₆ is selected from -CO₂H, CO₂Na, COR^{6a}, -SO₃H, or -P(O)(OH)₂.

Another preferred subclass of compounds of formula (I) are those wherein R₆ is the group -(Lc)- (acylamino acid group)-, wherein the (acylamino acid group) is represented by the formula: 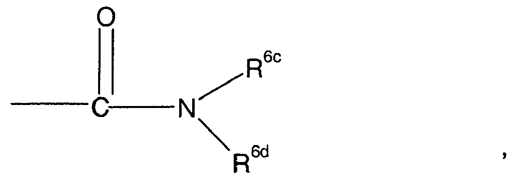 wherein R^{6c} is selected from the group consisting of H, (C₁-C₆)alkyl, (C₁-C₆)alkoxy, heteroaryl and aryl, -CF₃; and wherein NR^{6d} is an amino acid residue of either a natural or unnatural amino acid with the nitrogen atom being part of the amino group of the amino acid; and wherein the amino acid residue is derived from an amino acid selected from the group comprising isoleucine, valine, phenylalanine, aspartic acid, leucine, glycine, asparagine, cysteine, glutamine, glutamic acid, histidine, lysine, methionine, serine, threonine, tryptophan, tyrosine and derivatives thereof.

Another preferred subclass of compounds of formula (I) are those wherein R₆ is the group, -(Lₕ)-(N-hydroxyfunctional amide group) as previously defined.

Most preferred subclasses of compound of formula (I) are compounds where the acid linker -(La)-, or the N-hydroxyfunctional amide linker, -(Lₕ)-, or the acylamino acid linker -(L_{c})-, for R₆ is independently selected from the specific groups; 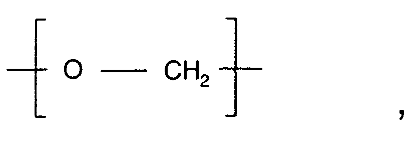 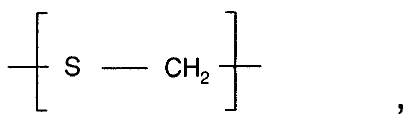 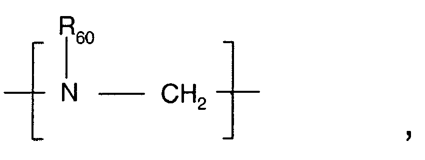 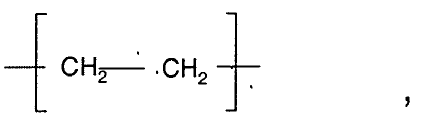 or 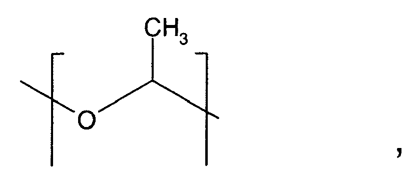 wherein R₆₀ is hydrogen or (C₁-C₈)alkyl.

The (N-hydroxyfunctional amide group) within the group R₆ is the group: 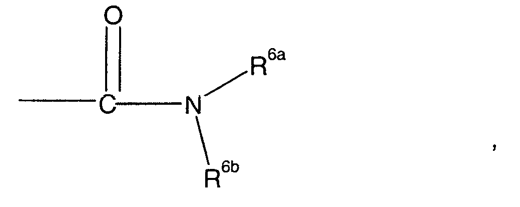 wherein R^{6a} is selected from the group consisting of OH, (C₁-C₆)alkoxy and aryloxy; and wherein R^{6b} is an organic substituent selected from the group consisting of H, (C₁-C₈)alkyl, aryl, (C₇-C₁₄)aralkyl, (C₇-C₁₄)alkaryl, (C₃-C₈)cycloalkyl, (C₁-C₈)alkoxyalkyl and these groups substituted with halogen, -CF₃, -OH, (C₁-C₈)alkyl, amino, carbonyl, and -CN. A more preferred R^{6a} group is selected from the group consisting of -OH, -OCH₃ and-OC₂H₅ while a more preferred R^{6b} is selected from the group consisting of H, (C₁-C₈)alkyl, aryl, (C₇₋C₁₄)aralkyl, (C₇-C₁₄)alkaryl, (C₃-C₈)cycloalkyl. A most preferred R^{6b} is a group selected from H, CH₃, C₂H₅ and C₃H₇. A salt or a prodrug derivative of the (N-hydroxyfunctional amide group) is also a suitable substituent.

### Preferred R₇ Substituents:

Preferred (acidic group) for R₇ is selected from the group consisting of -CO₂H, -SO₃H and -P(O) (OH)2. Most preferred for R₇ is the group hydrogen or a non-interfering substituent.

Most preferred compounds of the invention are those having the general formula (II) or (III) or (IV) or (V) or a pharmaceutically acceptable salt, solvate or prodrug derivative thereof; 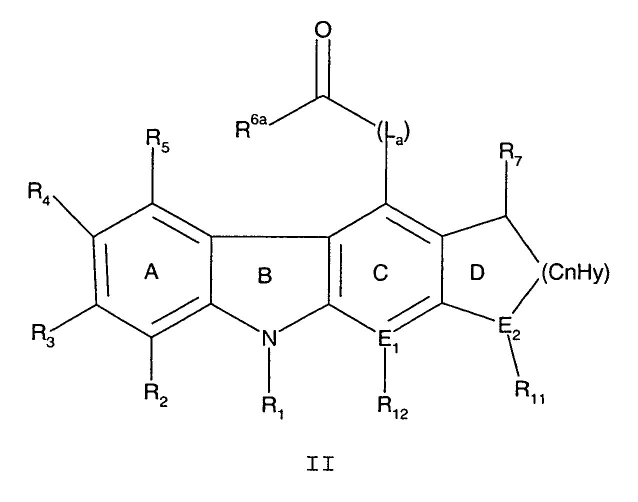 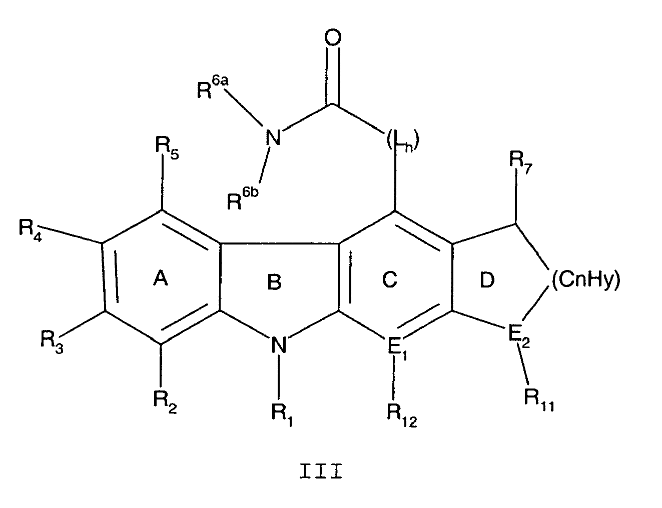 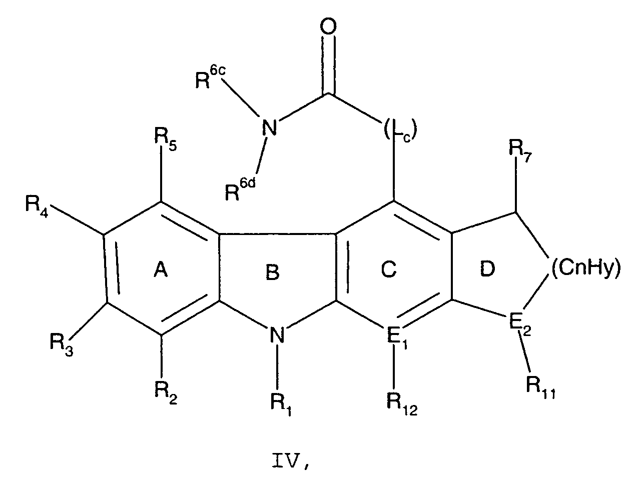 and 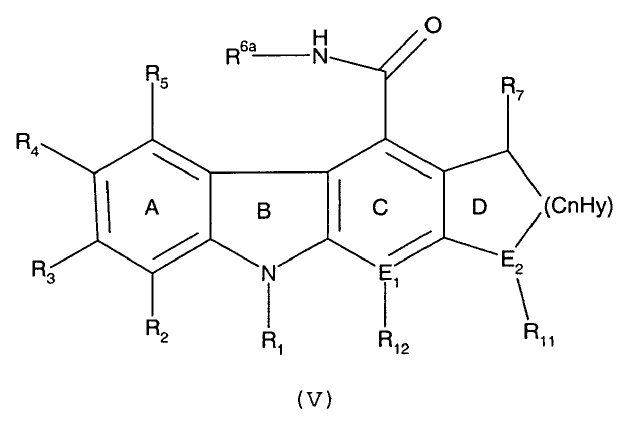 wherein;
E₁ and E₂ are independently C, N, S, or O with the appropriate number of hydrogen atoms or non-interfering substituents based on ring size and degree of unsaturation;
R₁ is the group -(L)-R₈₀; where, -(L)- is a divalent linking group of 1 to 12 atoms selected from carbon, hydrogen, oxygen, nitrogen, and sulfur; wherein the combination of atoms in -(L)- are selected from the group consisting of (i) carbon and hydrogen only, (ii) sulfur only, (iii) oxygen only, (iv) nitrogen and hydrogen only, (v) carbon, hydrogen, and sulfur only, and (vi) and carbon, hydrogen, and oxygen only; and where R₈₀ is a substituted or unsubstituted group selected from the group consisting of (C₅₋C₁₄)cycloalkyl, (C₅-C₁₄)cycloalkenyl, phenyl, naphthyl, norbornanyl, bicycloheptadienyl, toluyl, xylenyl, indenyl, stilbenyl, terphenylyl, diphenylethylenyl, phenyl-cyclohexenyl, acenaphthylenyl, and anthracenyl, biphenyl, bibenzylyl and related bibenzylyl homologues represented by the formula (a); 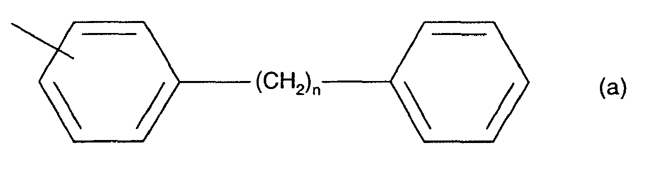 where n is a number from 1 to 8;
R₂, R₃, and/or R₄ where applicable is selected from hydrogen, methyl, ethyl, propyl, isopropyl, cyclopropyl, -F, -CF₃, -Cl, -Br, or -O-CH_{3;}
R₅ is -(L₅)-Z wherein (L₅) is a bond and Z is selected from the group consisting of 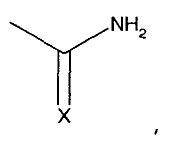 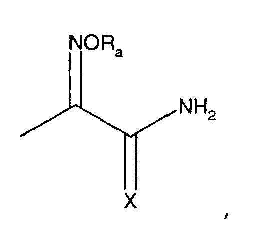 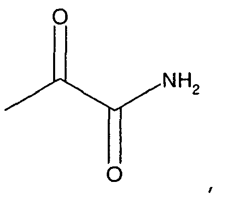 and

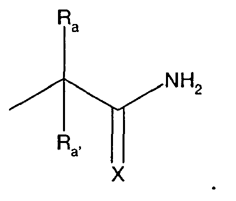

The acidic tetracyclic Compounds of the Invention

The acidic group tetracyclic compounds of the Invention are represented by a compound of formula (II) below: 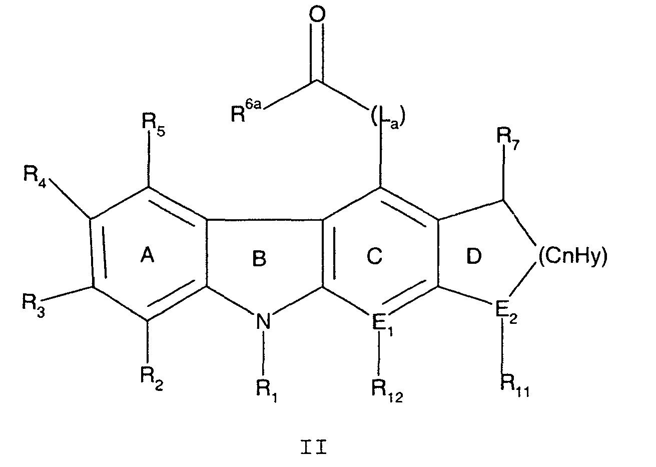 wherein E is carbon or nitrogen; n = 1, and
wherein (Lₐ) is selected from a group represented by the formula; 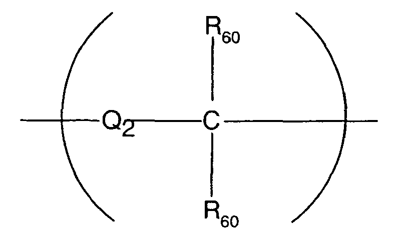 where Q₂ is selected from the group -(CH₂)-, -O-, -NR₆₀-, -C(O)-, and -S-, and each R₆₀ is independently selected from hydrogen, (C₁-C₈)alkyl, aryl, (C₁₋C₈)alkaryl, (C₁-C₈)alkoxy, aralkyl, and halo;
R₁ is as described previously;
R₂ is as described previously;
R₃, and R₄ are as described previously;
R₅ is -(L₅)- Z, where -(L₅)- is a divalent linker group selected from a bond or a divalent group selected from:

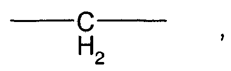

-O- ,

-S- ,

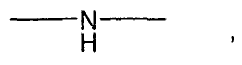

or 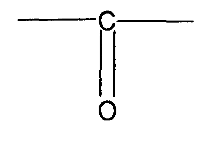 and Z is selected from an amide, thioamide or glyoxylamide, acetamide or thioacetamide, oxime, hydrazide radical (group) represented by the formulae, 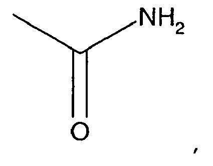 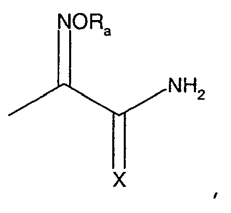 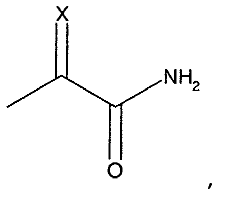 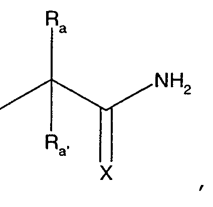 or 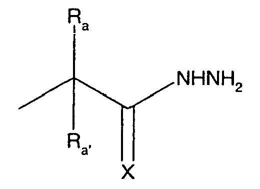 wherein X is oxygen or sulfur, Rₐ and Rₐ' are independently selected from hydrogen, (C₁-C₈)alkyl, aryl, and (C₁-C₈)alkaryl;
R₇ is selected from hydrogen, a non-interfering substituent, or the group, -(Lₐ)-(acidic group); wherein -(Lₐ)-, is selected from a group represented by the formula; 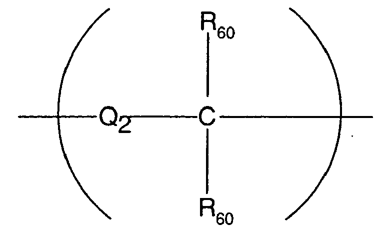 where Q₂ is selected from the group -(CH₂)-, -O-, -NR₆₀-, -C(O)-, and -S-, and each R₆₀ is independently selected from hydrogen, (C₁-C₈)alkyl, aryl, (C₁₋C₈)alkaryl, (C₁-C₈)alkoxy, aralkyl, and halo.

### The N-hydroxyfunctional amide tetracyclic Compounds of the Invention

The N-hydroxyfunctional amide tetracyclic Compounds of the Invention are represented by the general structure III below:

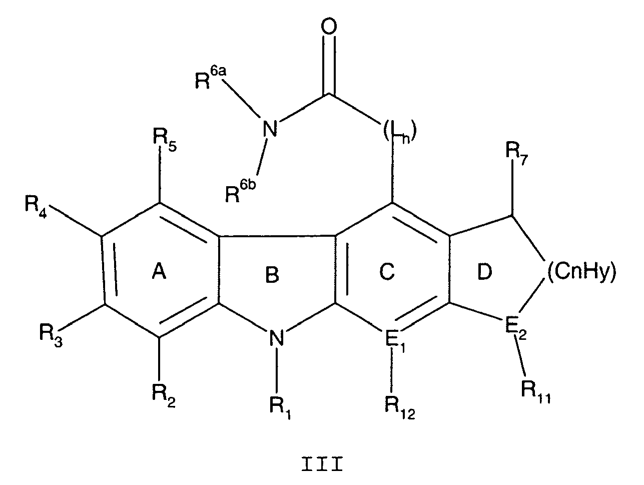

Wherein, E₁ and E₂ are independently carbon, nitrogen or sulfur with the appropriate number of non-interfering substituents based on D ring size and degree of unsaturation;
R₁, R₂, R₃ and R₄ are as described previously;
R₅ is -(L₅)- Z, where -(L₅)- is a divalent linker group selected from a bond or a divalent group selected from:

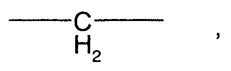

-O- ,

-S- ,

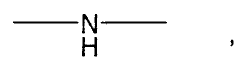

or 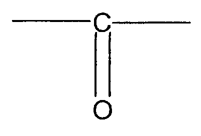 and Z is selected from an amide, thioamide or glyoxylamide, acetamide or thioacetamide, oxime, hydrazide radical (group) represented by the formulae, 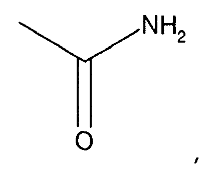 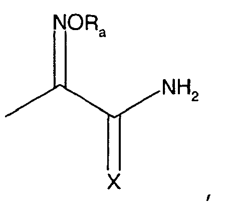 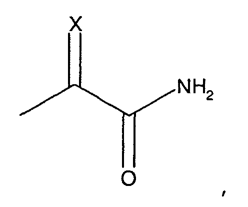 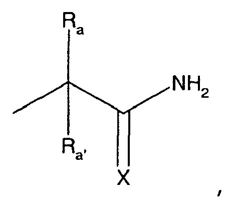 or 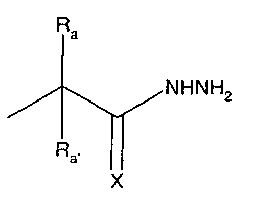 wherein X is oxygen or sulfur, Rₐ and Rₐ, are independently selected from hydrogen, (C₁-C₈)alkyl, aryl, and (C₁-C₈)alkaryl;

### Preferred R₆ substituents:

A preferred subclass of compounds of formula (III) are those wherein -(Lₕ)-, is selected from a group represented by the formula; 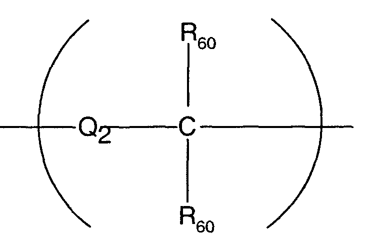 where Q₂ is selected from the group -(CH₂)-, -O-, -NH-, -C(O)-, and -S-, and each R₆₀ is independently selected from hydrogen, (C₁-C₈)alkyl, aryl, (C₁₋C₈)alkaryl, (C₁-C₈)alkoxy, aralkyl, and halo. Most preferred are compounds where -(Lₕ)-, is selected from the specific groups; 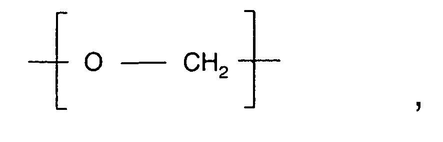 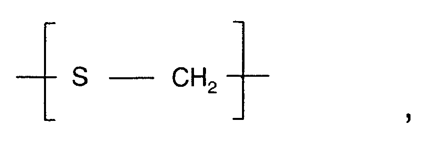 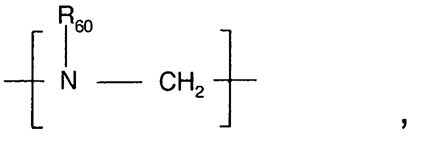 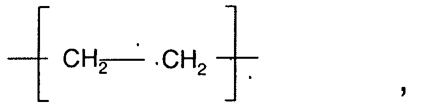 or 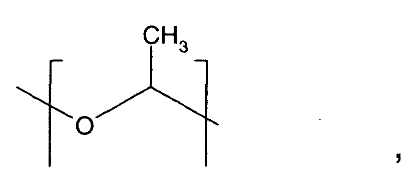 where R₆₀ is hydrogen or (C₁-C₈)alkyl.

Preferred is the group: 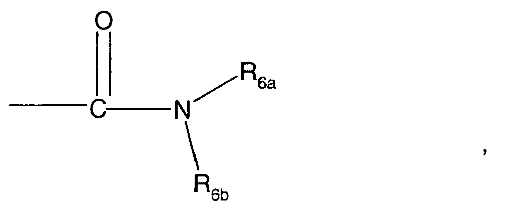 wherein R^{6a} is selected from the group consisting of OH, and (C₁-C₆)alkoxy; and wherein, R^{6b} is hydrogen, (C₁₋C₆)alkyl, phenyl and benzyl.

R₇ is preferably selected from hydrogen or a non-interfering substituent.

### The acylamino acid tetracyclic Compounds of the Invention:

The tetracyclic acylamino acid compounds of the invention are represented by the general formula (IV) or a pharmaceutically acceptable salt, solvate or prodrug thereof; 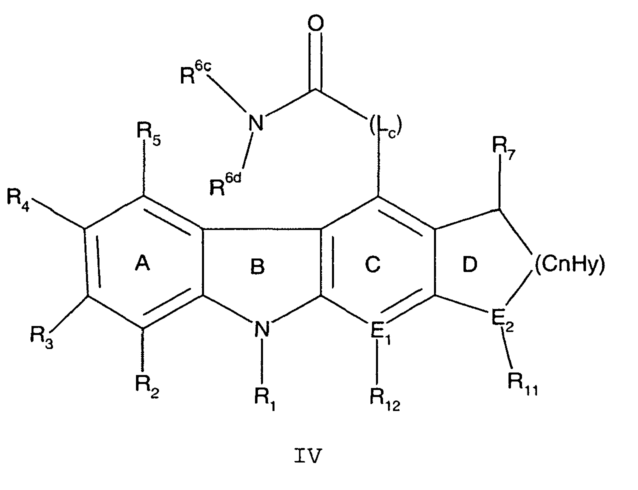 wherein
E₁ and E₂ are independently carbon or nitrogen or sulfur;
R₁, R₂, R₃, and R₄ are as described previously;
R₅ is -(L₅)- Z, where -(L₅)- is a divalent linker group selected from a bond or a divalent group selected from:

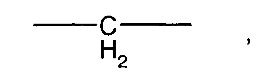

-O- ,

-S- ,

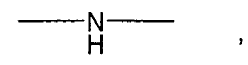

or 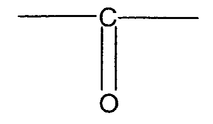 and Z is selected from an amide, thioamide or glyoxylamide, acetamide or thioacetamide, oxime, hydrazide radical (group) represented by the formulae, 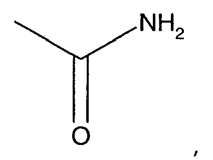 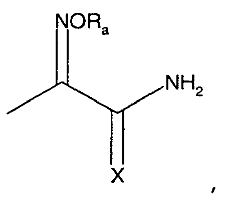 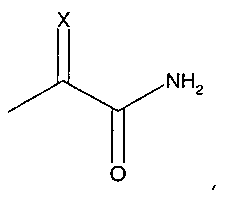 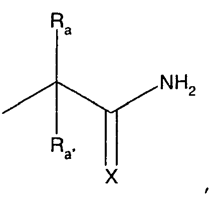 or 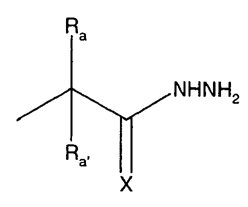 wherein X is oxygen or sulfur, Rₐ and R_{a'} are independently selected from hydrogen, (C₁-C₈)alkyl, aryl, and (C₇-C₁₄)alkaryl;

### Preferred R₆ substituents:

A preferred subclass of compounds of formula (IV) are those wherein -(L_{c})-, is selected from a group represented by the formula; 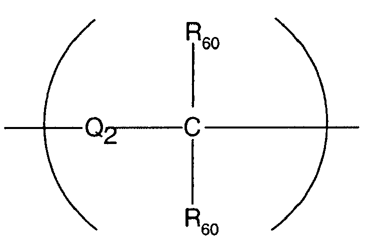 where Q₂ is selected from the group -(CH₂)-, -O-, -NH-, -C(O)-, and -S-, and each R₆₀ is independently selected from hydrogen, (C₁-C₈)alkyl, aryl, (C₁₋C₈)alkaryl, (C₁-C₈)alkoxy, aralkyl, and halo. Most preferred are compounds where -(L_{c})-, is selected from the specific groups; 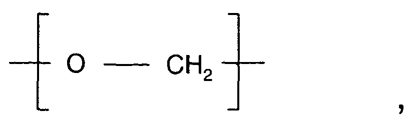 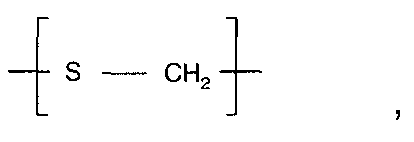 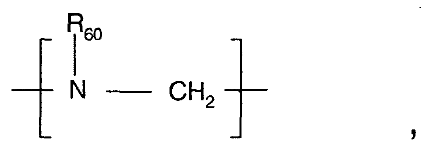 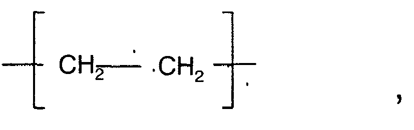 or 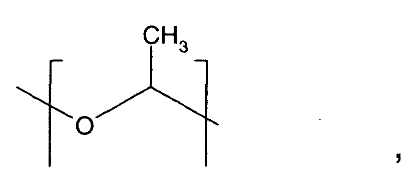 where R₆₀ is hydrogen or (C₁-C₈)alkyl.

Preferred is the group: 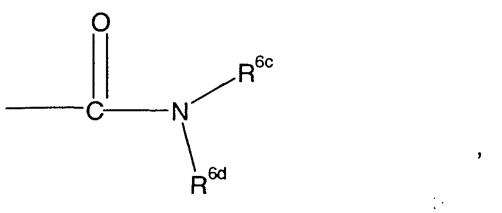 wherein R^{6c} is selected from the group consisting of H, (C₁-C₆)alkyl, (C₁-C₆)alkoxy, heteroaryl and aryl; and wherein NR^{6d} is an amino acid residue of either a natural or unnatural amino acid with the nitrogen atom being part of the amino group of the amino acid. A preferred R^{6c} group is the group hydrogen (H). A preferred source of amino acid residue is the amino acid group selected from the group comprising isoleucine, valine, phenylalanine, aspartic acid, leucine, glycine and isomers and derivatives thereof. A salt or a prodrug derivative of the (acylamino acid group) is also a suitable substituent.

Particularly preferred are R^{6d} groups that combine with the nitrogen atom to represent amino acid residues from the amino acid groups selected from: glycine, glycine methyl ester, L-alanine, L-alanine methylester, L-leucine, L-leucine methyl ester, L-aspartic acid, L-aspartic acid dimethylester, L-phenyl alanine, L-phenylalanine methyl ester, malonic acid, malonic acid dimethylester, L- valine, L-valine methyl ester, L-isoleucine, L-isoleucine methyl ester, or salt, and derivatives thereof.

R₇ is selected from hydrogen, a non-interfering substituent, or the group, -(Lₐ)-(acidic group); wherein -(Lₐ)-, is selected from a group represented by the formula; 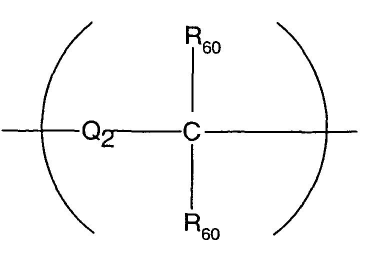 where Q₂ is selected from the group -(CH₂)-, -O-, -NR₆₀-, -C(O)-, and -S-, and each R₆₀ is independently selected from hydrogen, (C₁-C₈)alkyl, aryl, (C₁₋C₈)alkaryl, (C₁-C₈)alkoxy, aralkyl, and halo.

Preferred acylamino acid compounds of the invention are those having the general formula (IVa), or a pharmaceutically acceptable salt, solvate or prodrug derivative thereof; 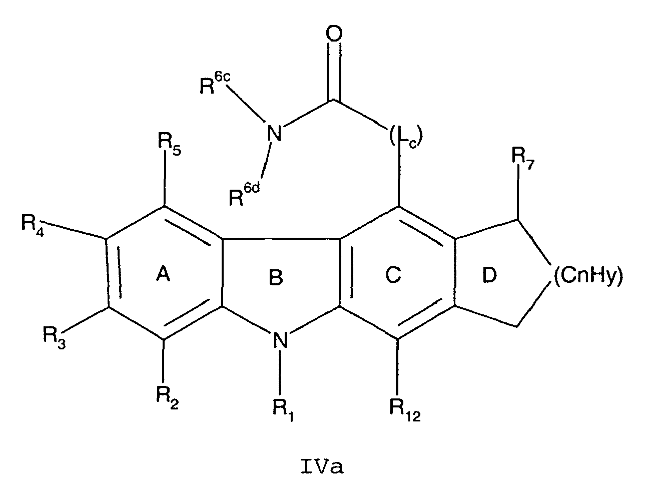 wherein;
n is 1 or 2;and y is 1, 2 or 4 depending on D ring unsaturation.
R₁ is selected from (C₁-C₈)alkyl, aryl, and alkylaryl; R₂, R₃, and R₄ are independently selected from the group consisting of hydrogen, methyl, ethyl, propyl, isopropyl, cyclopropyl, -F, -CF₃, -Cl, -Br, or -O-CH_{3;} R₅ is the group -(L₅)-Z, wherein Z is selected from the groups amide and hydrazide and wherein L₅ is a bond; and wherein R^{6c} is selected from the group consisting of H, (C₁-C₆)alkyl, (C₁-C₆)alkoxy, heteroaryl and aryl; and wherein NR^{6d} is an amino acid residue of either a natural or unnatural amino acid with the nitrogen atom being part of the amino group of the amino acid. A preferred R^{6c} group is the group hydrogen (H); and - (Lc)- is a divalent group selected from; 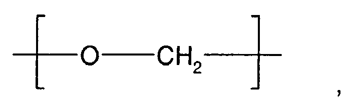 and 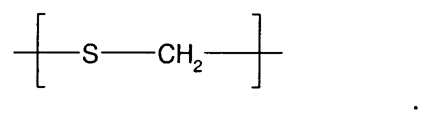 The tetracyclic-5-acetamide sPLA₂ inhibitor compounds of the present invention are represented by compounds of formula (V), and pharmaceutically acceptable salts and prodrug derivatives thereof, 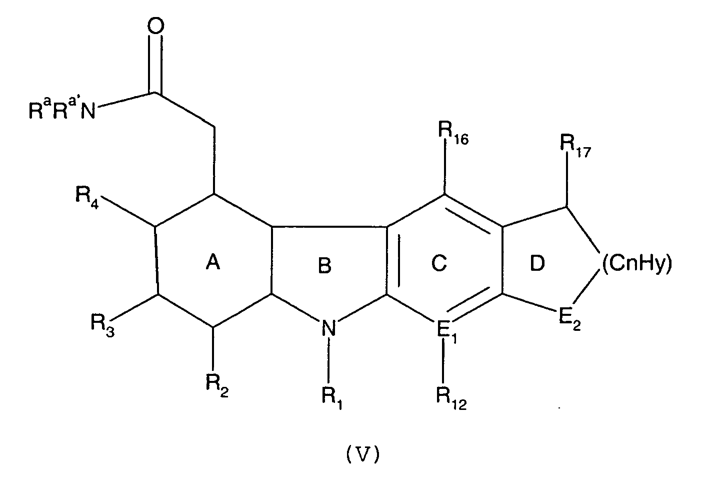 wherein;
the D ring has 0 to 2 double bonds and an appropriate number of hydrogen atom or non-interfering group appendages;
E₁ and E₂ are independently carbon or nitrogen;
R₂, R₃, and R₄ are independently selected from the group consisting of halo, (C₁-C₂)alkylthio, or (C₁₋C₂)alkoxy;
each R^{a} and R^{a}' are as described previously;
R₁₆ is the group, -(Lₕ)-(N-hydroxyfunctional amide group); or -(L_{c})-(acylamino acid group) ; or -(Lₐ)-(acidic group); each as defined for formula (I);
R₁₇ is hydrogen, (C₁-C₈)alkyl, (C₂-C₈)alkenyl, (C₂-C₈)alkynyl, (C₃-C₈)cycloalkyl, aryl selected from phenyl, holuyl, xylenyl, biphenyl, (C₇-Cₗ₂)aralkyl;
The tetracyclic-5-amide compounds

A compound of formula (VI) or a pharmaceutically acceptable salt, solvate or prodrug thereof represent tetracyclic-5-amide compounds of the invention; 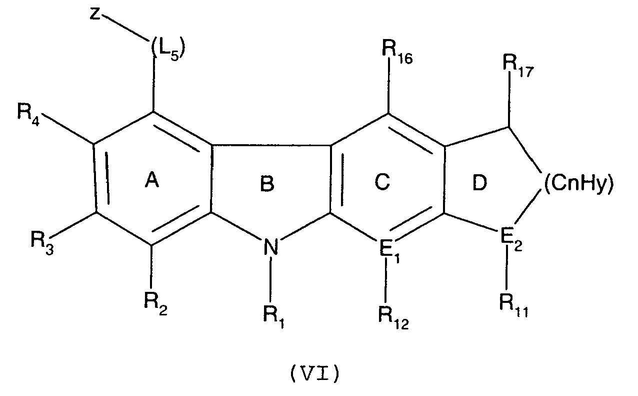 wherein ;
R₁ is as described previously;
R₂ is hydrogen, or a group containing 1 to 4 non-hydrogen atoms plus any required hydrogen atoms;
R₃ and R₄ are independently hydrogen or a non-interfering group;
-(L₅)- Z, is the group where -(L₅)- is a divalent linker group selected from a bond or a divalent group selected from:

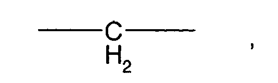

-O- ,

-S- ,

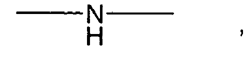

or 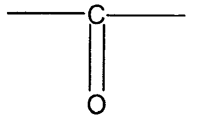 and Z is selected from an amide or thioamide radical or group represented by the formula, 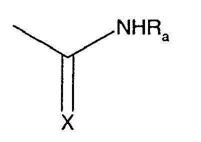 wherein, X is oxygen or sulfur; and Rₐ is selected from hydrogen, (C₁-C₈)alkyl, aryl, (C₁-C₈)alkaryl;
R₁₁ and R₁₂ are as described previously;
R₁₆ is the group, -(Lₐ)-(acidic group); or the group -(Lₕ)-(N-hydroxyfunctional amide group); or the group -(L_{c})-(acylamino acid group); each as defined for formula (I);
R₁₇ is selected from hydrogen, a non-interfering substituent, or the group, -(Lₐ)-(acidic group)

### The tetracyclic-5-glyoxylamide Compounds

Compounds of formula (VII) or a pharmaceutically acceptable salt, solvate or prodrug thereof represent tetracyclic-5-glyoxylamide compounds of the invention thereof; 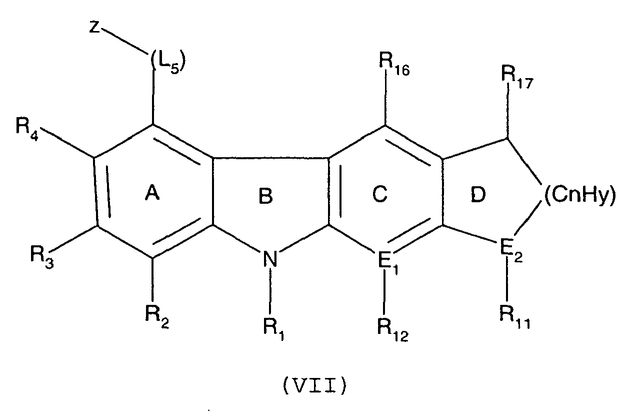 wherein ;
E₁ and E₂ are independently C or N;
R₂ is hydrogen, or a group containing 1 to 4 non-hydrogen atoms plus any required hydrogen atoms;
R₃ and R₄ is each independently selected from hydrogen or a non-interfering group;
-(L₅)- Z, is the group where -(L₅)- is a divalent linker group selected from a bond or a divalent group selected from:

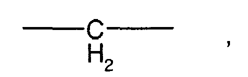

-O- ,

-S- ,

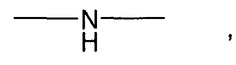

or 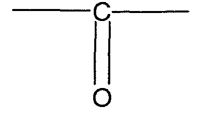 and Z is a glyoxylamide group represented by the formula, 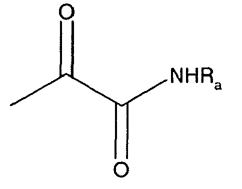 wherein, Rₐ is selected from hydrogen, (C₁-C₈)alkyl, aryl, (C₁-C₈)alkaryl, and aralkyl; and wherein a most preferred Rₐ is hydrogen;
R₁₁ is as described previously;
R₁₆ is the group, -(Lₐ)-(acidic group); or the group -(Lₕ)-(N-hydroxyfunctional amide group); or the group -(L_{c})-(acylamino acid group); each as defined for formula (I);
R₁₇ is selected from hydrogen, a non-interfering substituent, or the group, -(Lₐ)-(acidic group).

### The tetracyclic-5-oxime amide compounds

Compounds of formula (VIII) or a pharmaceutically acceptable salt, solvate or prodrug thereof represent tetracyclic-5-oxime amide compounds of the invention; 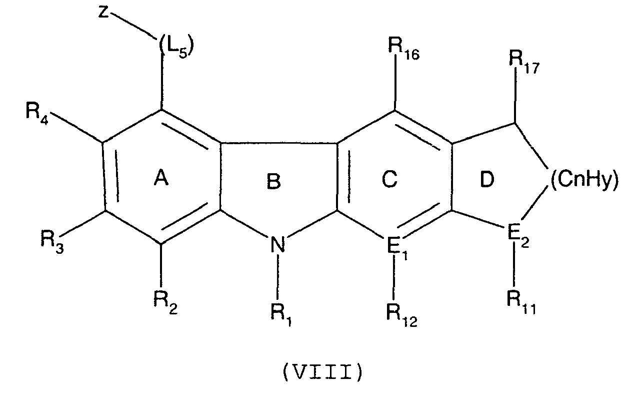 wherein;
E₁ and E₂ are independently C or N or S, or O with the appropriate number of non-interfering groups appended depending on ring saturation;
R₁₁ is as described previously;
R₁₂ is hydrogen, or a group containing 1 to 4 non-hydrogen atoms plus any required hydrogen atoms;
R₃ and R₄ are independently a non-interfering
group;
-(L₅)- Z, is the group where -(L₅)- is a divalent linker group selected from a bond or a divalent group selected from:

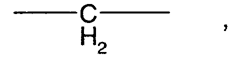

-O- ,

-S- ,

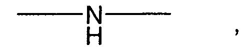

or 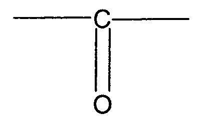 and Z is selected from an oxime amide group represented by the formula, 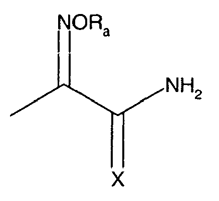 wherein, X is oxygen or sulfur; and Rₐ is selected from hydrogen, (C₁-C₈)alkyl, aryl, (C₁-C₈)alkaryl, (C₁-C₈)alkoxy, aralkyl and -CN;
R₁₆ is the group, hydrogen, or the group -(Lₐ)-(acidic group); or the group -(Lₕ)-(N-hydroxyfunctional amide group); or the group -(L_{c})-(acylamino acid group); as each is defined for formula (I);
R₁₇ is selected from hydrogen, a non-interfering substituent, or the group, -(Lₐ)-(acidic group).

Most preferred compounds (and all pharmaceutically acceptable salts, solvates and prodrug derivatives thereof) which are illustrative of the compounds of the invention for treatment of a human afflicted with Inflammatory Disease, a pharmaceutically acceptable salt, solvate, or a prodrug derivative of a compound selected from the group consisting of:
(10-Benzyl-6-carbamoyl-1,2,3,10-tetrahydrocyclopenta[a]carbazol-5-yloxy)acetic acid methyl ester;
(10-Benzyl-6-carbamoyl-1,2,3,10-tetrahydrocyclopenta[a]carbazol-5-yloxy)acetic acid;
(11-Benzyl-7-carbamoyl-2,3,4,11-tetrahydro-1*H-*benzo[a]carbazol-6-yloxy)acetic acid methyl ester, and
(11-Benzyl-7-carbamoyl-2,3,4,11-tetrahydro-1*H-*benzo[a]carbazol-6-yloxy)acetic acid;
(5-Benzyl-1-carbamoyl-5H-benzo[b]carbazol-11-yloxy)-acetic acid, sodium salt; and
(5-Benzyl-1-carbamoyl-5H-benzo[b]carbazol-11-yloxy) - acetic acid, methyl ester;
Preferred compounds of the invention are represented by the formulae (C1), (C2), (C3), (C4), (C5), (C6), (C7), or (C8);

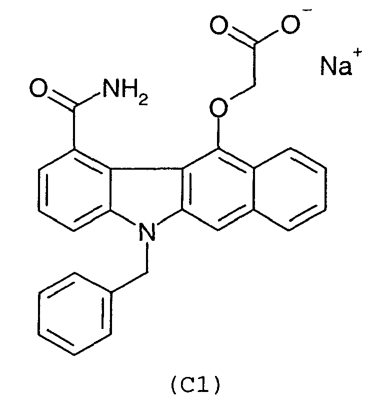

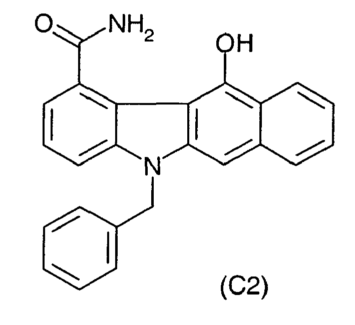

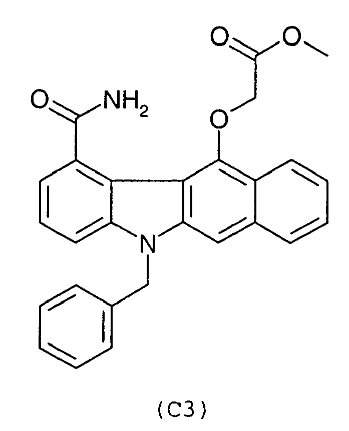

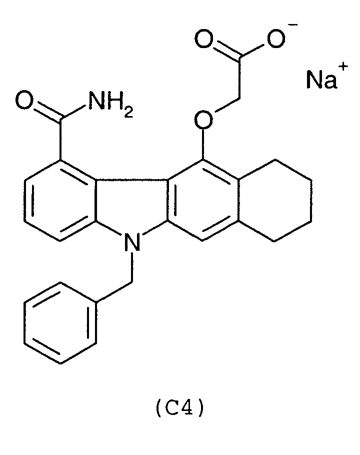

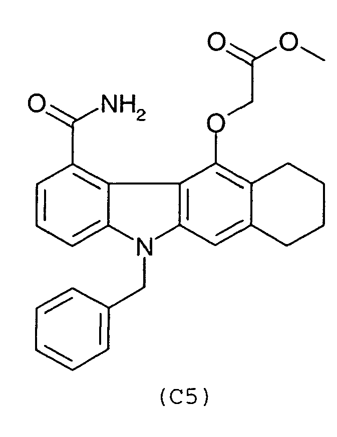

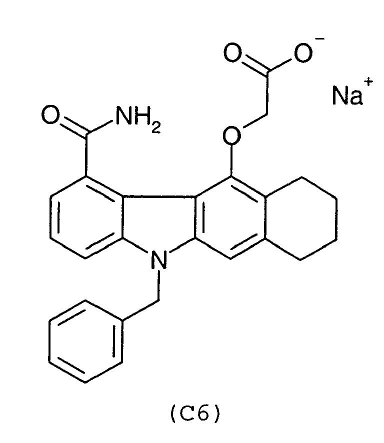

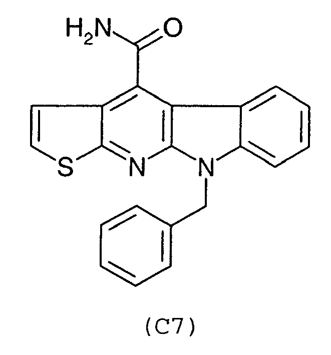

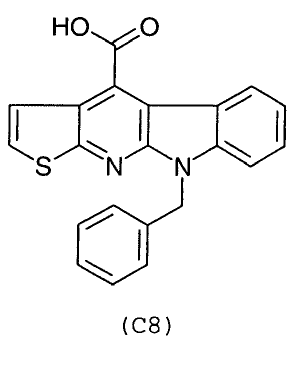

The salts of the tetracyclic compounds represented by formulae (I), (II), (III), (IV), (V), (VI), (VII), and (VIII) are an additional aspect of the invention.

In those instances when the compound of the invention possesses acidic or basic functional groups, various salts may be formed which are more water soluble and more physiologically suitable than the parent compound. Representative pharmaceutically acceptable salts, include but are not limited to, the alkali and alkaline earth salts such as lithium, sodium, potassium, calcium, magnesium, aluminum and the like. Salts are conveniently prepared from the free acid by treating the acid in solution with a base or by exposing the acid to an ion exchange resin.

Included within the definition of pharmaceutically acceptable salts are the relatively non-toxic, inorganic and organic base addition salts of compounds of the present invention, for example, ammonium, quaternary ammonium, and amine cations, derived from nitrogenous bases of sufficient basicity to form salts with the compounds of this invention (see, for example, S. M. Berge, et al., "Pharmaceutical Salts," J. Phar. Sci., 66: 1-19 (1977)). Moreover, the basic group(s) of the compound of the invention may be reacted with suitable organic or inorganic acids to form salts such as acetate, benzenesulfonate, benzoate, bicarbonate, bisulfate, bitartrate, borate, bromide, camsylate, carbonate, chloride, clavulanate, citrate, chloride, edetate, edisylate, estolate, esylate, fluoride, fumarate, gluceptate, gluconate, glutamate, glycolylarsanilate, hexylresorcinate, bromide, chloride, hydroxynaphthoate, iodide, isothionate, lactate, lactobionate, laurate, malate, maleate, mandelate, mesylate, methylbromide, methylnitrate, methylsulfate, mucate, napsylate, nitrate, oleate, oxalate, palmitate, pantothenate, phosphate, polygalacturonate, salicylate, stearate, subacetate, succinate, tannate, tartrate, tosylate, trifluoroacetate, trifluoromethane sulfonate, and valerate.

Certain compounds of the invention may possess one or more chiral centers, and thus, may exist in optically active forms. Likewise, when the compounds contain an alkenyl or alkenylene group, there exist the possibility of cis- and trans- isomeric forms of the compounds. The R- and S- isomers and mixtures thereof, including racemic mixtures as well as mixtures of cis- and trans-isomers, are contemplated by this invention. Additional asymmetric carbon atoms can be present in a substituent group such as an alkyl group. All such isomers as well as the mixtures thereof are intended to be included in the invention. If a particular stereoisomer is desired, it can be prepared by methods well known in the art by using stereospecific reactions with starting materials which contain the asymmetric centers and are already resolved or, alternatively by methods which lead to mixtures of the stereoisomers and subsequent resolution by known methods. For example, a racemic mixture may be reacted with a single enantiomer of some other compound. This changes the racemic form into a mixture of stereoisomers and diastereomers, because they have different melting points, different boiling points, and different solubilities and can be separated by conventional means, such as crystallization.

Prodrugs are derivatives of the compounds of the invention which have chemically or metabolically cleavable groups and become by solvolysis or under physiological conditions the compounds of the invention which are pharmaceutically active in vivo. Derivatives of the compounds of this invention have activity in both their acid and base derivative forms, but the acid derivative form often offers advantages of solubility, tissue compatibility, or delayed release in a mammalian organism (see, Bundgard, H., Design of Prodrugs, pp. 7-9, 21-24, Elsevier, Amsterdam 1985). Prodrugs include methyl, ethyl, propyl, isopropyl, n-butyl, isobutyl, tert-butyl, morpholinoethyl, and N,N-diethylglycolamido esters.

N,N-diethylglycolamido ester prodrugs may be prepared by reaction of the sodium salt of a compound of Formula (I) (in a medium such as dimethylformamide) with 2-chloro-N,N-diethylacetamide (available from Aldrich Chemical Co., Milwaukee, Wisconsin USA; Item No. 25,099-6). Morpholinylethyl ester prodrugs may be prepared by reaction of the sodium salt of a compound of formula (I) (in a medium such as dimethylformamide) with 4-(2-chloroethyl)morpholine hydrochloride (available from Aldrich Chemical Co., Milwaukee, Wisconsin USA, Item No. C4, 220-3).

### (III) Method of preparing the tetracyclic-5-amide Compound:

The tetracyclic-5-amide compounds are compounds of this invention and are also useful as intermediates or starting materials for preparing other compounds of the invention. The tetracyclic-5-amide compounds are prepared by following a Scheme such as Scheme 1, as shown below:

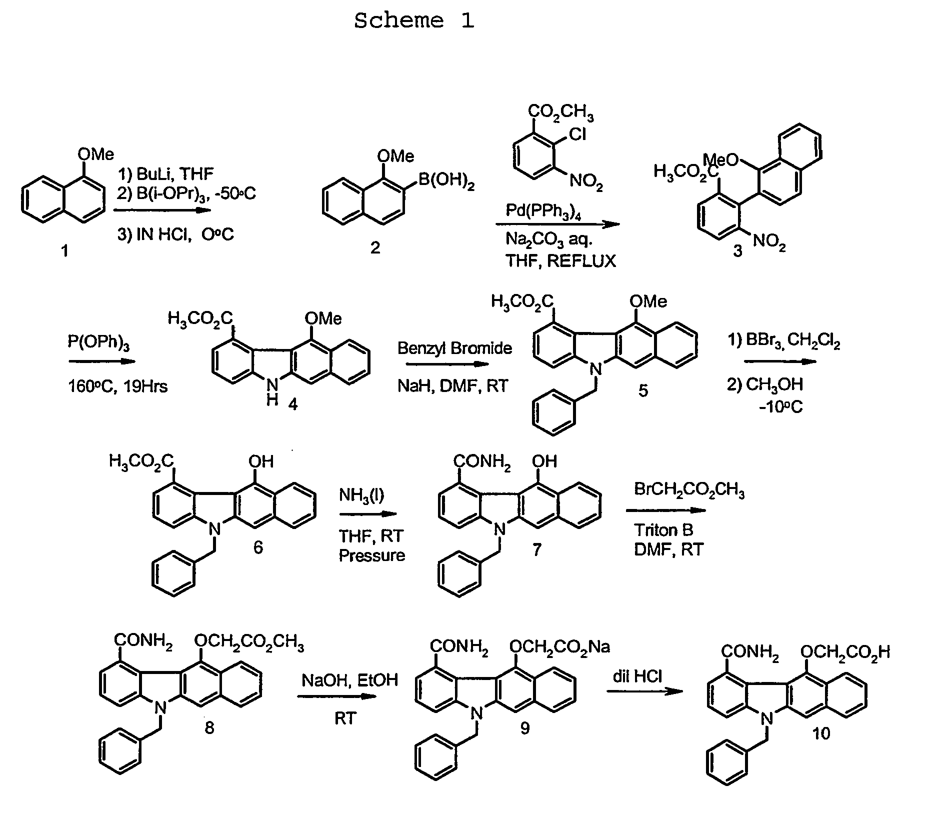

Scheme 1 depicts a protocol for preparing compounds of the invention wherein the D ring is a benzene ring system. By use of 1-methoxynaphthalene (availalble from Aldrich Chemical Co., Milwaukee, USA) substituted or unsubstituted at the 3 through 8 position(s), substituted analogs of compounds of the invention as disclosed, may be prepared. As shown, the reaction of 1-methoxynaphthalene with n-butyl lithium or similar organolithium reagent affords 2-lithio-1-methoxy naphthalene intermediate. Addition of a borate ester e.g., triisopropyl borate to the 2-lithio-1-methoxy naphthalene intermediate, generates the corresponding boronic acid compound (2) upon acidic work-up with, for example, aqueous HCl. The boronic acid compound (2) is then coupled with methyl-2-halo-3-nitrobenzoate (preferably chloro analog i.e., methyl-2-chloro-3-nitrobenzoate) in a Suzuki type coupling reaction (*Syn th. Commun., 11*, *513 (1981)*). The reaction involves use of tetrakis(triphenylphosphine)palladium(0) or other suitable palladium catalyst, and a base such as sodium carbonate. Upon work-up and isolation, i.e., by chromatography, the coupling reaction product (3) is obtained. One of skill in the art is aware that substituted analogs of the compound (3) may be prepared by using methyl-3-halo-3-nitro benzoate reagents with non-interfering substituents at the 4, 5 and/or 6 position(s) of the benzene ring. The compound (3) is reductively cyclized to the carbazole-type tetracyclic compound of (4) using about a molar equivalent triphenyl phosphite under sealed tube conditions (see "The Reactivity of Organophosphorus Compounds. Part XIX. Reduction of Nitro-compounds by Triethyl Phosphite: A Convenient New Route to Carbazoles, Indoles, Indazoles, Triazoles, and Related Compounds," J. I. G. Cadogan, M. Cameron-Wood, R. K. Mackie, and R. J. G. Searle, *J. Chem. Soc.,* **1965**, 4831.). The reductive cyclization reaction for converting compound (3) to compound (4) is performed at temperatures of about 100 °C to 180 °C, preferably about 160 °C. The compound (4) is alkylated or arylated at the carbazole nitrogen to introduce the R₁ group, by a base catalyzed deprotonation followed by a nucleophilic attack on an electrophile. Electrophiles suitable for this reaction are those necessary to incorporate the R₁ group described previously and include for example, alkyl, aryl, and arylalkyl groups as the halides, sulfonates or other leaving groups. For example the reaction of compound (4) with sodium hydride or a suitable base (i.e. n-BuLi, lithium triisopropyl amide, etc) in a suitable solvent e.g., dimethylformamide, followed by addition of benzyl bromide for example, affords upon work-up the compound of formula (5). The compound (5) is demethylated by reaction with boron tribromide or sodium thioethoxide in a suitable solvent such as dichloromethane. About 1.0 to 2.0 equivalents of boron tribromide is typically sufficient to effect complete de-methylation. The de-methylation reaction temperature is from about -12 °C to about 10 °C. Work-up is effected by stirring with methyl alcohol, or other suitable protic solvent. The stirring in methyl alcohol is followed by neutralization with a base e.g. sodium bicarbonate. This is followed by extraction, and purification of the organic phase by methods known to one of skill; in the art, to afford compound (6).

A solution of compound (6) or analog thereof, in THF, is reacted with condensed ammonia in a sealed tube from a temperature of about 20 °C to about 70 °C, preferably at about 50 °C, over a period of 1 to 7 days, preferably 4 days. Condensed ammonia is obtained by running a stream of ammonia gas over the solution at about -78 °C as desired. Alternatively, the reaction can be performed by directly heating a mixture of excess ammonia gas and compound (6) in a sealed tube under temperature and time conditions as described above, to afford the compound (7).

The compound (7) is then subjected to a phase transfer coupling reaction with 2-bromomethylacetate using 2 non-interfering basic catalyst such as benzyltrimethylammonium hydroxide (triton-B, Rohm and Haas Corporation trademark) cesium carbonate, potassium carbonate, and the like, to afford the compound (8) or analog thereof based on starting material.

The compound of formula (8), itself, a compound of the invention, may be converted to the free acid or acid salt. The salt compound (9) or analog thereof, is obtained by reaction with sodium hydroxide (saponification) or lithium hydroxide in a suitable solvent e.g., THF. The product is isolated by drying or extraction into the aqueous phase followed by drying to afford compound (9) or analog thereof.

The free acid (10) is optionally obtained by acidifying the product of saponification (9) or other basification reaction, e.g. with lithium hydroxide. Most strong inorganic acids are suitable for acidification as described previously. However, the use of dilute HCl is preferred. The free acid (10) may be extracted into an organic phase if soluble, and dried by common laboratory methods or dried by removing water from the aqueous phase.

Compounds of formula I or intermediates thereto, wherein the D ring is a cyclohexyl or a substituted cylcohexane ring are prepared by starting with 6-methoxy-5-halo-1,2,3,8 tetrahydronaphthalene or analog substituted with non-interfering groups. The protocol according to Scheme 2 below; 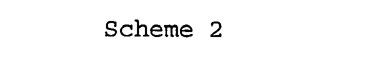 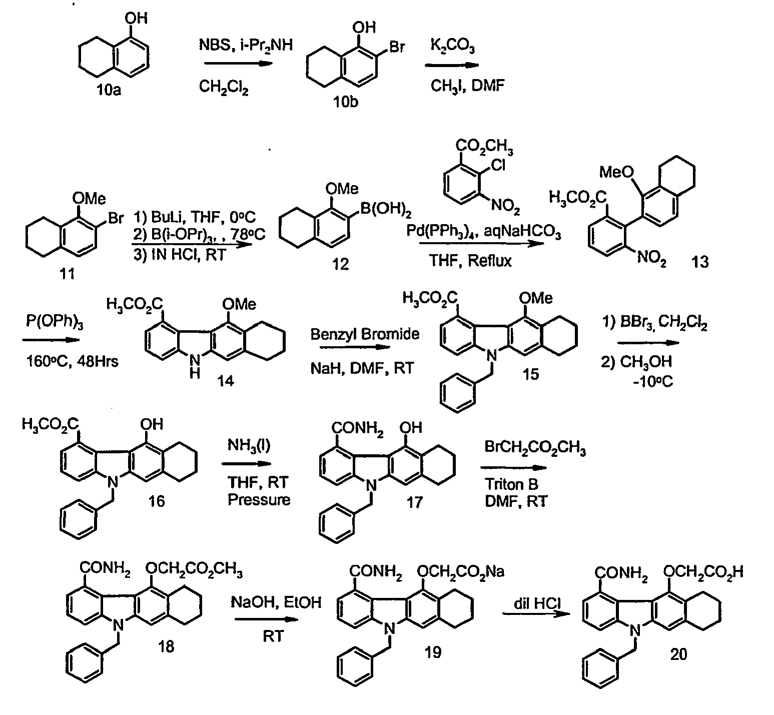 is exemplary for this class of compounds. The starting material 6-methoxy-5-halo-1,2,3,8 tetrahydronaphthalene (11) or analog thereof, is itself prepared from 5,6,7,8-tetrahydronaphthalene (10a) by a halogenation reaction using for example, n-bromosuccinimide to afford the 6-halo compound of formula (10b) or analog thereof. The compound (10b) is then methylated at the hydroxy group to afford the methoxy compound (11). The protocol of Scheme (2) is similar to that of Scheme (1) except for the starting material. The compound (18) is saponified to the sodium salt (19) or converted to other salts and/or hydrolyzed to the free acid (20).

Similarly, compounds of formula I wherein the C and D rings together constitute a naphthyl group, are obtained from the compound (18) prepared as shown in Scheme 2, by aromatization of the D ring with for example, DDQ (2,3-dichloro-5,6-dicyanobenzoquinone - Lancaster Synthesis, Aldrich Chemical Co.). For example, aromatization of compound (18) affords compound (8) of Scheme 1. Compound (8) of scheme 1 can be elaborated to the sodium salt and or acid as shown in Scheme 1. The aromatization reaction to convert compound (18) to compound (8) is performed in a suitable solvent such as 1,4-dioxane at about reflux (about the boiling point of 1,4-dioxane) temperatures for about 1 to 3 hours, preferably 1 hour. A protocol according to Scheme (3) 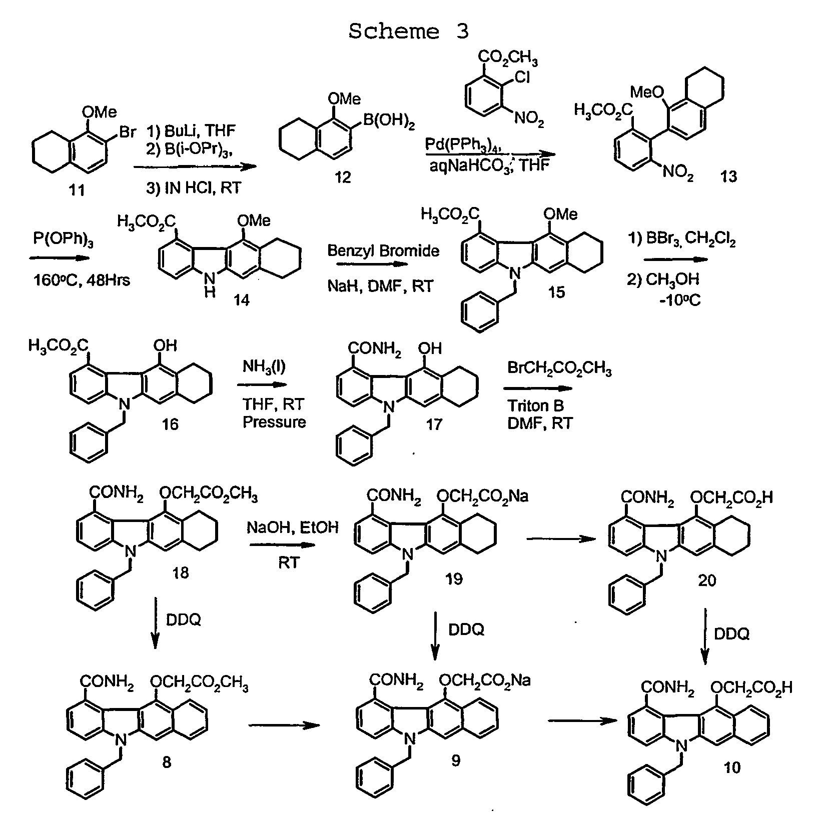 represent the steps to the naphthyl compounds (C+D ring = naphthyl) discussed above. The aromatization reaction results in an isolable intermediate methyl ester compound (a compound of the invention), which is optionally saponified to the intermediate sodium salt and/or hydrolyzed to the free acid compound (20).

Methods for preparing the hydrazide (hydrazone) compounds of the invention include the method represented below in scheme 4: 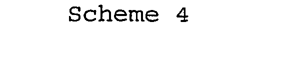 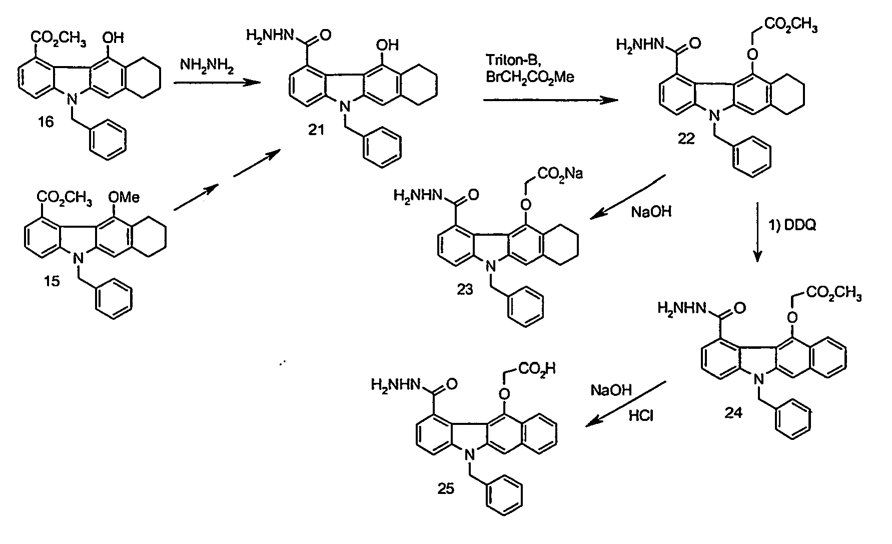 According to scheme 4, the compound (16) obtained as described above (scheme 3) may be converted to a hydrazide (hydrazone) compound of the invention, e.g. compound (21), by reaction with hydrazine. Alternatively compound (15) may be reacted with hydrazine to afford an intermediate hydrazone compound which may be further dealkylated at the (6) position using boron tribromide or sodium thioethoxide as described previously, to form the hydroxy compound (21) . The dealkylated compound (21) is reacted with bromomethyl acetate under basic catalysis conditions using for example, triton-B^{™} as described above, to afford the ester (22). The ester (22) may be hydrolyzed to afford the corresponding acid compound, saponified to the sodium salt, or aromatized to the compound of formula (24). Optionally, the aromatized compound (24) may be converted to the acid (25) by use of a base such as sodium hydroxide or lithium hydroxide in a suitable solvent or solvent mixture followed by acidification (i.e., acid wash).

### The tetracyclic-6-glyoxylamide Compounds or Intermediates

The tetracyclic glyoxylamide intermediates and compounds of the invention may be prepared by reacting 1,2 dichloro-3-nitrobenzene with the compound (12) in place of 1-chloro-2-methoxy-3-nitrobenzene as shown in Scheme 5 below:

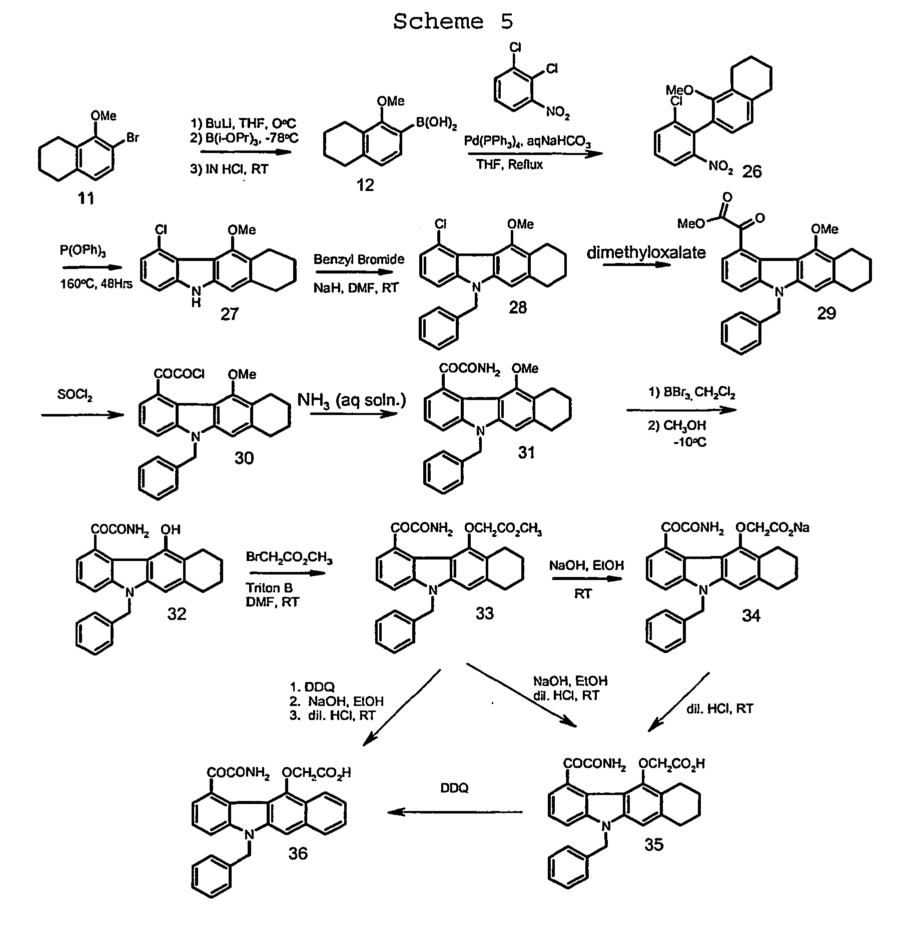

Alternatively, 1-chloro-2-bromo-3-nitrobenzene may be reacted with the compound (12). Compound (12) is prepared as described previously in Scheme (2). In either case a mixture of products is possible and the desired product (26) is isolated and purified by chromatography, distillation, crystallization or a combination of these methods. Again, the Suzuki type coupling is employed to afford the compound (26). The compound (26) is reacted with triphenylphosphite as in Scheme (1) or (2) to afford the reductive cyclization product (27). The compound (27) is alkylated at the carbazole nitrogen atom by reaction with a molar or slight excess, of NaH in a polar aprotic solvent, such as for example dimethylformamide, followed by addition of an electrophile such as for example, benzylbromide, to afford the compound (28). The compound (28) is reacted with n-butyl lithium or other suitable base to afford by a metal-halogen exchange reaction, an un-isolated lithio-intermediate for example. This is followed by addition of dimethyl oxalate to the lithio-intermediate to afford the methyl oxalate compound (29). Reaction of compound (29) with a halogenating agent i.e. anhydrous HCl, thionyl chloride, affords the halogen compound (e.g. chloride (30)). The compound (30) or analog thereof, is reacted with ammonia condensate (condensation of ammonia gas by cooling to form liquid ammonia as discussed previously) to afford the glyoxylamide compound (31). The ammonation may be accomplished by reacting compound (30) and ammonia in a pressure vessel at about 30 to 80 °C for about 10 minutes to 4 hours, followed by appropriate cooling (i.e. about -78 °C), and isolation of product. Compound (31) is then demethylated at the 6 position (or at the 7 position if starting with a compound that places methoxy group at the 7 position). De-methylation is accomplished by reaction with boron tribromide following a procedure described in the examples to afford the compound (32) having a hydroxyl group at position 6 (or 7 as the case may be). The compound (32) may be elaborated to the oxyacetic acid methyl ester derivative (33) by reaction with bromoacetic acid methyl ester (methylbromoacetate) to afford the methyl ester (33). Formation of compound (33) is accomplished in the presence of a phase transfer catalyst such as tetra-n- butylammonium bromide, or triton-B^{™}. The methyl ester (33) may be saponified to the salt i.e. sodium salt (34) using sodium hydroxide or to the potassium salt using potassium hydroxide. Optionally, the salt, i.e. sodium salt (34) may be hydrolyzed to the acid (35) using dilute HCl or other suitable acids. The acid (35) may be aromatized to the naphthyl compound (36). Alternatively, the methylester (33) may be aromatized to an intermediate naphthyl compound followed by saponification and acidification (i.e. sodium hydroxide treatment followed by acid wash) to the acid (36)

### The tetracyclic-5-acetamide compounds

The tetracyclic compounds of the present invention having the acetamide group at position (5) may be prepared as shown in Scheme 6 below. 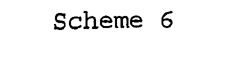 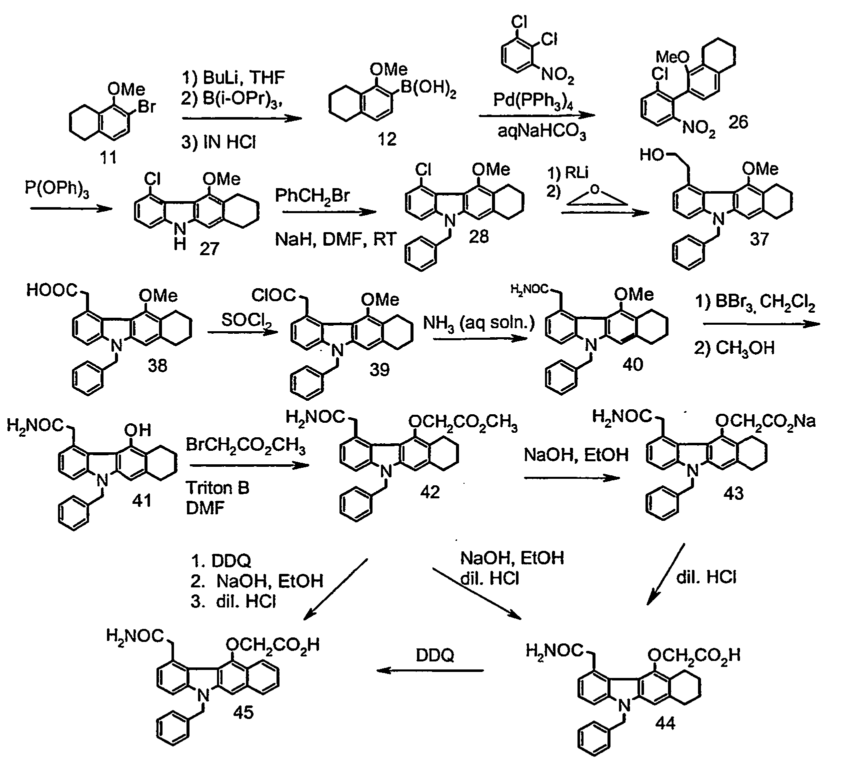 Starting with compound 11, the compounds 26, 27 and 28 are prepared as discussed above according to scheme 5. The compound (28) is subjected to a metal-halogen exchange reaction by the use of n-butyl lithium, t-butyl lithium, or other suitable organic base to afford a lithio intermediate (for example) under inert conditions and polar aprotic solvent(s). To the above *lithio-*intermediate compound is added a terminal epoxide, (i.e. ethylene oxide), to afford the terminal alcohol (37) by a nucleophilic ring opening reaction upon aqueous acidic work-up. The terminal alcohol product (37) is isolated preferably by aqueous work up or by other methods known to one of skill in the art. The terminal alcohol (37) is oxidized to the acid (38), for example by use of sodium hypochlorite or other terminal alcohol oxidizing agents. Other oxidizing agents for the conversion of terminal primary alcohol to the acid are known to one of skill in the art. The acid (38) is converted to an acid chloride (39) by reaction with, for example thionyl chloride, which is then ammoniated to afford the acetamide (40). Alternatively, the acid (38) is converted to the amide i.e. acetamide (40) by reaction with ammonia as described supra or by use of amide forming reagents and conditions known to one of skill in the art or taught in reference literature such as for example, J. March, *Advanced Organic Chemistry,* 3^{rd} edition, John Wiley Publishing NY, NY.

Other methods may include use of reagents for conversion of acids to amides such as the use of activated ester intermediates such as that obtained by use of DCC, DCU, or benzotriazole reagents (R. C. Larock, *Comprehensive Organic Transformations 2*^{*nd*} edition, Wiley-VCH, NY, NY, 1941, (1999)). The acetamide (40) is then demethylated with boron tribromide as described supra to afford the hydroxy compound (41). This is followed by elaboration of the hydroxy group of compound (41) to the oxymethyl acetate derivative (42). This is accomplished for example, by a basic catalysis reaction with methyl bromoacetate and cesium carbonate or TRITON-B^{™} in methylene chloride. The methylester compound (42) may be saponified to the sodium salt (43) followed by hydrolysis to the acid (44) following a procedure described previously. The acid (44) may be aromatized to the naphthyl compound (45).

Alternatively, the methyl ester (42) may be aromatized and hydrolyzed to the acid (45) by a procedure described previously in scheme 5.

### Preparing the Tetracyclic-5-oxime compounds:

The tetracyclic-5-oxime compounds of the invention can be prepared following the protocol of scheme 7 below; 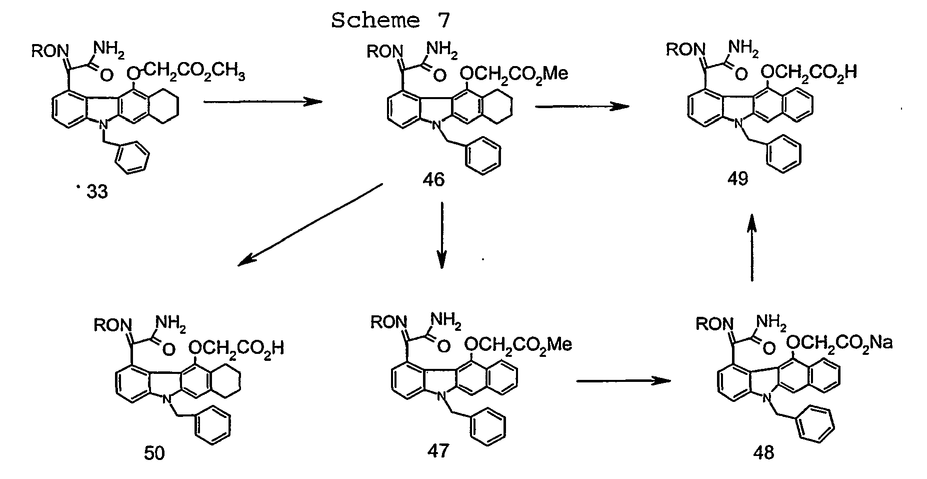 To introduce the oxime functionality, the methyl ester of the tetracyclic glyoxylamide (compound 33 in scheme 5) is heated with hydroxylamine hydrochloride (when R is H) in a THF/methanol mixture for about 1 to 8 hours or until the reaction is deemed complete. The reaction product is isolated by chromatography or other known laboratory procedure. Substituted oximes such as when R is methyl, ethyl, phenyl or other substituent can be prepared by reacting the corresponding substituted hydroxylamine hydrochloride or free base with the glyoxylamide (33) as described supra. The ester functionality at the 6 or 7 position on the resulting tetracyclic nucleus, as in for example, compound (46) can be: (a) converted to the acid by hydrolysis using lithium hydroxide or other known ester hydrolysis methods to afford compound (50); via the sodium salt, or potassium salt, or other metal salt (not shown) depending on the base used; or (b) aromatized to the naphthyl compound (47) by using DDQ under reaction conditions similar to that described in the examples. The compound of formula (47) may than be hydrolyzed to the free acid compound (49) via the sodium salt (48). Compound (46) could be aromatized and then hydrolyzed to afford compound (49) directly.

### The Acylamino acid Compounds

The acylamino derivatives of compounds 9, 19, 20, 23, 25, 35, 36, 43, 44, 48 or 49 representing compounds described above as per schemes 1 through scheme 7. Any of the compounds 9, 19, 20, 23, 25, 35, 36, 43, 44, 48 or 49 each having a oxyacetic acid group at the 6 position may be converted to the corresponding acylamino acid compound by reaction with a C-terminal protected amino acid. Where a C-terminal protected amino acid is used, the protected amide compound or the resulting oxo-amino acid compound (e.g. compound 51 *infra*) is also a compound of the present invention. For example, the tetracyclic-5-glyoxylamide-6-acylamino acid derivative compounds of the invention are prepared by room temperature base catalyzed condensation of the amino acid protected at the C-terminus by a protecting group known in the literature (preferably as the methyl ester), with the tetracyclic-5-glyoxylamide acid derivative compound of formula (35), for example, as shown in Scheme 8 below:

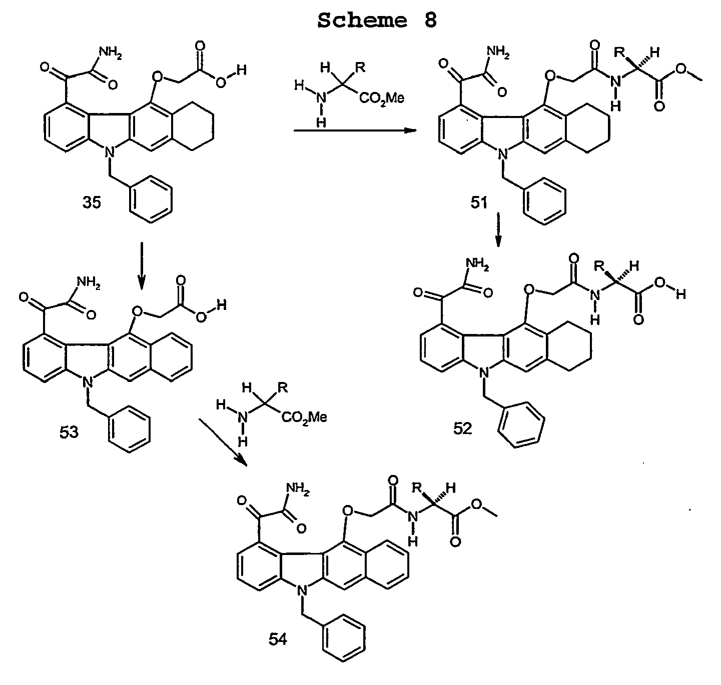

The product of the condensation reaction (51) itself a compound of the invention is hydrolyzed to the free acid, i.e., to remove the amino acid protecting group. Typically, the condensation or coupling is performed in a solvent such as dimethylformamide, tetrahydrofuran or aqueous mixtures of the like. In general protic solvents are preferred for the purpose of this invention. The reaction is base catalyzed, including use of weak organic or inorganic bases. Organic bases such as collidine are preferred. The reaction is also preferably run in the presence of agents that retard or reduce racemization of the amino acid or its derivative, such as for example, benzotriazolyl-N-oxy-tris(dimethylamino)phosphonium hexafluorophosphate. Upon completion of the reaction, the mixture is concentrated in *vacuo.* The resulting product mixture is chromatographed or subjected to crystallization conditions to obtain the target compound (i.e. compound (52)).

The compound (35) could alternatively be aromatized utilizing DDQ as described previously, to afford the compound (53). The compound (53) is then coupled with a protected acylamino acid group as shown to afford the corresponding acylamino acid derivative (54) where the C and D rings are fused to form a naphthalenyl ring system.

Other suitable amino acid forming reactions and methods are applicable to introduce the acylamino acid functionality and are well known in the art. References texts include for example J. March Advanced Organic Chemistry, Wiley Interscience publishers, New York, N.Y, 1985, and R. C. Larock Comprehensive Organic Transformations, VCH Publishers, New York, N.Y, 1989.

Acylamino acid derivatives of the tetracyclic oximes, oxime amide, thioacetamides and acetamides may be prepared from the corresponding acid such as compound (49) (Scheme 7) by methods described above for preparing the acylamino acid derivatives of the glyoxylamide compounds. For example the oxime amide compound (49) (Scheme 7), above may be converted to the corresponding acylamino acid derivative by an amide coupling reaction. Similarly, the tetracyclic-5-acetamide oxyacid compounds (i.e. compound (44)), may be converted to the corresponding acylamino acid derivative at the 6 or 7 position as described previously.

### Preparing the tetracyclic-6-N-hydroxyfunctional amide compounds

The tetracyclic-5-glyoxylamide-6-N-hydroxyfunctional amide compounds of the invention may be prepared from the compounds 10, 20, 23, 25, 35, 36, 43, 44, 48 or 49 representing the tetracyclic-5-amide, the tetracyclic-5-hydrazide, the tetracyclic-5-glyoxylamide, the tetracyclic-5-acetamide and the tetracyclic-5-oxime compounds prepared as described previously. Any of the compounds 10, 20, 25, 31, 38 or 40 each having a oxyacid group at the (6) position may be converted to the corresponding N-hydroxyfunctional amide compounds of the present invention by methods known to one of skill in the art. In the protocol beginning with acid compound (10), the acid (10) is converted to the N-hydroxyfunctional amide compound (55) as shown in Scheme 9 below:

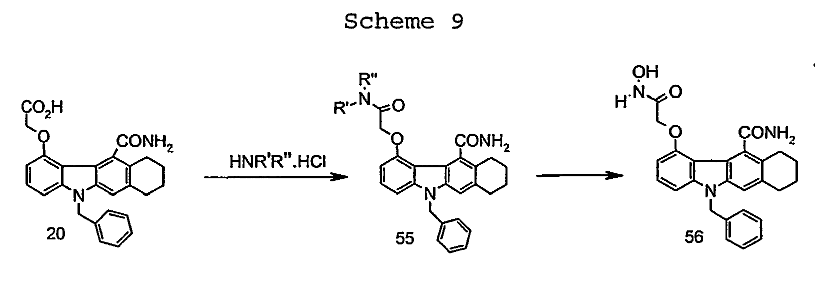

The above transformation may be accomplished by coupling the compound of formula (20), prepared from compound (18) as shown in Scheme 2, with a protected and/or substituted or unsubstituted hydroxylamine group or derivative, in the presence of a coupling agent. This results in a protected N-hydroxyfunctional amide derivative compound (55). For example, the acid compound (20) is reacted with o-(tertbutyldimethylsilyl) hydroxylamine at ambient temperature, in the presence of excess 2,4,6-collidine (collidine) and benzotriazol-1-yl-oxytris(dimethylamino)phosphonium hexafluorophosphonate (coupling catalyst, see *Tetrahedron Lett.*, 1219 (1975)) to afford after about 1-10 hours, the o-(tertbutyldimethylsilyl) substituted N-hydroxyfunctional amide derivative (55). The silyl or other protecting group is removed by well known methods such as for example, the use of trifluoroacetic acid for removal of silyl protecting groups) to afford, for example, the N-hydroxyfunctional amide compound (56) wherein R^{6a} is hydroxy and R^{6b} is hydrogen.

Typically, the condensation or coupling is performed in a solvent such a dimethylformamide, tetrahydrofuran or aqueous mixtures of the like. In general protic solvents are preferred for the purpose of this invention. A base including for example, weak organic or inorganic bases catalyzes the reaction. Organic bases such as collidine are preferred. The reaction is also preferably run in the presence of agents that retard or reduce racemization of the hydroxyfunctional amide, the substituted hydroxylamine or its derivative. A particularly preferred agent is benzotriazolyl-N-oxy-tris(dimethylamino)phosphonium hexafluorophosphate. Upon completion of the reaction, the mixture is concentrated in *vacuo.* The resulting product mixture is chromatographed or crystallized, e.g., by sonication to obtain the target compound.

An alternate preparation method is the interconversion of compounds of the invention as shown for example in Scheme (10):

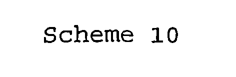

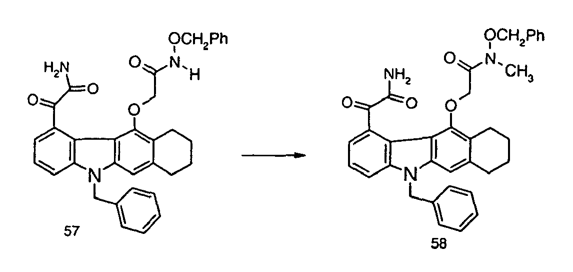

The conversion of a compound of formula (57) to a compound of formula (58) may be accomplished, for example, by base catalyzed alkylation with n-BuLi and methyl iodide. These and other methods of functional group inter-conversions and are well known in the art and can be found in reference texts such as for example J. March Advanced Organic Chemistry, Wiley Interscience publishers, New York, N.Y, 1985, and R. C. Larock Comprehensive Organic Transformations, Wiley-VCH Publishers, New York, NY, 1999.

Other tetracyclic-6-hydroxyfunctional sPLA₂ derivative compounds disclosed herein, including for example, the tetracyclic-5-acetamide-6-hydroxyfunctional amide derivative sPLA₂ inhibitors. These are similarly prepared by condensation of a protected or unprotected, substituted or unsubstituted hydroxylamine or derivative thereof, as discussed above.

### IV. Uses of the Compounds of the Invention:

The tetracyclic compounds described herein are believed to achieve their beneficial therapeutic action principally by direct inhibition of mammalian (including human) sPLA₂, and not by acting as antagonists for arachidonic acid, nor other active agents below arachidonic acid in the arachidonic acid cascade, such as 5-lipoxygenases, cyclooxygenases, and etc.

The use of the invention for inhibiting sPLA₂ mediated release of fatty acids comprises contacting mammalian sPLA₂ with a therapeutically effective amount of tetracyclic compounds corresponding to Formulae (I) or (II) or (III) or (IV) or (V) or (VI) or (VII) or (VIII) as described herein including a combination thereof, a salt or a prodrug derivative thereof.

Another aspect of this invention relates to the use of a tetracyclic compound of the invention for the manufacture of a medicament for treating Inflammatory Diseases such as inflammatory bowel disease, septic shock, adult respiratory distress syndrome, pancreatitis, trauma, asthma, bronchial asthma, allergic rhinitis, rheumatoid arthritis, osteoarthritis, and related diseases.

As previously noted the compounds of this invention are useful for inhibiting sPLA₂ mediated release of fatty acids such as arachidonic acid. By the term, "inhibiting" is meant the prevention or therapeutically significant reduction in release of sPLA₂ initiated fatty acids by the compounds of the invention. By "pharmaceutically acceptable" it is meant the carrier, diluent or excipient must be compatible with the other ingredients of the formulation and not deleterious to the recipient thereof.

The specific dose of a compound administered according to this invention to obtain therapeutic or prophylactic effect will, of course, be determined by the particular circumstances surrounding the case, including, for example, the compound administered, the route of administration and the condition being treated. Typical daily doses will contain a non-toxic dosage level of from about 0.01 mg/kg to about 50 mg/kg of body weight of an active compound of this invention.

Preferably compounds of the invention per Formula (I) or (II) or (III) or (IV) or (V) or (VI) or (VII) or (VIII) or pharmaceutical formulations containing these compounds are in unit dosage form for administration to a mammal. The unit dosage form can be a capsule or tablet itself, or the appropriate number of any of these. The quantity of Active Ingredient in a unit dose of composition may be varied or adjusted from about 0.1 to about 1000 milligrams or more according to the particular treatment involved. It may be appreciated that it may be necessary to make routine variations to the dosage depending on the age and condition of the patient. The dosage will also depend on the route of administration.

The compound can be administered by a variety of routes including oral, aerosol, rectal, transdermal, subcutaneous, intravenous, intramuscular, and intranasal.

Pharmaceutical formulations of the invention are prepared by combining (e.g., mixing) a therapeutically effective amount of the tetracyclic compound of the invention together with a pharmaceutically acceptable carrier or diluent therefor. The present pharmaceutical formulations are prepared by known procedures using well-known and readily available ingredients.

In making the compositions of the present invention, the Active ingredient will usually be admixed with a carrier, or diluted by a carrier, or enclosed within a carrier which may be in the form of a capsule, sachet, paper or other container. When the carrier serves as a diluent, it may be a solid, semisolid or liquid material which acts as a vehicle, or can be in the form of tablets, pills, powders, lozenges, elixirs, suspensions, emulsions, solutions, syrups, aerosols (as a solid or in a liquid medium), or ointment, containing, for example, up to 10% by weight of the active compound. The compounds of the present invention are preferably formulated prior to administration.

For the pharmaceutical formulations any suitable carrier known in the art can be used. In such a formulation, the carrier may be a solid, liquid, or mixture of a solid and a liquid. For example, for intravenous injection the compounds of the invention may be dissolved in at a concentration of 2 mg/ml in a 4% dextrose/0.5% Na citrate aqueous solution. Solid form formulations include powders, tablets and capsules. A solid carrier can be one or more substance, which may also act as flavoring agents, lubricants, solubilizers, suspending agents, binders, tablet disintegrating agents and encapsulating material.

Tablets for oral administration may contain suitable excipients such as calcium carbonate, sodium carbonate, lactose, calcium phosphate, together with disintegrating agents, such as maize, starch, or alginic acid, and/or binding agents, for example, gelatin or acacia, and lubricating agents such as magnesium stearate, stearic acid, or talc. A preferred tablet formulation for oral administration is one that affords rapid dissolution in the mouth of a patient in need thereof.

In powders the carrier is a finely divided solid which is in admixture with the finely divided Active ingredient. In tablets the Active ingredient is mixed with a carrier having the necessary binding properties in suitable proportions and compacted in the shape and size desired. The powders and tablets preferably contain from about 1 to about 99 weight percent of the Active ingredient which is the novel compound of this invention. Suitable solid carriers are magnesium carbonate, magnesium stearate, talc, sugar lactose, pectin, dextrin, starch, gelatin, tragacanth, methyl cellulose, sodium carboxymethyl cellulose, low melting waxes, and cocoa butter.

Sterile liquid form formulations include suspensions, emulsions, syrups and elixirs.

The Active ingredient can be dissolved or suspended in a pharmaceutically acceptable carrier, such as sterile water, sterile organic solvent or a mixture of both. The Active ingredient can often be dissolved in a suitable organic solvent, for instance aqueous propylene glycol. Other compositions can be made by dispersing the finely divided Active ingredient in aqueous starch or sodium carboxymethyl cellulose solution or in a suitable oil.

The following pharmaceutical formulations 1 through 8 are illustrative only and are not intended to limit the scope of the invention in any way. "Active ingredient", refers to a compound according to Formula (I) or (II) or (III) or (IV) or (V) or (VI) or (VII) or (VIII) or a pharmaceutically acceptable salt, solvate, or prodrug thereof.

### Formulation 1.

Hard gelatin capsules are prepared using the following ingredients:

| | Quantity (mg/capsule) |
|---|---|
| Active ingredient | 250 |
| Starch, dried | 200 |
| Magnesium stearate | 10 |
| Total | 460 mg |

### Formulation 2

A tablet is prepared using the ingredients below:

| | Quantity (mg/tablet) |
|---|---|
| Active ingredient | 250 |
| Cellulose, microcrystalline | 400 |
| Silicon dioxide, fumed | 10 |
| Stearic acid | 5 |
| Total | 665 mg |

The components are blended and compressed to form tablets each weighing 665 mg.

### Formulation 3

An aerosol solution is prepared containing the following components:

| | Weight |
|---|---|
| Active ingredient | 0.25 |
| Ethanol | 25.75 |
| Propellant 22 (Chlorodifluoromethane) | 74.00 |
| Total | 100.00 |

The active compound is mixed with ethanol and the mixture added to a portion of the propellant 22, cooled to -30°C and transferred to a filling device. The required amount is then fed to a stainless steel container and diluted with the remainder of the propellant. The valve units are then fitted to the container.

### Formulation 4

Tablets, each containing 60 mg of Active ingredient, are made as follows:

| | |
|---|---|
| Active ingredient | 60 mg |
| Starch | 45 mg |
| Microcrystalline cellulose | 35 mg |
| Polyvinylpyrrolidone (as 10% solution in water) | 4 mg |
| Sodium carboxymethyl starch | 4.5 mg |
| Magnesium stearate | 0.5 mg |
| Talc | 1 mg |
| Total | 150 mg |

The Active ingredient, starch and cellulose are passed through a No. 45 mesh U.S. sieve and mixed thoroughly. The aqueous solution containing polyvinylpyrrolidone is mixed with the resultant powder, and the mixture then is passed through a No. 14 mesh U.S. sieve. The granules so produced are dried at 50°C and passed through a No. 18 mesh U.S. sieve. The sodium carboxymethyl starch, magnesium stearate and talc, previously passed through a No. 60 mesh U.S. sieve, are then added to the granules which, after mixing, are compressed on a tablet machine to yield tablets each weighing 150 mg.

### Formulation 5

Capsules, each containing 80 mg of Active ingredient, are made as follows:

| | |
|---|---|
| Active ingredient | 80 mg |
| Starch | 59 mg |
| Microcrystalline cellulose | 59 mg |
| Magnesium stearate | 2 mg |
| Total | 200 mg |

The Active ingredient, cellulose, starch, and magnesium stearate are blended, passed through a No. 45 mesh U.S. sieve, and filled into hard gelatin capsules in 200 mg quantities.

### Formulation 6

Suppositories, each containing 225 mg of Active ingredient, are made as follows:

| | |
|---|---|
| Active ingredient | 225 mg |
| Saturated fatty acid glycerides | 2,000 mg |
| Total | 2,225 mg |

The Active ingredient is passed through a No. 60 mesh U.S. sieve and suspended in the saturated fatty acid glycerides previously melted using the minimum heat necessary. The mixture is then poured into a suppository mold of nominal 2g capacity and allowed to cool.

### Formulation 7

Suspensions, each containing 50 mg of Active ingredient per 5 ml dose, are made as follows:

| | |
|---|---|
| Active ingredient | 50 mg |
| Sodium carboxymethyl cellulose | 50 mg |
| Syrup | 1.25 ml |
| Benzoic acid solution | 0.10 ml |
| Flavor | q.v. |
| Color | q.v. |
| Purified water to total | 5 ml |

The Active ingredient is passed through a No. 45 mesh U.S. sieve and mixed with the sodium carboxymethyl cellulose and syrup to form a smooth paste. The benzoic acid solution, flavor and color are diluted with a portion of the water and added, with stirring. Sufficient water is then added to produce the required volume.

### Formulation 8

An intravenous formulation may be prepared as follows:

| | |
|---|---|
| Active ingredient | 100 mg |
| Isotonic saline | 1,000 ml |

The solution of the above ingredients generally is administered intravenously to a subject at a rate of 1 ml per minute.

### Assay

The following chromogenic assay procedure was used to identify and evaluate inhibitors of recombinant human secreted phospholipase A₂. The assay described herein has been adapted for high volume screening using 96 well microtiter plates. A general description of this assay method is found in the article, "Analysis of Human Synovial Fluid Phospholipase A₂ on Short Chain Phosphatidylcholine-Mixed Micelles: Development of a Spectrophotometric Assay Suitable for a Microtiterplate Reader", by Laure J. Reynolds, Lori L. Hughes, and Edward A. Dennis, Analytical Biochemistry, 204, pp. 190-197, 1992 (the disclosure of which is incorporated herein by reference):

### Reagents:

### REACTION BUFFER -

| | |
|---|---|
| CaCl₂·2H₂O | (1.47 g/L) |
| KCl | (7.455 g/L) |

| | |
|---|---|
| Bovine Serum Albumin (fatty acid free) | (1 g/L) |
| (Sigma A-7030, product of Sigma Chemical Co., St. Louis MO, USA) | |
| TRIS HCl | (3.94 g/L) |
| pH 7.5 (adjust with NaOH) | |

### ENZYME BUFFER -

0.05 NaOAc.3H₂O, pH 4.5
0.2 NaCl
Adjust pH to 4.5 with acetic acid
DTNB - 5,5'-dithiobis-2-nitrobenzoic acid
RACEMIC DIHEPTANOYL THIO - PC
racemic 1,2-bis(heptanoylthio)-1,2-dideoxy-*sn*- glycero-3-phosphorylcholine
TRITON X-100^{™} prepare at 6.249 mg/ml in reaction buffer to equal 10uM.

### REACTION MIXTURE -

A measured volume of racemic dipheptanoyl thio PC supplied in chloroform at a concentration of 100 mg/ml is taken to dryness and redissolved in 10 millimolar TRITON X-100^{™} nonionic detergent aqueous solution. Reaction Buffer is added to the solution, then DTNB to give the Reaction Mixture.

The reaction mixture thus obtained contains 1mM diheptanoly thio-PC substrate, 0.29 mm Triton X-100^{™} detergent, and 0.12 mm DTMB in a buffered aqueous solution at pH 7.5. Assay Procedure:
1. Add 0.2 ml reaction mixture to all wells;
2. Add 10 ul test compound (or solvent blank) to appropriate wells, mix 20 seconds;
3. Add 50 nanograms of sPLA₂ (10 microliters) to appropriate wells;
4. Incubate plate at 40 °C for 30 minutes;
5. Read absorbance of wells at 405 nanometers with an automatic plate reader.

Tests were done in triplicate. Typically, compounds were tested at a final concentration of 5 ug/ml. Compounds were considered active when they exhibited 40% inhibition or greater compared to uninhibited control reactions when measured at 405 nanometers. Lack of color development at 405 nanometers evidenced inhibition. Compounds initially found to be active were re-assayed to confirm their activity and, if sufficiently active, IC₅₀ values were determined. Typically, the IC₅₀ values (see, Table I, below) were determined by diluting test compound serially two-fold such that the final concentration in the reaction ranged from 45 ug/mL to 0.35 ug/ml. More potent inhibitors required significantly greater dilution. In all cases, % inhibition measured at 405 nanometers generated by enzyme reactions containing inhibitors relative to the uninhibited control reactions was determined. Each sample was titrated in triplicate and result values were averaged for plotting and calculation of IC₅₀ values. IC₅₀ values were determined by plotting log concentration versus inhibition values in the range from 10-90% inhibition.

### Results

| Compound of Example# | IC₅₀ (uM)(micromolar) |
|---|---|
| 1 | 38.6 |
| 2 | 410 |

While the present invention has been illustrated above by certain specific embodiments, it is not intended that these specific examples should limit the scope of the invention as described in the appended claims.

### Experimental

All of the products of the Examples described below as well as intermediates used in the following procedures showed satisfactory NMR and IR spectra. They also had the correct mass spectral values.

### Example 1

### (5-Benzyl-1-carbamoyl-5H-benzo[b]carbazol-11-yloxy)-acetic acid, sodium salt.

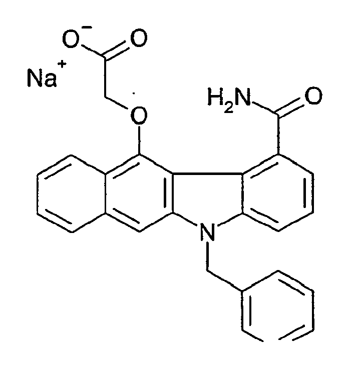

### 1. Preparation of 1-Methoxynaphthalene-2-boronic acid

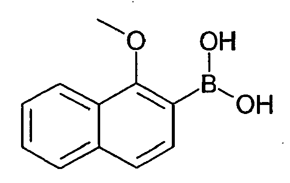

To a stirred mixture of 1-methoxynaphthalene (20.0 g, 126.6 mmol) in THF (100 mL) was added n-butyllithium (53.16 mL, 132.9 mmol, 2.5M solution in hexanes) at room temperature over 5 min. The reaction mixture was cooled to -50°C, and triisopropyl borate (43.8 mL, 35.7 g, 190 mmol) was added dropwise over 0.25 h. The resulting precipitate was broken up by the addition of THF (100 mL), and the mixture was allowed to warm to 0°C with stirring. At this point the mixture was poured into 1N HCl (100 mL), the product extracted with ethyl acetate, the combined extracts dried over anhydrous sodium sulfate, and filtered. After concentrating the filtrate in vacuo at ambient temperature, the residue was partially dissolved in diethyl ether, triturated with hexane, and the resulting precipitate filtered and washed with hexane to afford 10.64 g (41%) of the title compound 1-methoxynaphthalene-2-boronic acid as an off-white crystalline solid (MW 202.02, C₁₁H₁₁BO₃).

¹H NMR (DMSO-d₆) δ 8.08-8.06 (m, 1H), 7.87-7.84 (m, 1H), 7.56 (d, 1H, J=8 Hz), 7.50-7.46 (m, 3H), 6.51 (br s, 2H), and 3.91 (s, 3H).

### 1a. Preparation of 2-Chloro-3-nitrobenzoic acid, methyl ester

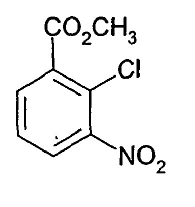

A solution of 2-chloro-3-nitrobenzoic acid (20.16 g, 100 mmol), iodomethane (15.6 g, 110 mmol), and potassium carbonate (15.0 g, 108.5 mmol) in DMF (100 mL) was stirred at room temperature for 48 h. The mixture was poured into 1.5 liters of H₂O. The resultant precipitate was collected by filtration, washed with H₂O, and dried in vacuo to afford 20.0 g (93%) of the title compound 2-chloro-3-nitrobenzoic acid, methyl ester as a white solid (MW 215.59).

¹H NMR (CDCl₃) δ 8.42 (dd, 1H, J=1 and 8 Hz), 8.18 (dd, 1H, J=1 and 8 Hz), 7.43 (t, 1H, J=8 Hz), and 3.9 (s, 3H). IR (KBr, cm⁻¹) 1743, 1719, 1595, 1540, 1532, 1433, 1357, 1300, and 730. MS (FD) m/e 215, 216. Anal. Calcd. for C₈H₆NClO₄: C, 44.57; H, 3.81; N, 6.50. Found C, 44.19; H, 3.45; N, 6.19.

### 2. Preparation of 2-(1-Methoxynaphthalen-2-yl)-3-nitrobenzoic acid, methyl ester

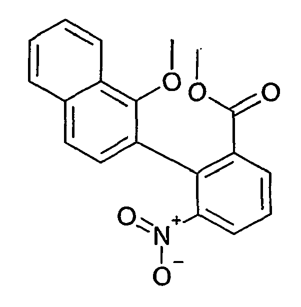

A solution of 2-chloro-3-nitrobenzoic acid methyl ester, (2.16 g, 10.0 mM), 1-methoxynaphthalene-2-boronic acid, (2.22 g, 11.0 mM), tetrakis(triphenylphosphine) palladium(0) (0.584 g, 0.5 mM), and 2M aqueous sodium carbonate solution (10.5 mL, 21.0 mM) in THF (50 mL) was stirred at reflux for 31 h in the absence of light. The mixture was cooled to room temperature, the THF removed in vacuo, and the resulting aqueous residue dissolved in ethyl acetate and brine. The solvent layers were separated, the aqueous layer extracted with ethyl acetate (4x25 mL), the combined ethyl acetate extracts washed successively with H₂O, 1N HCl, saturated aqueous NaHCO₃ solution, and saturated brine, dried over magnesium sulfate, filtered, and concentrated in vacuo. The resultant oil was purified by column chromatography on silica gel (elution with 1:1 chloroform/toluene) to afford 2.54 g (75%) of the title compound 2-(1-methoxynaphthalen-2-yl)-3-nitrobenzoic acid, methyl ester as a yellow solid (MP 139-141 °C, MW 337.34).

¹H NMR (CDCl₃) δ 8. 13-8.11 (m, 1H), 8.08 (d, 1H, J=9 Hz), 8.00 (d, 1H, J=9 Hz), 7.86-7.84 (m, 1H), 7.64 (d, 1H, J=9 Hz), 7.60 (d, 1H, J=8 Hz), 7.52-7.49 (m, 2H), 7.21 (d, 1H, J=8 Hz), 3.61 (s, 3H), and 3.53 (s, 3H). IR (KBr, cm⁻¹) 3020-2830 (multiple peak grouping), 1737, 1531, 1369, 1277, 1109, and 757. MS (ESI) m/e 306, 338, 360. Anal. Calcd for C₁₉H₁₅NO₅: C, 67.65; H, 4.48; N, 4.15. Found C, 65.77; H, 4.31; N, 4.00.

### 3. Preparation of 11-Methoxy-5H-benzo[b]carbazole-1-carboxylic acid, methyl ester

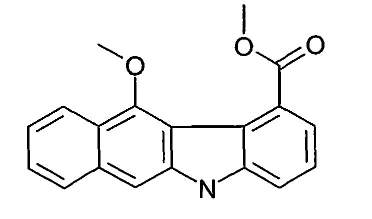

A solution of 2-(1-methoxynaphthalen-2-yl)-3-nitrobenzoic acid, methyl ester, (1.52 g, 4.5 mM) in triphenyl phosphite (7.19 g, 6.1 mL, 22.5 mM) was heated at 160 °C for 19 h under a nitrogen atmosphere. The mixture was cooled to room temperature and dried azeotropically in vacuo with toluene, then purified by column chromatography on silica gel (elution with gradient hexane/ethyl acetate) to afford 0.37 g (27%) of the title compound 11-methoxy-5H-benzo[b]carbazole-1-carboxylic acid, methyl ester as a yellow solid (MP 190-196 °C, MW 305.34).

¹H NMR (CDCl₃) δ 8.27 (d, 1H, J=9 Hz), 8.08 (br s, 1H), 7.89 (d, 1H, J=9 Hz), 7.54 (s, 1H), 7.50-7.45 (m, 3H), 7.41 (d, 1H, J=7 Hz), 7.32 (d, 1H, J=8 Hz), 4.03 (s, 3H), and 3.91 (s, 3H). IR (CHCl₃, cm⁻¹) 3469, 3030-2850 (multiple peak grouping), 1725, 1641, 1606, 1433, 1400, 1343, 1303, 1288, 1170, 1141, and 1090. MS (ESI) m/e 274, 304, and 306. Anal. Calcd for C₁₉H₁₅NO₃: C, 74.74; H, 4.95; N, 4.59. Found C, 74.02; H, 5.02; N, 4.17.

### 4. Preparation of 5-Benzyl-11-methoxy-5H-benzo[b]carbazole-1-carboxylic acid, methyl ester

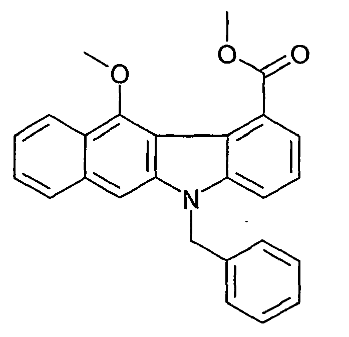

A solution of 11-methoxy-5H-benzo[b]carbazole-1-carboxylic acid, methyl ester, (0.458 g, 1.5 mM) in 5 mL DMF was added to a 60% NaH mineral oil dispersion (0.12 g, 3.0 mM, washed twice with hexane) at room temperature. Following cessation of gas evolution, benzyl bromide (0.20 mL, 0.288 g, 1.65 mM) was added and the mixture stirred at room temperature for 4 hours. The mixture was diluted with ethyl acetate and H₂O, the solvent layers separated, and the aqueous layer extracted with ethyl acetate (4x25 mL). The combined ethyl acetate extracts were washed with H₂O, 1N HCl, saturated aqueous NaHCO₃ solution, and saturated brine, dried over magnesium sulfate, filtered, concentrated in vacuo, and triturated with hexanes. The resultant yellow-orange gum was purified by column chromatography on silica gel (elution with 9:1 toluene/ethyl acetate) to afford 0.51 g (86%) of the title compound 5-benzyl-11-methoxy-5H-benzo[b]carbazole-1-carboxylic acid, methyl ester as a yellow-orange foam (MW 395.46).

¹H NMR (CDCl₃) δ 8.28 (d, 1H, J=9 Hz), 7.88 (d, 1H, J=9 Hz), 7.51-7.17 (m, 11H), 5.55 (s, 2H), 4.04 (s, 3H), and 3.94 (s, 3H). IR (C_{H}Cl₃, cm⁻¹) 3100-2850 (multiple peak grouping), 1725, 1634, 1596, 1453, 1346, 1310, 1287, 1171, and 1114. MS (ESI) m/e 364, 396. MS (FD) m/e 395. Anal. Calcd for C₂₆H₂₁NO₃: C, 78.97; H, 5.35; N, 3.54. Found C, 78.38; H, 5.27; N, 3.53.

### 5. Preparation of 5-Benzyl-11-hydroxy-5H-benzo[b]carbazole-1-carboxylic acid, methyl ester

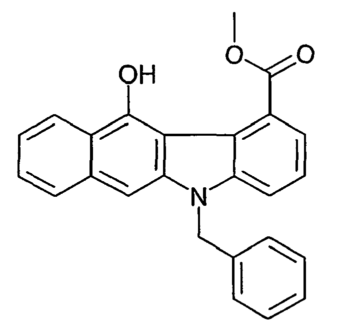

Boron tribromide (0.148 mL, 1.56 mM) was slowly added to a stirred solution of 5-benzyl-11-methoxy-5H-benzo [b] carbazole-1-carboxylic acid, methyl ester, (0.474 g, 1.2 mM) in 8 mL methylene chloride at -10 °C. After 1.5 h, the mixture was quenched with methanol (1.22 mL, 30.0 mM) and allowed to warm to room temperature with stirring over 2 h. The mixture was diluted with methylene chloride, washed with H₂O, 1N HCl, saturated aqueous NaHCO₃ solution, and saturated brine, dried over magnesium sulfate, filtered, and concentrated in vacuo. The resultant orange-brown solid was purified by column chromatography on silica gel (elution with gradient hexane/ethyl acetate) to afford 0.245 g (47%) of the title compound 5-benzyl-11-hydroxy-5H-benzo[b]carbazole-1-carboxylic acid, methyl ester as an orange foam (MW 381.44) .

¹H NMR (CDCl₃) δ 11.14 (br s, 1H), 8.60 (d, 1H, J=8 Hz), 7.94 (br s, 1H), 7.75 (br s, 1H), 7.51-7.41 (m, 2H), 7.37-7.33 (t, 1H, J=7 Hz), 7.26-7.21 (m, 5H), 7.12 (d, 2H, J=6 Hz), 5.55 (s, 2H), and 4.15 (s, 3H). IR (KBr, cm⁻¹) 3420 (br), 3050-2940 (multiple peak grouping), 1727, 1652, 1628, 1452, 1438, 1279, 1197, 1179, and 747. MS (ESI) m/e 350, 364, 380, and 382. Anal. Calcd for C₂₅H₁₉NO₃: C, 78.72; H, 5.02; N, 3.67. Found C, 77.43; H, 4.78; N, 3.55.

### 6. Preparation of 5-Benzyl-11-hydroxy-5H-benzo[b]carbazole-1-carboxylic acid amide

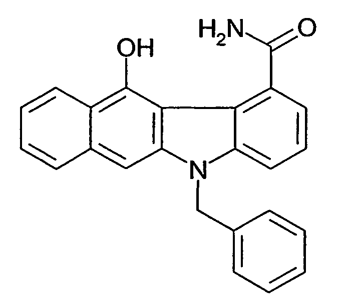

A solution of 5-benzyl-11-hydroxy-5H-benzo[b]carbazole-1-carboxylic acid, methyl ester, (0.229 g, 0.60 mM) in 8 mL THF was placed in an Ace Glass pressure tube (203 mm x 38 mm) with a small stirring bar. A N₂(g) stream was installed over the solution surface. The pressure tube and reaction solution were cooled to -78 °C, and - 8 mL NH₃(1) was condensed into the reaction vessel by introduction of an NH₃(g) stream. The pressure tube was sealed with a teflon O-ring screw cap, the resultant orange solution stirred at -78 °C for 10 minutes, then the cooling bath was removed and the reaction solution allowed to warm to ambient temperature behind a blast shield. After 48 h, the pressure tube was re-cooled to - 78 °C and the internal NH₃(g) pressure was released by careful removal of the Teflon^{Tm} cap. The reaction solution was allowed to slowly warm to room temperature while NH₃(g) bubbled off, then the solution was concentrated in vacuo to afford 0.22 g (99%) of the title compound 5-benzyl-11-hydroxy-5H-benzo[b]carbazole-1-carboxylic acid amide as a yellowish-green solid (MW 366.42).

¹H NMR (DMSO-d6) δ 11.52 (s, 1H), 9.08 (br s, 1H), 8.63 (br s, 1H), 8.37 (d, 1H, J=8 Hz), 7.80 (d, 2H, J=8 Hz), 7.73 (d, 1H, J=6 Hz), 7.53-7.42 (m, 5H), 7.29-7.11 (m, 4H), and 5.69 (s, 2H). IR (KBr, cm⁻¹) 3480-2920 (multiple peak grouping), 1725, 1658, 1645, 1631, 1594, 1578, 1440, 1295, and 749. MS (ESI) m/e 350, 365, and 367. Anal. Calcd for C₂₄H₁₈N₂O₂: C, 78.67; H, 4.95; N, 7.65. Found C, 76.10; H, 5.17; N, 6.20.

### 7. Preparation of (5-Benzyl-l-carbamoyl-5H-benzo[b]carbazol-11-yloxy)-acetic acid, methyl ester

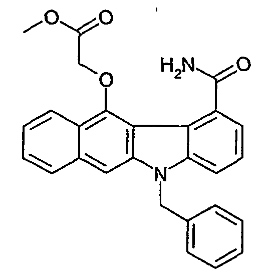

40% Methanolic Triton B (0.378 mL, 0.832 mM) was added to a solution of 5-benzyl-11-hydroxy-5H-benzo[b]carbazole-1-carboxylic acid amide, (0.235 g, 0.64 mM) in 5 mL DMF at room temperature. After 2 minutes, methyl bromoacetate (0.125 mL, 0.202 g, 1.28 mM) was added and the resultant mixture stirred at room temperature for 2 h. Cesium carbonate (0.105 g, 0.32 mM) was then added as a solid, and the mixture allowed to stir at room temperature for an additional 1 h. The mixture was diluted with ethyl acetate, washed with H₂O, 1N HCl, saturated aqueous NaHCO₃ solution, and saturated brine, dried over magnesium sulfate, filtered, and concentrated in vacuo. The resulting brown residue was purified by column chromatography on silica gel (elution with ethyl acetate) to afford 0.151 g (53%) of the title compound (5-benzyl-1-carbamoyl-5H-benzo [b] carbazol-11-yloxy)-acetic acid, methyl ester as a brown foam (MP 120-125 °C, MW 438.49).

¹H NMR (CDCl₃) δ 8.30 (d, 1H, J=9 Hz), 8.05 (d, 1H, J=9 Hz), 7.51-7.46 (m, 3H), 7.36-7.26 (m, 7H), 7.18 (d, 1H, J=8 Hz), 5.91 (br s, 2H), 5.81 (s, 2H), 4.72 (s, 2H), and 3.72 (s, 3H). IR (KBr, cm⁻¹) 3430, 3330, 3150-2830 (multiple peak grouping), 1735, 1659, 1595, 1436, 1393, 1213, 1158, and 750. MS (ESI) m/e 422, 439. MS (FD) m/e 438. Anal. Calcd for C₂₇H₂₂N₂O₄: C, 73.96; H, 5.06; N, 6.39. Found C, 70.52; H, 5.39; N, 5.42.

### Preparation of (5-Benzyl-1-carbamoyl-5H-benzo[b]carbazol-11-yloxy)-acetic acid, sodium salt

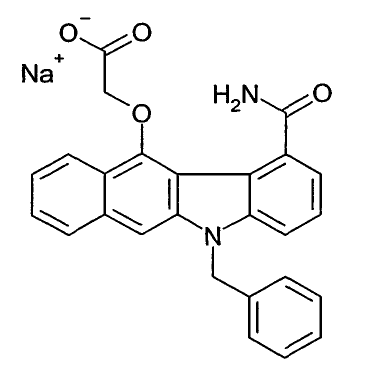

A solution of (5-benzyl-1-carbamoyl-5H-benzo[b]carbazol-11-yloxy)-acetic acid, methyl ester, (0.088 g, 0.2 mM) and 1N NaOH (0.22 mL, 0.22 mM) in 5 mL of ethanol was stirred for 3.5 h at 25 °C. A small volume of diethyl ether/hexanes was added, then the mixture was cooled in the refrigerator. The resultant yellowish-brown precipitate was collected by filtration, washed with a small amount of EtOH/diethyl ether/hexanes, then dried in vacuo to afford 0.065 g (73%) of the title compound (5-benzyl-1-carbamoyl-5H-benzo[b]carbazol-11-yloxy)-acetic acid, sodium salt as a brown solid (MW 446.44, exact mass minus sodium 423.13).

¹H NMR (DMSO-d6) δ 8.44 (d, 1H, J=8 Hz), 8.23 (br s, 1H), 7.97 (d, 1H, J=8 Hz), 7.51-7.38 (m, 4H), 7.36-7.06 (m, 8H), 5.78(s, 2H), and 4.18 (s, 2H). IR (KBr, cm⁻¹) 3426, 3061, 1728, 1664, 1594, 1495, 1419, 1380, 1347, 1321, 1305, 1264, 1216, 1157, 1013, 750, and 696. MS (ESI) m/e 425. MS (FAB) m/e 447. Anal. Calcd for C₂₆H₁₉N₂NaO₄: C, 69.95; H, 4.29; N, 6.27. Found C, 65.21; H, 4.42; N, 5.06.

### Example 2

### (5-Benzyl-1-carbamoyl-7,8,9,10-tetrahydro-5H-benzo[b]carbazol-11-yloxy)-acetic acid, sodium salt

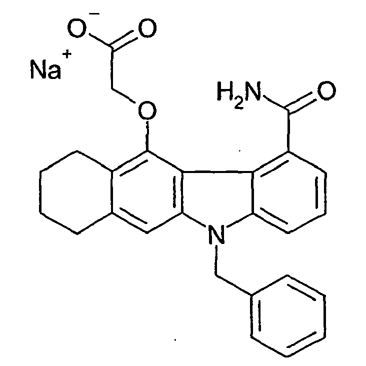

### 1. Preparation of 2-Bromo-5,6,7,8-tetrahydronaphthalen-1-ol

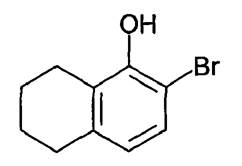

To a stirred mixture of 5,6,7,8-tetrahydronaphthalen-1-ol (29.2 g, 195 mmol) in dichloromethane (300 mL) was added diisopropylamine (2.75 mL, 19.5 mmol) at room temperature. The reaction mixture was cooled to 0°C, and N-bromosuccinimide (36.62g, 205 mmol) dissolved in dichloromethane (1.0 L) was added dropwise over 6.5 h. The mixture was then allowed to stir at room temperature for an additional 1 h. At this point 1N HCl was slowly added until the mixture reached pH 1, added H₂O (100 mL), separated the dichloromethane solvent layer, dried the organic extracts over anhydrous sodium sulfate, and filtered. Concentrated the filtrate in vacuo at ambient temperature to produce a clear oil, added hexane (1.0 L) to precipitate unreacted N-bromosuccinimide, collected the resulting precipitate and washed with hexane. The filtrate was concentrated in vacuo, and the resultant oil purified by column chromatography on silica gel (elution with 2.5% ethyl acetate/hexane) to afford 32.3 g (73%) of the title compound of 2-bromo-5,6,7,8-tetrahydronaphthalen-1-ol as an off-white solid (MW 227.10, C₁₀H₁₁BrO) .

¹H NMR (DMSO-d₆) δ 8.73 (s, 1H), 7.13 (d, 1H, J=8 Hz), 6.46 (d, 1H, J=8 Hz), 2.56-2.55 (m, 4H), and 1.69-1.58 (m, 4H). MS (ESI) m/e 225, 227 (negative ions).

### 2. Preparation of G-Bromo-5-methoxy-1,2,3,4-tetrahydronaphthalene

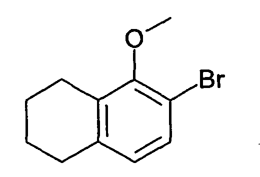

A solution of 2-bromo-5,6,7,8-tetrahydronaphthalen-1-ol, (28 g, 123.3 mmol), iodomethane (23.09 mL, 52.66 g, 371 mmol), and potassium carbonate (51.52 g, 373.3 mmol) in DMF (450 mL) was stirred at room temperature for 48 h. The mixture was poured into H₂O (500 mL) and extracted with ethyl acetate (4x250 mL). The organic extracts were combined, washed with H₂O (4x250 mL), dried over anhydrous sodium sulfate, filtered, and concentrated in vacuo to afford 29.6 g (quantitative yield) of the title compound 6-bromo-5-methoxy-1,2,3,4-tetrahydronaphthalene as a pale yellow oil (MW 241.13, C₁₁H₁₃BrO).

¹H NMR (DMSO-d₆) δ 7.24 (d, 1H, J=8 Hz), 6.73 (d, 1H, J=8 Hz), 3.65 (s, 3H), 2.67-2.59 (m, 4H), and 1.66-1.63 (m, 4H) .

### 3. Preparation of 1-Methoxy-5,6,7,8-tetrahydronaphthalene-2-boronic acid

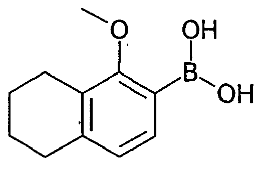

To a stirred mixture of 6-bromo-5-methoxy-1,2,3,4-tetrahydronaphthalene, (15 g, 62.21 mmol) in THF (150 mL) cooled to 0°C was added n-butyllithium (43.14 mL, 69.03 mmol, 1.6M solution in hexanes) over 5 min. The reaction mixture was stirred for 1 h then cooled to -78°C, and triisopropyl borate (21.72 mL, 17.7 g, 94.14 mmol) was added dropwise over 0.25 h. The mixture was allowed to warm to room temperature with stirring then poured into 1N HCl (100 mL). The product was extracted with ethyl acetate (4x100 mL), the combined organic extracts dried over anhydrous sodium sulfate, and filtered. After concentrating the filtrate in vacuo at ambient temperature, the oily yellow residue was partially dissolved in diethyl ether, triturated with hexane, and the resulting precipitate filtered and washed with hexane to afford 1.64 g (13%) of the title compound 1-methoxy-5,6,7,8-tetrahydronaphthalene-2-boronic acid as an off-white crystalline solid (MW 206.05, C₁₁H₁₅BO₃).

¹H NMR (DMSO-d₆) δ 7.79 (s, 2H), 7.18 (d, 1H, J=8 Hz), 6.75 (d, 1H, J=8 Hz), 3.64 (s, 3H), 2.67-2.64 (m, 2H), 2.61-2.59 (m, 2H) and 1.67-1.65 (m, 4H).

### 4. Preparation of 2-Chloro-3-nitrobenzoic acid, methyl ester

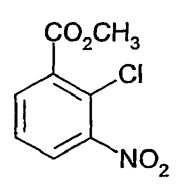

A solution of 2-chloro-3-nitrobenzoic acid (20.16 g, 100 mmol), iodomethane (15.6 g, 110 mmol), and potassium carbonate (15.0 g, 108.5 mmol) in DMF (100 mL) was stirred at room temperature for 48 hours. The mixture was poured into 1.5 liters of H₂O. The resultant precipitate was collected by filtration, washed with H₂O, and dried in vacuo to afford 20.0 g (93%) of the title compound 2-chloro-3-nitrobenzoic acid, methyl ester as a white solid (MW 215.59).

¹H NMR (CDCl₃) δ 8.42 (dd, 1H, J=1 and 8 Hz), 8.18 (dd, 1H, J=1 and 8 Hz), 7.43 (t, 1H, J=8 Hz), and 3.9 (s, 3H). IR (KBr, cm⁻¹) 1743, 1719, 1595, 1540, 1532, 1433, 1357, 1300, and 730. MS (FD) m/e 215, 216. Anal. Calcd for C₈H₆NClO₄: C, 44.57; H, 3.81; N, 6.50. Found C, 44.19; H, 3.45; N, 6.19.

### 5. Preparation of 2-(1-Methoxy-5,6,7,8-tetrahydronaphthalen-2-yl)-3-nitrobenzoic acid, methyl ester

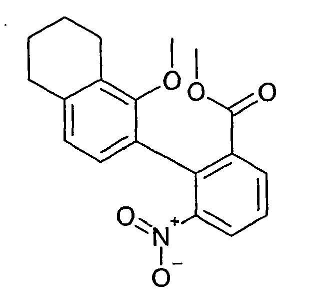

A solution of 2-chloro-3-nitrobenzoic acid, methyl ester, (2.06 g, 9.61 mM), 1-methoxy-5,6,7,8-tetrahydronaphthalene-2-boronic acid, (2.08 g, 10.09 mM), tetrakis(triphenylphosphine)palladium(0) (0.583 g, 0.5 mM), and 2M aqueous sodium carbonate solution (10.5 mL, 21.0 mM) in THF (50 mL) was stirred at reflux for 48 hours in the absence of light. The mixture was cooled to room temperature, the THF removed in vacuo, and the resulting aqueous residue dissolved in ethyl acetate and brine. The solvent layers were separated, the aqueous layer extracted with ethyl acetate (4x25 mL), the combined ethyl acetate extracts washed successively with H₂O, 1N HCl, saturated aqueous NaHCO₃ solution, and saturated brine, dried over magnesium sulfate, filtered, and concentrated in vacuo. The resultant black oil was purified by column chromatography on silica gel (elution with 5-15% ethyl acetate/hexane) to afford 0.656 g (20%) of the title compound 2-(1-methoxy-5,6,7,8-tetrahydronaphthalen-2-yl)-3-nitrobenzoic acid, methyl ester as a yellow solid (MW 341.37, C₁₉H₁₉NO₅).

¹H NMR (DMSO-d₆) δ 8.10 (dd, 1H, J=1 and 8 Hz), 8.00 (dd, 1H, J=1 and 8 Hz), 7.70 (t, 1H, J=8 Hz), 6.84 (d, 1H, J=8 Hz), 6.78 (d, 1H, J=8 Hz), 3.54 (s, 3H), 3.22 (s, 3H), 2.71 (br s, 2H), 2.62-2.61 (m, 2H), and 1.70 (br s, 4H). IR (CHCl₃, cm⁻¹) 2936, 1733, 1602, 1535, 1489, 1363, 1297, 1188, and 967. MS (ESI) m/e 342. MS (FD) m/e 341. Anal. Calcd for C₁₉H₁₉NO₅: C, 66.85; H, 5.61; N, 4.10. Found C, 66.00; H, 5.07; N, 3.37.

### 6. Preparation of 11-Methoxy-7,8,9,10-tetrahydro-5H-benzo[b]carbazole-1-carboxylic acid, methyl ester

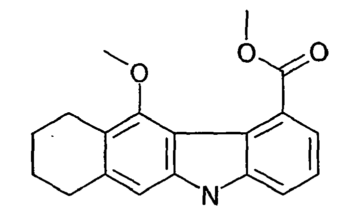

A solution of 2-(1-methoxy-5,6,7,8-tetrahydronaphthalen-2-yl)-3-nitrobenzoic acid, methyl ester, (0.5 g, 1.46 mM) in triphenyl phosphite (2.26 g, 1.91 mL, 7.29 mM) was heated at 160 °C for 48 h under a nitrogen atmosphere. The mixture was cooled to room temperature and dried azeotropically in vacuo with toluene, then purified by column chromatography on silica gel (elution with 10% ethyl acetate/hexane) to afford 0.2 g (44%) of the title compound 11-methoxy-7,8,9,10-tetrahydro-5H-benzo[b]carbazole-1-carboxylic acid, methyl ester as a tan solid (MP 194-196 °C, MW 309.37).

¹H NMR (CDCl₃) δ 8.00 (br s, 1H), 7.42 (d, 1H, J=8 Hz), 7.36 (t, 1H, J=8 Hz), 7.27 (d, 1H, J=8 Hz), 6.93 (s, 1H), 3.95 (s, 3H), 3.67 (s, 3H), 2.93 (br s, 2H), 2.89 (br s, 2H), and 1.84-1.81 (m, 4H). IR (KBr, cm⁻¹) 3263, 2932, 2831, 1701, 1631, 1436, 1306, 1294, 1142, 983, 761, and 740. MS (ESI) m/e 278, 308, and 310. Anal. Calcd for C₁₉H₁₉NO₃: C, 73.77; H, 6.19; N, 4.53. Found C, 73.87; H, 6.15; N, 4.45.

### 7. Preparation of 5-Benzyl-11-methoxy-7,8,9,10-tetrahydro-5H-benzo[b]carbazole-1-carboxylic acid, methyl ester

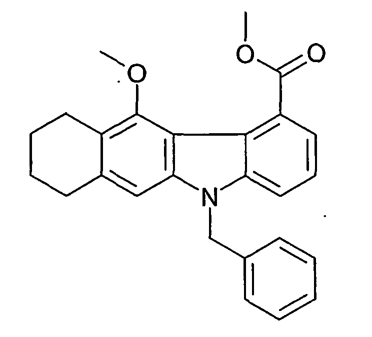

A solution of 11-methoxy-7,8,9,10-tetrahydro-5H-benzo[b]carbazole-1-carboxylic acid, methyl ester, (0.155 g, 0.5 mM) in 5 mL DMF was added to a 60% NaH mineral oil dispersion (0.04 g, 1.0 mM, washed twice with hexane) at room temperature. Following cessation of gas evolution, benzyl bromide (0.067 mL, 0.094 g, 0.55 mM) was added and the mixture stirred at room temperature for 3 h. The mixture was diluted with ethyl acetate and H₂O, the solvent layers separated, and the aqueous layer extracted with ethyl acetate (4x25 mL). The combined ethyl acetate extracts were washed with H₂O, 1N HCl, saturated aqueous NaHCO₃ solution, and saturated brine, dried over magnesium sulfate, filtered, concentrated in vacuo, and triturated with hexanes. The resultant tan solid was dried in vacuo, and purified by column chromatography on silica gel (elution with 9:1 toluene/ethyl acetate) to afford 0.133 g (66%) of the title compound 5-benzyl-11-methoxy-7,8,9,10-tetrahydro-5H-benzo[b]carbazole-1-carboxylic acid, methyl ester as an off-white foam (MP 144-146 °C , MW 399.49).

¹H NMR (CDCl₃) δ 7.37-7.32 (m, 3H), 7.28-7.23 (m, 3H), 7.10 (d, 2H, J=8 Hz), 6.89 (s, 1H), 5.44 (s, 2H), 3.97 (s, 3H), 3.69 (s, 3H), 2.91-2.89 (m, 4H), and 1.83-1.81 (m, 4H). IR (KBr, cm⁻¹) 2934, 1734, 1453, 1427, 1304, 1277, 1169, 1135, 1109, 754, and 713. MS (ESI) m/e 368, 400. Anal. Calcd for C₂₆H₂₅NO₃: C, 78.17; H, 6.31; N, 3.51. Found C, 77.68; H, 6.31; N, 3.79

### 8. Preparation of 5-Benzyl-11-hydroxy-7,8,9,10-tetrahydro-5H-benzo[b]carbazole-1-carboxylic acid, methyl ester

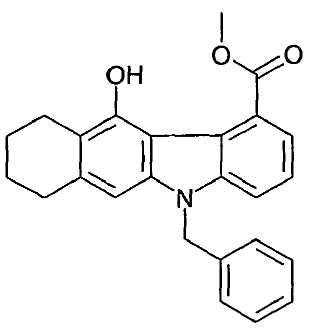

Boron tribromide (0.3 mL, 3.0 mM) was slowly added to a stirred solution of 5-benzyl-11-methoxy-7,8,9,10-tetrahydro-5H-benzo[b]carbazole-1-carboxylic acid, methyl ester, (0.12 g, 0.3 mM) in 5 mL methylene chloride at -10 °C. After 5.5 h, the mixture was quenched with methanol (3.0 mL, 75.0 mM) and allowed to warm to room temperature with stirring over 2 h. The mixture was diluted with methylene chloride, washed with H₂O, 1N HCl, saturated aqueous NaHCO₃ solution, and saturated brine, dried over magnesium sulfate, filtered, and concentrated in vacuo to afford 0.108 g (93%) of the title compound 5-benzyl-11-hydroxy-7,8,9,10-tetrahydro-5H-benzo[b]carbazole-1-carboxylic acid, methyl ester as a yellow solid (MP 174-176 °C, MW 385.47).

¹H NMR (CDCl₃) δ 10 .41 (s, 1H), 7.92 (d, 1H, J=8 Hz), 7.49 (d, 1H, J=8 Hz), 7.34 (t, 1H, J=8 Hz), 7.27-7.21 (m, 3H), 7.07-7.05 (m, 2H), 6.67 (s, 1H), 5.47 (s, 2H), 4.09 (s, 3H), 2.90-2.87 (m, 4H), 1.87-1.80 (m, 2H), and 1.79-1.77 (m, 2H). IR (KBr, cm⁻¹) 3460 (br), 3130-2830 (multiple peak grouping), 1671, 1437, 1294, 1270, and 751. MS (ESI) m/e 354, 384, and 386. Anal. Calcd for C₂₅H₂₃NO₃: C, 77.90; H, 6.01; N, 3.63. Found C, 77.71; H, 6.10; N, 3.53.

### 9. Preparation of 5-Benzyl-11-hydroxy-7,8,9,10-tetrahydro-5H-benzo[b]carbazole-1-carboxylic acid amide

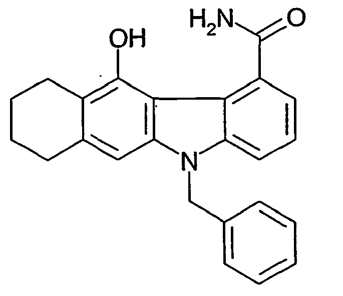

A solution of 5-benzyl-11-hydroxy-7,8,9,10-tetrahydro-5H-benzo[b]carbazole-1-carboxylic acid, methyl ester, (0.097 g, 0.25 mM) in 8 mL THF was placed in an Ace Glass pressure tube (203 mm x 38 mm) with a small stirring bar. A N₂(g) stream was installed over the solution surface. The pressure tube and reaction solution were cooled to 78 °C, and ~ 8 mL NH₃(1) was condensed into the reaction vessel by introduction of an NH₃(g) stream. The pressure tube was sealed with a teflon O-ring screw cap, the resultant brown solution stirred at -78 °C for 10 minutes, then the cooling bath was removed and the reaction solution allowed to warm to ambient temperature behind a blast shield. After 24 h, the pressure tube was re-cooled to -78 °C and the internal NH₃(g) pressure was released by careful removal of the Teflon cap. The reaction solution was allowed to slowly warm to room temperature while NH₃(g) bubbled off, then the solution was concentrated in vacuo to afford 0.92 g (quantitative) of the title compound 5-benzyl-11-hydroxy-7,8,9,10-tetrahydro-5H-benzo[b]carbazole-1-carboxylic acid amide as a tan solid (MP 225 °C dec, MW 370.46).

¹H NMR (DMSO-d6) δ 10.56 (s, 1H), 8.88 (br s, 1H), 8.40 (br s, 1H), 7.67 (dd, 1H, J=2 and 7 Hz), 7.40-7.34 (m, 2H), 7.22 (d, 1H, J=7 Hz), 7.19 (d, 2H, J=8 Hz), 7.09 (d, 2H, J=7 Hz), 6.79 (s, 1H), 5.58 (s, 2H), 2.81-2.78 (m, 2H), 2.69-2.66 (m, 2H), and 1.74-1.69 (m, 4H). IR (KBr, cm⁻¹) 3408, 3255, 2918, 2866, 2837, 1626, 1599, 1576, 1551, 1452, 1435, 1332, 1292, 1271, and 696. MS (ESI) m/e 354, 369, and 371. Anal. Calcd for C₂₄H₂₂N₂O₂: C, 77.81; H, 5.99; N, 7.56. Found C, 74.69; H, 6.08; N, 6.74.

### 10. Preparation of (5-Benzyl-1-carbamoyl-7,8,9,10-tetrahydro-5H-benzo[b]carbazol-11-yloxy)-acetic acid, methyl ester

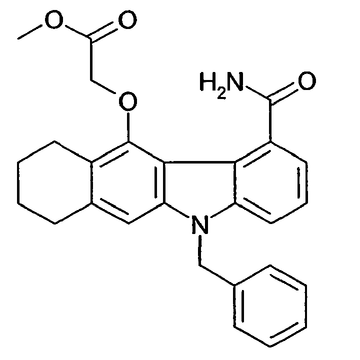

40% Methanolic Triton B (0.148 mL, 0.325 mM) was added to a solution of 5-benzyl-11-hydroxy-7,8,9,10-tetrahydro-5H-benzo[b]carbazole-1-carboxylic acid amide, (0.094 g, 0.25 mM) in 5 mL DMF at room temperature. After 2 minutes, methyl bromoacetate (0.049 mL, 0.076 g, 0.5 mM) was added and the resultant mixture stirred at room temperature for 2 h. Cesium carbonate (0.041 g, 0.125 mM) was then added as a solid, and the mixture allowed to stir at room temperature for an additional 2 h. The mixture was diluted with ethyl acetate, washed with H₂O, 1N HCl, saturated aqueous NaHCO₃ solution, and saturated brine, dried over magnesium sulfate, filtered, and concentrated in vacuo. The resulting tan foam was purified by column chromatography on silica gel (elution with ethyl acetate) to afford 0.035 g (31%) of the title compound (5-benzyl-1-carbamoyl-7,8,9,10-tetrahydro-5H-benzo[b]carbazol-11-yloxy)=acetic acid, methyl ester as a white solid (MP 174-177 °C, MW 442.52).

¹H NMR (CDCl₃) δ 7.40-7.31 (m, 3H), 7.28-7.22 (m, 3H), 7.09 (d, 2H, J=6 Hz), 6.93 (s, 1H), 5.92 (br s, 1H), 5.76 (br s, 1H), 5.45 (s, 2H), 4.56 (s, 2H), 3.86 (s, 3H), 2.92-2.88 (m, 4H), and 1.82-1.80 (m, 4H). IR (KBr, cm⁻¹) 3457, 3110, 2929, 2854, 1767, 1677, 1592, 1440, 1205, 1183, 1175, and 699. MS (ESI) m/e 426, 443, and 465. Anal. Calcd for C₂₇H₂₆N₂O₄: C, 73.29; H, 5.92; N, 6.33. Found C, 71.27; H, 5.70; N, 5.73.

### 11. Preparation of (5-Benzyl-1-carbamoyl-7,8,9,10-tetrahydro-5H-benzo[b]carbazol-11-yloxy)-acetic acid, sodium salt

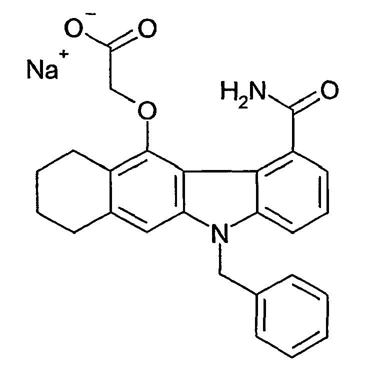

A solution of (5-benzyl-1-carbamoyl-7,8,9,10-tetrahydro-5H-benzo[b]carbazol-11-yloxy)-acetic acid, methyl ester, (0.027 g, 0.06 mM) and 1N NaOH (0.066 mL, 0.066 mM) in 5 mL of ethanol was stirred for 3 h at 25 °C. A small volume of diethyl ether/hexanes was added, then the mixture was cooled in the refrigerator. The resultant white precipitate was collected by filtration, washed with a small amount of EtOH/diethyl ether/hexanes, then dried in vacuo to afford 0.019 g (70%) of the title compound (5-benzyl-1-carbamoyl-7,8,9,10-tetrahydro-5H-benzo[b]carbazol-11-yloxy)-acetic acid, sodium salt as a white solid (MW 450.47, exact mass minus sodium 427.17). ¹H NMR (DMSO-d6) δ 8.13 (br s, 1H), 7.51 (d, 1H, J=9 Hz), 7.32 (t, 1H, J=8 Hz) 7.25-7.17 (m, 4H), 7.07-7.02 (m, 4H), 5.57(s, 2H), 3.99 (s, 2H), 2.89-2.82 (m, 2H), 2.80-2.77 (m, 2H), and 1.77-1.66 (m, 4H). IR (KBr, cm⁻¹) 3380, 3170, 2935, 1702, 1675, 1622, 1596, 1448, 1455, 1437, 1420, 1302, and 1252. MS (ESI) m/e 412, 427, and 429. Anal. Calcd for C₂₆H₂₃N₂NaO₄: C, 69.32; H, 5.15; N, 6.22. Found C, 66.76; H, 4.95; N, 5.97.

### Example 3

### 1. 9-Benzyl-9H-1-thia-9,10-diaza-cyclopenta[b]fluorene-4-carboxylic acid amide

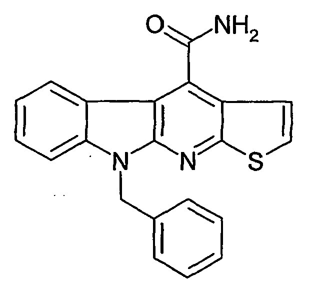

### 2. Preparation of 9-Benzyl-9H-1-thia-9,10-diazacyclopenta[b]fluorene-4-carboxylic acid

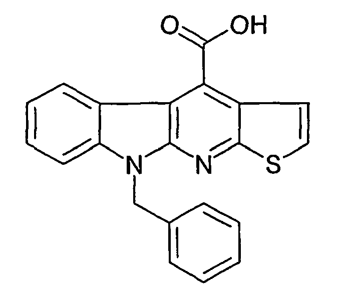

A solution of 9H-1-thia-9,10-diaza-cyclopenta[b]fluorene-4-carboxylic acid, methyl ester, (0.025 g, 0.089 mM) in 3 mL DMF was added to a 60% NaH mineral oil dispersion (0.007 g, 0.178 mM) at room temperature. Following cessation of gas evolution, benzyl bromide (0.012 mL, 0.017 g, 0.098 mM) was added and the mixture stirred at room temperature for 3.5 h. The mixture was diluted with ethyl acetate and H₂O, the solvent layers separated, and the aqueous layer extracted with ethyl acetate (4x25 mL). The combined ethyl acetate extracts were washed with H₂O, 1N HCl, saturated aqueous NaHCO₃ solution, and saturated brine, dried over magnesium sulfate, filtered, and concentrated in vacuo to afford 0.013 g of an oily solid that was identified as unreacted 9H-1-thia-9,10-diazacyclopenta[b]fluorene-4-carboxylic acid, methyl ester. Adjusted the above aqueous layer from the initial reaction workup to pH 1 with 1N HCl, and extracted with ethyl acetate (4x25 mL). The combined ethyl acetate extracts were washed with H₂O, 1N HCl, and saturated brine, dried over magnesium sulfate, filtered, and concentrated in vacuo to afford 0.023 g (74%) of the title compound 9-benzyl-9H-1-thia-9,10-diazacyclopenta[b]fluorene-4-carboxylic acid as a yellow solid (MW 358.42, C₂₁H₁₄N₂O₂S).

¹H NMR (CDCl₃) δ 8.67 (d, 1H, J=8 Hz), 7.90 (d, 1H, J=6 Hz), 7.50-7.47 (m, 2H), 7.39 (d, 1H, J=8 Hz), 7.30 (t, 1H, J=8 Hz), 7.26-7.23 (m, 5H), and 5.80 (s, 2H). MS (ESI) m/e 357, 359.

### 1. Preparation of 9-Benzyl-9H-1-thia-9,10-diazacyclopenta[b]fluorene-4-carboxylic acid amide

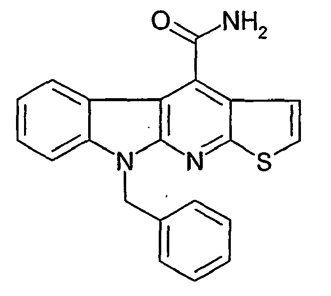

To a solution of 9-benzyl-9H-1-thia-9,10-diazacyclopenta[b]fluorene-4-carboxylic acid, (0.021 g, 0.06 mM) in 5 mL 1,2-dichloroethane was added oxalyl chloride (0.021 mL, 0.24 mM) and one drop of DMF. The mixture was stirred at room temperature for 1 h, then concentrated and dried azeotropically in vacuo with toluene. Dissolved the resultant yellow solid in 5 mL 1,2-dichloroethane and placed in an Ace Glass pressure tube (203 mm x 38 mm) with a small stirring bar. A N₂(g) stream was installed over the solution surface. The pressure tube and reaction solution were cooled to -78 °C, and ∼ 5 mL NH₃(l) was condensed into the reaction vessel by introduction of an NH₃(g) stream. The pressure tube was sealed with a teflon O-ring screw cap, the resultant yellow solution stirred at -78 °C for 10 minutes, then the cooling bath was removed and the reaction solution allowed to warm to ambient temperature behind a blast shield. After 72 h, the pressure tube was re-cooled to - 78 °C and the internal NH₃(g) pressure was released by careful removal of the Teflon cap. The reaction solution was allowed to slowly warm to room temperature while NH₃(g) bubbled off, then the solution was concentrated in vacuo to afford 0.015 g (71%) of the title compound 9-benzyl-9H-1-thia-9,10-diaza-cyclopenta[b]fluorene-4-carboxylic acid amide as a yellow solid (MP 242-245 °C, MW 357.44).

¹H NMR (CDCl₃) δ 8.37 (d, 1H, J=8 Hz), 7.57 (d, 1H, J=6 Hz), 7.47 (t, 1H, J=7 Hz), 7.41 (d, 1H, J=6 Hz), 7.35 (d, 1H, J=8 Hz), 7.29 (t, 1H, J=6 Hz), 7.27-7.20 (m, 4H), 6.96 (s, 1H), 6.22 (br s, 1H), 6.19 (br s, 1H), and 5.74 (s, 2H). IR (KBr, cm⁻¹) 3365, 3181, 3090, 2953, 2923, 2852, 1645, 1575, 1470, 1419, 1295, 1128, and 743. MS (ESI) m/e 356, 358. Anal. Calcd for C₂₁H₁₅N₃OS: C, 70.57; H, 4.23; N, 11.76. Found C, 69.75; H, 5.72; N, 8.56.

## Claims

1. A tetracyclic compound of formula (I), or a pharmaceutically acceptable salt, solvate or methyl, ethyl, propyl, isopropyl, n-butyl, isobutyl, tert-butyl, morpholinoethyl, or N,N-diethylglycolamido ester thereof; 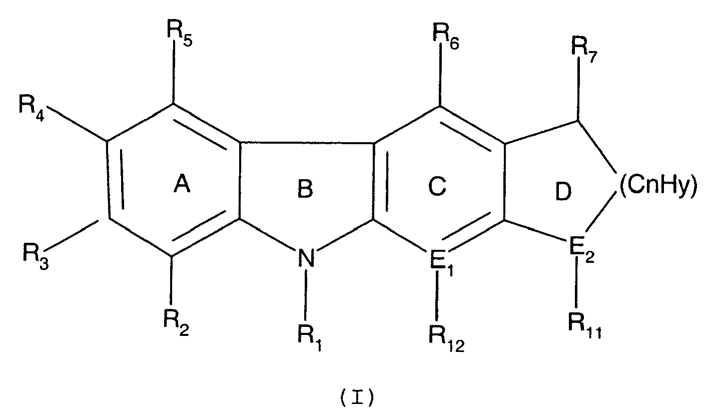 wherein ;
E₁ is C or N and E₂ is C, CH, N, O, or S;
n is 1, 2 or 3 and y is an appropriate number of hydrogen atoms based on the value of (n) and also based on ring unsaturation;
the D ring has zero, single or multiple unsaturation as chemically possible based on ring size and conjugational or electronic effects;
R₁ is the group -(L)-R₈₀; where, -(L)- is a group represented by any one of the following formulae (Ia), (Ib), (Ic), (Id), (Ie), or (If): 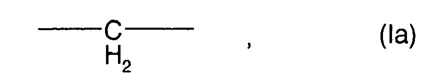
-O- , (Ib)
-S- , (Ic) 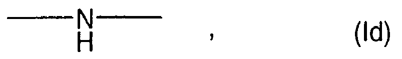 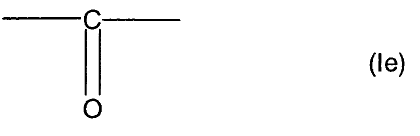
or 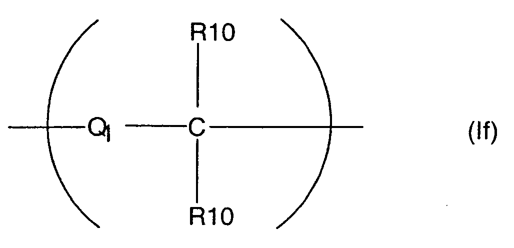 where Q₁ is a bond or any of the divalent groups (Ia), (Ib), (Ic), (Id), and (Ie), and each R₁₀ is independently hydrogen, (C₁-C₈)alkyl, (C₁-C₈)haloalkyl or (C₁-C₈)alkoxy; and where R₈₀ is a substituted or unsubstituted group selected from the group consisting of (C₅-C₁₄)cycloalkyl, (C₅-C₁₄)cycloalkenyl, phenyl, naphthyl, norbornanyl, bicycloheptadienyl, toluyl, xylenyl, indenyl, stilbenyl, terphenylyl, diphenylethylenyl, phenyl-cyclohexenyl, acenaphthylenyl, anthracenyl, biphenyl, bibenzylyl and related bibenzylyl homologues represented by the formula (a); 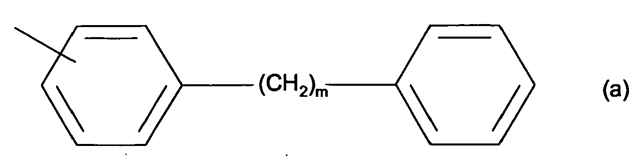 where m is a number from 1 to 8;
R₂ is hydrogen, methyl, ethyl, propyl, isopropyl, cyclopropyl, -F, -CF₃, -Cl, -Br, or -O-CH_{3;}
R₃, and R₄ are independently selected from the group consisting of hydrogen, (C₁-C₄)alkyl, (C₂₋C₄)alkenyl, -O-((C₁-C₄) alkyl) , -S-((C₁-C₃) alkyl) ,-(C₃-C₄)cycloalkyl, -CF₃, halo, -NO₂, -CN, and -SO₃;
R₅ is -(L₅)- Z, where -(L₅)- is a divalent linker group selected from a bond, or a divalent group selected from: 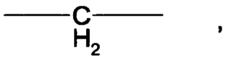
-O- ,
-S- , 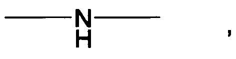
or 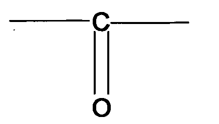 and Z is selected from an amide, thioamide, oxime amide, oxime thioamide, glyoxylamide, hydrazide, ureido or acetamide group represented by the formulae, 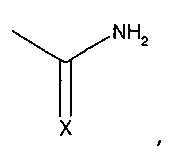 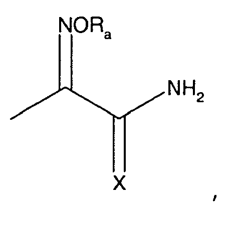 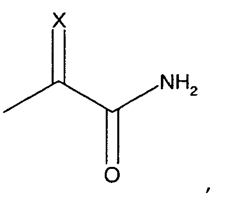 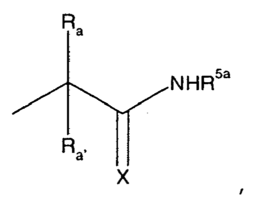 or 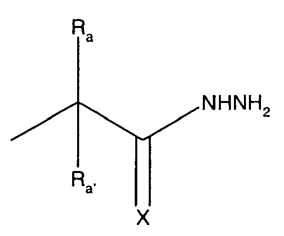 wherein X is oxygen or sulfur, Rₐ and R_{a'} are independently selected from hydrogen, (C₁-C₈)alkyl, aryl, (C₁-C₈)alkaryl, (C₁-C₈)alkoxy, aralkyl and -CN, and R^{5a} is hydrogen, methyl or ethyl;
R₆ is the group, hydrogen, CONH₂, CONHR^{6b}, -(La)-(acidic group), -(Lₕ)-(N-hydroxyfunctional amide group) or -(Lc)-(acylamino acid group)-, wherein;
-(Lₐ)-, -(Lₕ)- or -(Lc)- is selected from a group represented by the formula; 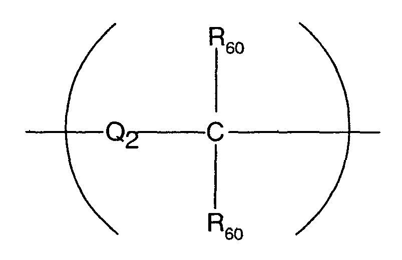 where Q₂ is selected from the group -(CH₂)-, -O-, -NR₆₀-, -C(O)-, and -S-, and each R₆₀ is independently selected from hydrogen, (C₁-C₈)alkyl, aryl, (C₁₋C₈)alkaryl, (C₁-C₈)alkoxy, aralkyl, and halo;
the (acidic group) is selected from CO₂H, CO₂Na, CO₂K, COR^{6a}, SO₃H or P(O)(OH)₂;
a (N-hydroxyfunctional amide group) is represented by the formula: 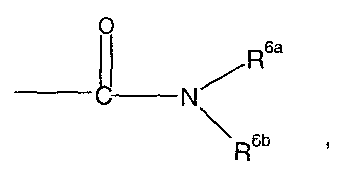 wherein; R^{6a} is selected from the group consisting of OH, (C₁-C₆)alkoxy, and aryloxy; and wherein R^{6b} is hydrogen or an organic substituent selected from the group consisting of (C₁-C₈)alkyl, aryl, (C₇-C₁₄)aralkyl, (C₇-C₁₄)alkaryl, (C₃-C₈)cycloalkyl, (C₁-C₈)alkoxyalkyl and these groups substituted with halogen, -CF₃, -OH, (C₁-C₈)alkyl, amino, carbonyl, and -CN; and
the (acylamino acid group) is represented by the formula: 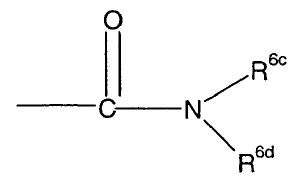 wherein R^{6c} is selected from the group consisting of H, (C₁-C₆)alkyl, (C₁-C₆)alkoxy, heteroaryl, aryl, and -CF₃; and wherein NR^{6d} is an amino acid residue of an amino acid with the nitrogen atom being part of the amino group of the amino acid; and wherein the amino acid residue is derived from an amino acid selected from the group comprising isoleucine, valine, phenylalanine, aspartic acid, leucine, glycine, asparagine, cysteine, glutamine, glutamic acid, histidine, lysine, methionine, serine, threonine, tryptophan, tyrosine and derivatives thereof;
R₇ is selected from hydrogen, (C₁-C₈)alkyl, (C₂₋C₈)alkenyl, (C₂-C₈) alkynyl, (C₇-C₁₂)aralkyl, (C₇₋C₁₂)alkaryl, (C₃-C₈)cycloalkyl, (C₃-C₈)cycloalkenyl, phenyl, toluyl, xylenyl, biphenyl, (C₁-C₈)alkoxy, (C₂₋C₈)alkenyloxy, (C₂-C₈)alkynyloxy, (C₂-C₁₂)alkoxyalkyl, (C₂-C₁₂)alkoxyalkyloxy, (C₂-C₁₂)alkylcarbonyl, (C₂₋C₁₂)alkylcarbonylamino, the group -(Lₕ)-(N-hydroxyfunctional amide group) as previously defined, or the group -(L_{c})-(acylamino acid group) as previously defined, or the group, -(Lₐ)-(acidic group) as previously defined;
and provided that at least one of R₆ or R₇ is the group-(Lₕ)-(N-hydroxyfunctional amide group), or the group -(L_{c})-(acylamino acid group), or the group, - (Lₐ)-(acidic group);
R₁₁ and R₁₂ where applicable based on the identity of E₁ and E₂ are independently selected from hydrogen, (C₁-C₈)alkyl, C₂-C₈)alkenyl, (C₂-C₈)alkynyl, (C₇-C₁₂) aralkyl, (C₇-C₁₂)alkaryl, (C₃-C₈)cycloalkyl, (C₃-C₈)cycloalkenyl, phenyl, toluyl, xylenyl, biphenyl, (C₁-C₈)alkoxy, (C₂-C₈)alkenyloxy, (C₂₋C8)alkynyloxy, (C₂-C₁₂)alkoxyalkyl, (C₂₋C₁₂)alkoxyalkyloxy, (C₂-C₁₂)alkylcarbonyl, and (C₂₋C₁₂)alkylcarbonylamino.

2. The compound of Claim 1 wherein E₁ and E₂ are carbon and R₃, and R₄ are independently hydrogen, (C₁₋C₄)alkyl, (C₂-C₄)alkenyl, -O-(C₁-C₃ alkyl), -S-(C₁-C₃ alkyl), (C₃-C₄)cycloalkyl, -CF₃, halo, -NO₂, -CN, or - SO₃.

3. The compound of Claim 1 wherein the D ring has 1, 2 or 3 double bonds depending on ring size.

4. A compound according to Claim 1 wherein E₁ and E₂ are both carbon.

5. The compound of Claim 1 wherein -(Lₕ)-, -(Lₐ)-, or -(L_{c})-, for R₆ is a divalent group independently selected from, 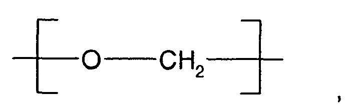 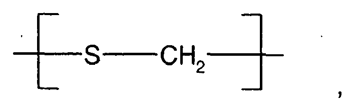 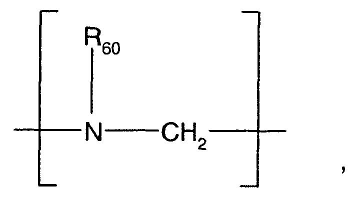 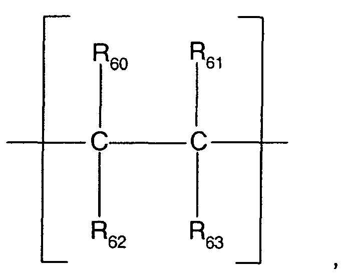 or 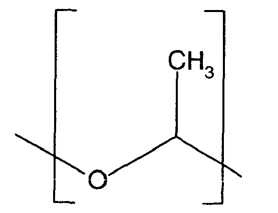 where R₆₀, R₆₁, R₆₂, and R₆₃ are independently selected from hydrogen, and (C₁-C₈)alkyl.

6. The compound of Claim 1 wherein for R₅, Z is the group represented by the formula; 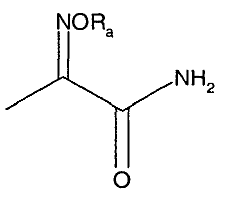 and the linking group -(L₅)- is a bond; and Rₐ is hydrogen, methyl, ethyl, propyl, isopropyl, phenyl or benzyl.

7. The compound of Claim 1 wherein for R₅, Z is the group represented by the formula; 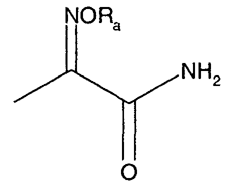 and the linking group -(L₅)- is a bond; and Rₐ is hydrogen.

8. The compound of Claim 1 wherein for R₅, Z is the group represented by the formula; 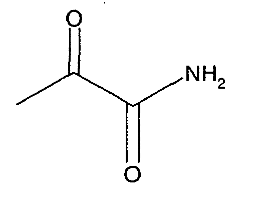 and the linking group -(L₅)- is a bond.

9. The compound of Claim 1 wherein for R₅, Z is the group represented by the formula; 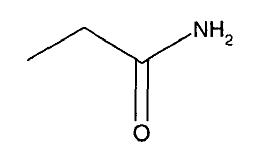 and the linking group -(L₅)- is a bond.

10. The compound of Claim 1 wherein for R₅ the divalent linking group -(L₅)- is a bond.

11. The compound of claim 1 wherein R₆ is the group, -(L_{c})-(acylamino acid group) and wherein the (acylamino acid group) is: 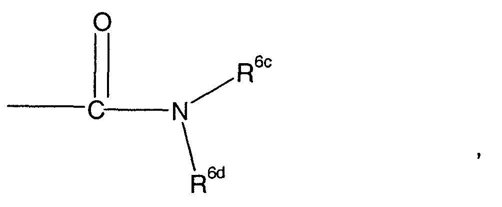 and R^{6c} is selected from the group consisting of H, (C₁₋C₆)alkyl, (C₁-C₆)alkoxy, heteroaryl, aryl and -CF₃; and wherein NR^{6d} is an amino acid residue of a amino acid with the nitrogen atom being part of the amino group of the amino acid; and wherein the amino acid residue is derived from an amino acid selected from the group comprising isoleucine, valine, phenylalanine, aspartic acid, leucine, glycine, asparagine, cysteine, glutamine, glutamic acid, histidine, lysine, methionine, serine, threonine, tryptophan, tyrosine and derivatives thereof.

12. The compound of claim 1 wherein R₆ is the group, -(Lₐ)-(acidic group) and wherein the (acidic group) is selected from the group consisting of -COOH, -COONa, and -COOK.

13. A tetracyclic compound represented by the formula (II'), or a pharmaceutically acceptable salt, solvate or methyl, ethyl, propyl, isopropyl, n-butyl, isobutyl, tert-butyl, morpholinoethyl, or N,N-diethylglycolamido ester thereof; 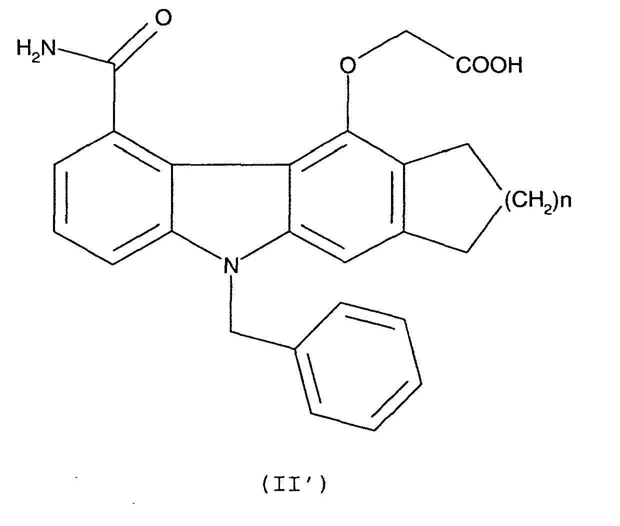 wherein ;
n is 1 or 2.

14. A tetracyclic compound represented by the formula (III'), or a pharmaceutically acceptable salt, solvate or methyl, ethyl, propyl, isopropyl, n-butyl, isobutyl, tert-butyl, morpholinoethyl, or N,N-diethylglycolamido ester thereof;

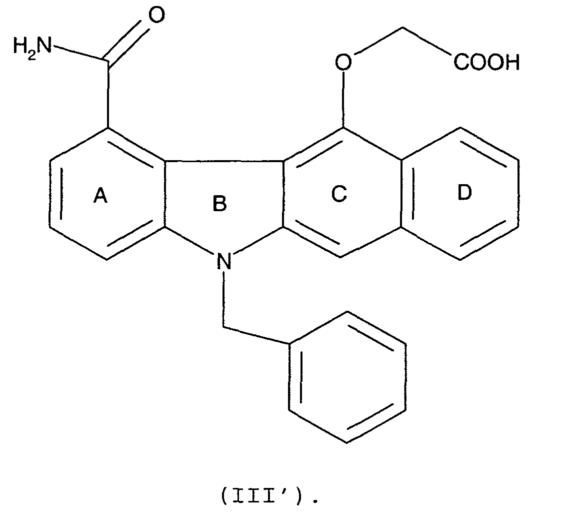

15. A compound according to formula I selected from the group consisting of:
(10-Benzyl-6-carbamoyl-1,2,3,10-tetrahydrocyclopenta[a]carbazol-5-yloxy)acetic acid methyl ester,
(10-Benzyl-6-carbamoyl-1,2,3,10-tetrahydrocyclopenta[a]carbazol-5-yloxy)acetic acid,
(11-Benzyl-7-carbamoyl-2,3,4,11-tetrahydro-1*H-*benzo[a]carbazol-6-yloxy)acetic acid methyl ester, and
(11-Benzyl-7-carbamoyl-2,3,4,11-tetrahydro-1*H-*benzo[a]carbazol-6-yloxy)acetic acid, or a pharmaceutically acceptable salt, solvate or methyl, ethyl, propyl, isopropyl, n-butyl, isobutyl, tert-butyl, morpholinoethyl, or N,N-diethylglycolamido ester thereof.

16. A tetracyclic compound represented by the formulae (C1), (C2), (C3) (C4), or (C5):

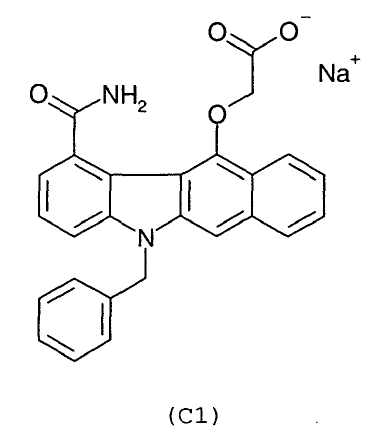

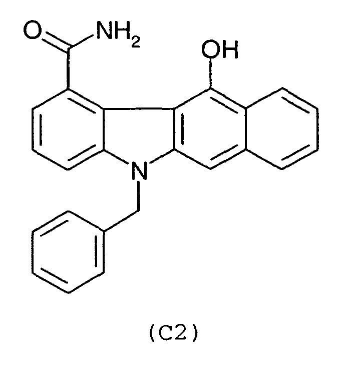

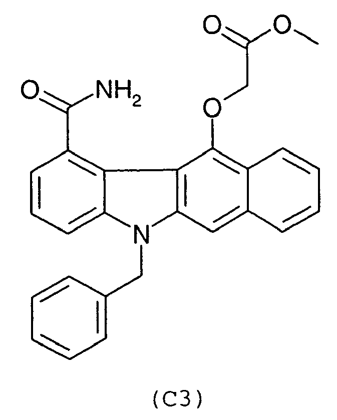

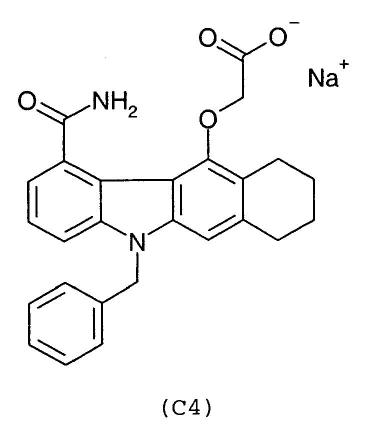

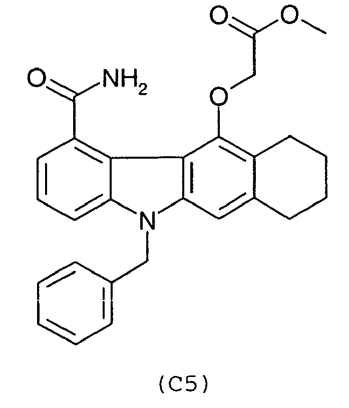

17. 9-Benzyl-9H-1-thia-9,10-diazacyclopenta[b]fluorene-4-carboxylic acid amide or a pharmaceutically acceptable salt thereof.

18. A pharmaceutical formulation comprising a tetracyclic compound of Claim 1 through 17 together with a pharmaceutically acceptable carrier or diluent.

19. A tetracyclic compound of any of Claims 1 through 17 for use in therapy.

20. A pharmaceutical formulation containing a therapeutically effective amount of the compound of any of Claims 1 thorough 17 useful for inhibiting sPLA₂ mediated release of fatty acid.

21. Use of a compound according to any of Claims 1 through 17 and mixtures thereof for the manufacture of a medicament for the treatment of Inflammatory Diseases.

## Patentansprüche

1. Tetracyclische Verbindung der Formel (1) oder ein pharmazeutisch annehmbares Salz, Solvat oder Methyl-, Ethyl-, Propyl-, Isopropyl-, n-Butyl-, Isobutyl-, tert-Butyl-, Morpholinoethyl- oder N,N-Diethylglycolamidoester hiervon 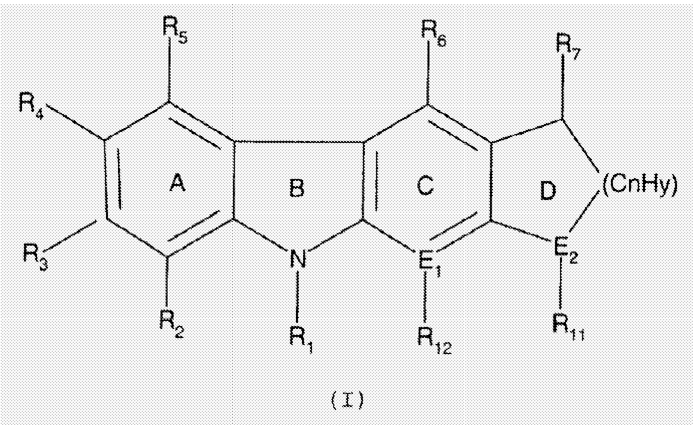 worin
E₁ für C oder N steht und E₂ für C, CH, N, O oder S steht,
n für 1, 2 oder 3 steht und y für eine geeignete Anzahl an Wasserstoffatomen basierend auf dem Wert von (n) und auch basierend auf der Ungesättigtheit des Rings steht,
der D Ring null, eine oder mehrere ungesättigte Bindungen aufweist, wie dies chemisch basierend auf der Ringgröße und die Konjugationseffekte oder elektronischen Effekte möglich ist,
R₁ für die Gruppe -(L)-R₈₀ steht, worin -(L)- für eine Gruppe steht, die durch eine der folgenden Formeln (la), (Ib), (Ic), (Id), (Ie) oder (If) dargestellt wird: 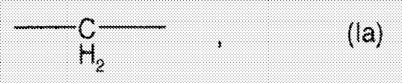
-O- , (Ib)
-S- , (Ic) 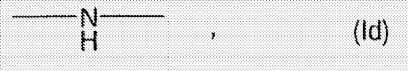 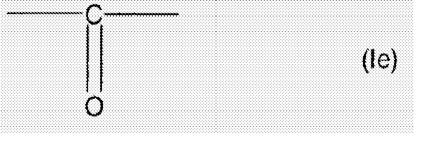
oder 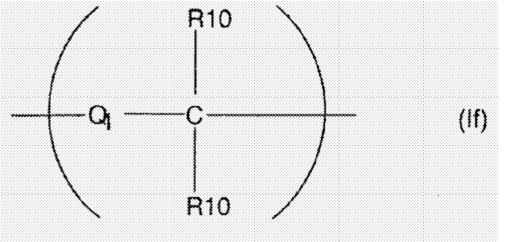 worin Q₁ für eine Bindung oder eine der divalenten Gruppen (la), (Ib), (Ic), (Id) und (Ie) steht und R₁₀ jeweils unabhängig für Wasserstoff, C₁-C₈ Alkyl, C₁-C₈ Halogenalkyl oder C₁-C₈ Alkoxy steht und worin R₈₀ für eine substituierte oder unsubstituierte Gruppe steht, die aus der Gruppe ausgewählt ist, welche besteht aus C₅-C₁₄ Cycloalkyl, C₅-C₁₄ Cycloalkenyl, Phenyl, Naphthyl, Norbornanyl, Bicycloheptadienyl, Toluyl, Xylenyl, Indenyl, Stilbenyl, Terphenylyl, Diphenylethylenyl, Phenylcyclohexenyl, Acenaphthylenyl, Anthracenyl, Biphenyl, Bibenzylyl und verwandte Bibenzylylhomloge, die durch die Formel (a) dargestellt werden 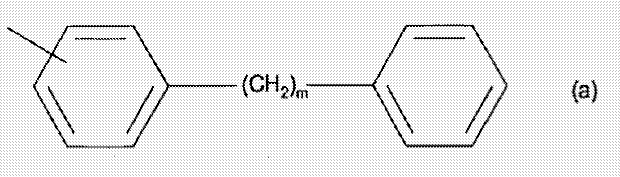 worin m für eine Zahl von 1 bis 8 steht,
R₂ für Wasserstoff, Methyl, Ethyl, Propyl, Isopropyl, Cyclopropyl, -F, -CF₃, -Cl, -Br oder -O-CH₃ steht
R₃ und R₄ unabhängig aus der Gruppe ausgewählt sind, die besteht aus Wasserstoff, C₁-C₄ Alkyl, C₂-C₄ Alkenyl, -O-(C₁-C₄ Alkyl), -S-(C₁-C₃ Alkyl), -C₃-C₄ Cycloalkyl, -CF₃, Halogen, -NO₂, -CN und -SO₃,
R₅ für -(L₅)-Z steht, worin -(L₅)- für eine divalente Linkergruppe steht, die ausgewählt ist aus einer Bindung oder einer divalenten Gruppe, die ausgewählt ist aus: 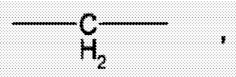
-O- ,
-S- , 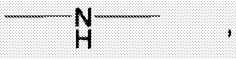
oder 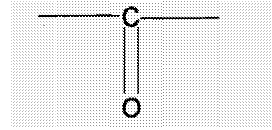 und Z ausgewählt ist aus einer Amid-, Thioamid-, Oximamid-, Oximthioamid-, Glyoxylamid-, Hydrazid-, Ureido- oder Acetamidgruppe, die durch die folgenden Formeln dargestellt ist 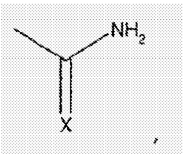 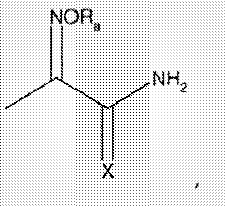 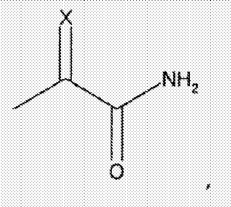 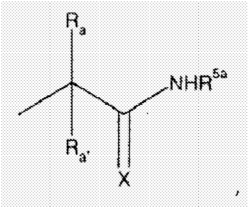 oder 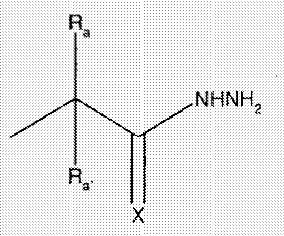 worin X für Sauerstoff oder Schwefel steht, Rₐ und Rₐ' unabhängig ausgewählt sind aus Wasserstoff, C₁₋C₈ Alkyl, Aryl, C₁-C₈ Alkaryl, C₁-C₈ Alkoxy, Aralkyl und -CN und R^{5a} für Wasserstoff, Methyl oder Ethyl steht,
R₆ steht für die Gruppe Wasserstoff, CONH₂, CONHR^{6b}, -(La)-(Säuregruppe), -(Lₕ)-(N-hydroxyfunktionelle Amidgruppe) oder -(L_{c})-(Acylaminosäuregruppe)-, worin
-(Lₐ)-, -(Lₕ)- oder -(L_{c})- aus einer Gruppe ausgewählt ist, die durch die folgende Formel dargestellt wird 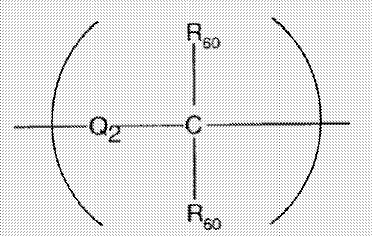 worin Q₂ aus der Gruppe -(CH₂)-, -O-, -NR₆₀-, -C(O)- und -S- ausgewählt ist und R₆₀ jeweils unabhängig ausgewählt ist aus Wasserstoff, C₁-C₈ Alkyl, Aryl, C₁-C₈ Alkaryl, C₁-C₈ Alkoxy, Aralkyl und Halogen,
die (Säuregruppe) ausgewählt ist aus CO₂H, CO₂Na, CO₂K, COR_{6a,} SO₃H oder P(O)(OH)₂,
eine (N-hydroxyfunktionelle Amidgruppe) durch die folgende Formel dargestellt wird 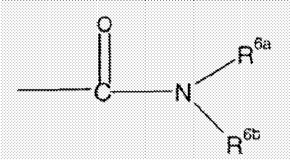 worin R^{6a} aus der Gruppe ausgewählt ist, die aus OH, C₁-C₆ Alkoxy und Aryloxy besteht und worin R^{6b} für Wasserstoff oder einen organischen Substituenten steht, der aus der Gruppe ausgewählt ist, welche besteht aus C₁-C₈ Alkyl, Aryl, C₇-C₁₄ Aralkyl, C₇-C₁₄ Alkaryl, C₃-C₈ Cycloalkyl, C₁-C₈ Alkoxyalkyl, und diese Gruppen mit Halogen, -CF₃, -OH, C₁-C₈ Alkyl, Amino, Carbonyl und -CN substituiert sind, und
die (Acylaminosäuregruppe) durch die folgende Formel dargestellt wird 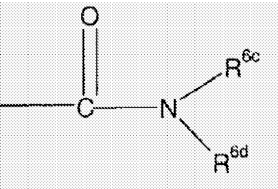 worin R^{6c} aus der Gruppe ausgewählt ist, die besteht aus H, C₁-C₆ Alkyl, C₁-C₆ Alkoxy, Heteroaryl, Aryl und -CF₃ und worin NR^{6d} für einen Aminosäurerest einer Aminosäure steht, wobei das Stickstoffatom Teil der Aminogruppe der Aminosäure ist und worin der Aminosäurerest von einer Aminosäure abgeleitet ist, die aus der Gruppe ausgewählt ist, die umfasst Isoleucin, Valin, Phenylalanin, Asparaginsäure, Leucin, Glycin, Asparagin, Cystein, Glutamin, Glutaminsäure, Histidin, Lysin, Methionin, Serin, Threonin, Tryptophan, Tyrosin und Derivate hiervon,
R₇ ausgewählt ist aus Wasserstoff, C₁-C₈ Alkyl, C₂-C₈ Alkenyl, C₂-C₈ Alkinyl, C₇-C₁₂ Aralkyl, C₇-C₁₂ Alkaryl, C₃-C₈ Cycloalkyl, C₃-C₈ Cycloalkenyl, Phenyl, Toluyl, Xylenyl, Biphenyl, C₁-C₈ Alkoxy, C₂-C₈ Alkenyloxy, C₂-C₈ Alkinyloxy, C₂-C₁₂ Alkoxyalkyl, C₂-C₁₂ Alkoxyalkyloxy, C₂-C₁₂ Alkylcarbonyl, C₂-C₁₂ Alkylcarbonylamino, der Gruppe -(Lₕ)-(N-hydroxyfunktionelle Amidgruppe), wie sie vorher definiert wurde, oder der Gruppe -(L_{c})-(Acylaminosäuregruppe), wie sie vorher definiert wurde oder der Gruppe -(Lₐ)-(Säuregruppe), wie sie vorher definiert wurde,
und mit der Maßgabe, dass zumindest eines von R₆ oder R₇ für die Gruppe -(Lₕ)-(N-hydroxyfunktionelle Amidgruppe) oder die Gruppe -(L_{c})-(Acylaminosäuregruppe) oder die Gruppe -(Lₐ)-(Säuregruppe) steht,
R₁₁ und R₁₂, wo dies möglich ist, basierend auf der Identität von E₁ und E₂ unabhängig ausgewählt sind aus Wasserstoff, C₁-C₈ Alkyl, C₂-C₈ Alkenyl, C₂-C₈ Alkinyl, C₇-C₁₂ Aralkyl, C₇-C₁₂ Alkaryl, C₃-C₈ Cycloalkyl, C₃-C₈ Cycloalkenyl, Phenyl, Toluyl, Xylenyl, Biphenyl, C₁-C₈ Alkoxy, C₂-C₈ Alkenyloxy, C₂-C₈ Alkinyloxy, C₂-C₁₂ Alkoxyalkyl, C₂-C₁₂ Alkoxyalkyloxy, C₂-C₁₂ Alkylcarbonyl und C₂-C₁₂ Alkylcarbonylamino.

2. Verbindung nach Anspruch 1, worin E₁ und E₂ für Kohlenstoff stehen und R₃ und R₄ unabhängig für Wasserstoff, C₁-C₄ Alkyl, C₂-C₄ Alkenyl, -O-(C₁-C₃ Alkyl), -S-(C₁-C₃ Alkyl), C₃-C₄ Cycloalkyl, -CF₃, Halogen, -NO₂, -CN oder -SO₃ stehen.

3. Verbindung nach Anspruch 1, worin der D Ring 1, 2 oder 3 Doppelbindungen in Abhängigkeit der Ringgröße aufweist.

4. Verbindung nach Anspruch 1, worin E₁ und E₂ beide für Kohlenstoff stehen.

5. Verbindung nach Anspruch 1, worin -(Lₕ)-, -(Lₐ)- oder -(L_{c})- für R₆ eine divalente Gruppe ist, die unabhängig ausgewählt ist aus 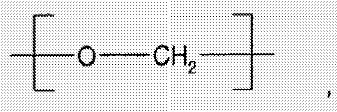 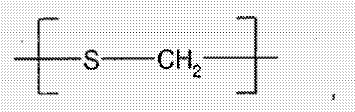 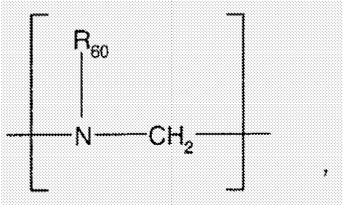 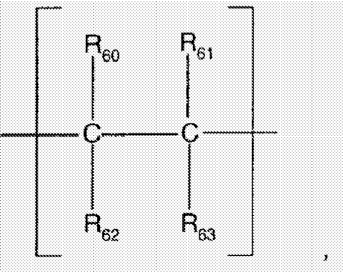 oder 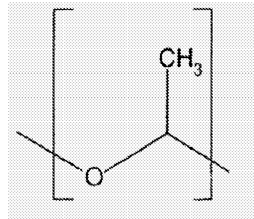 worin R₆₀, R₆₁, R₆₂ und R₆₃ unabhängig aus Wasserstoff und C₁-C₈ Alkyl ausgewählt sind.

6. Verbindung nach Anspruch 1, worin für R₅ Z für die durch die folgende Formel dargestellte Gruppe steht 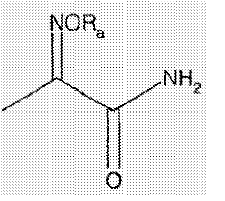 und die Bindungsgruppe -(L₅)- für eine Bindung steht und Rₐ für Wasserstoff, Methyl, Ethyl, Propyl, Isopropyl, Phenyl oder Benzyl steht.

7. Verbindung nach Anspruch 1, worin für R₅ Z für die durch die folgende Formel dargestellte Gruppe steht 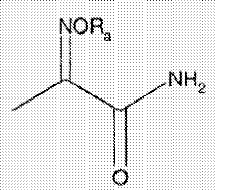 und die Bindungsgruppe -(L₅)- für eine Bindung steht und Rₐ für Wasserstoff steht.

8. Verbindung nach Anspruch 1, worin für R₅ Z für die durch die folgende Formel dargestellte Gruppe steht 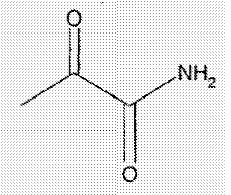 und die Bindungsgruppe -(L₅)- für eine Bindung steht.

9. Verbindung nach Anspruch 1, worin für R₅ Z für die durch die folgende Formel dargestellte Gruppe steht 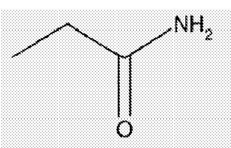 und die Bindungsgruppe -(L₅)- für eine Bindung steht.

10. Verbindung nach Anspruch 1, worin für R₅ die divalente Bindungsgruppe -(L₅)- für eine Bindung steht.

11. Verbindung nach Anspruch 1, worin R₆ für die Gruppe -(L_{c})-(Acylaminosäuregruppe) steht und worin die (Acylaminosäuregruppe) steht für 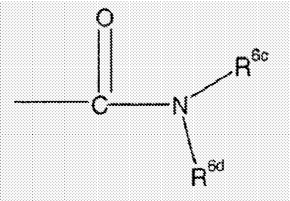 und R^{6c} aus der Gruppe ausgewählt ist, die besteht aus H, C₁-C₆ Alkyl, C₁-C₆ Alkoxy, Heteroaryl, Aryl und -CF₃ und worin NR 6^{d} für einen Aminosäurerest einer Aminosäure steht, wobei das Stickstoffatom Teil der Aminogruppe der Aminosäure ist und worin der Aminosäurerest von einer Aminosäure abgeleitet ist, die aus der Gruppe ausgewählt ist, die umfasst Isoleucin, Valin, Phenylalanin, Asparaginsäure, Leucin, Glycin, Asparagin, Cystein, Glutamin, Glutaminsäure, Histidin, Lysin, Methionin, Serin, Threonin, Tryptophan, Tyrosin und Derivate hiervon.

12. Verbindung nach Anspruch 1, worin R₆ für die Gruppe -(Lₐ)-(Säuregruppe) steht und worin die (Säuregruppe) aus der Gruppe ausgewählt ist, die besteht aus -COOH, -COONa und -COOK.

13. Tetracyclische Verbindung, die durch die Formel (II') dargestellt wird oder ein pharmazeutisch annehmbares Salz, Solvat oder Methyl-, Ethyl-, Propyl-, Isopropyl-, n-Butyl-, Isobutyl-, tert-Butyl-, Morpholinoethyl- oder N,N-Diethylglycolamidoester hiervon 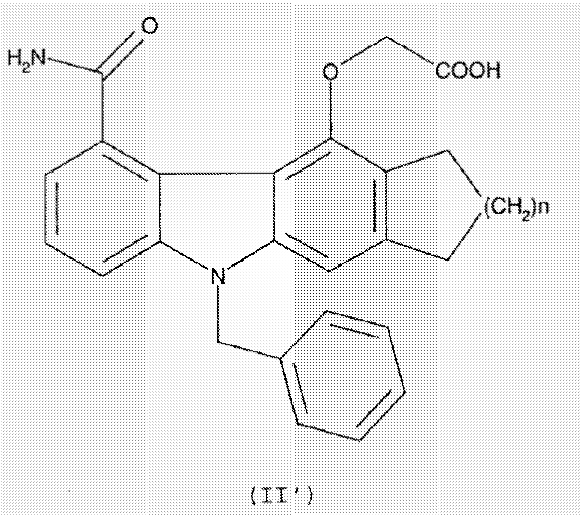 worin n für 1 oder 2 steht.

14. Tetracyclische Verbindung, die durch die Formel (III') dargestellt wird oder ein pharmazeutisch annehmbares Salz, Solvat oder Methyl-, Ethyl-, Propyl-, Isopropyl-, n-Butyl-, Isobutyl-, tert-Butyl-, Morpholinoethyl- oder N,N-Diethylglycolamidoester hiervon

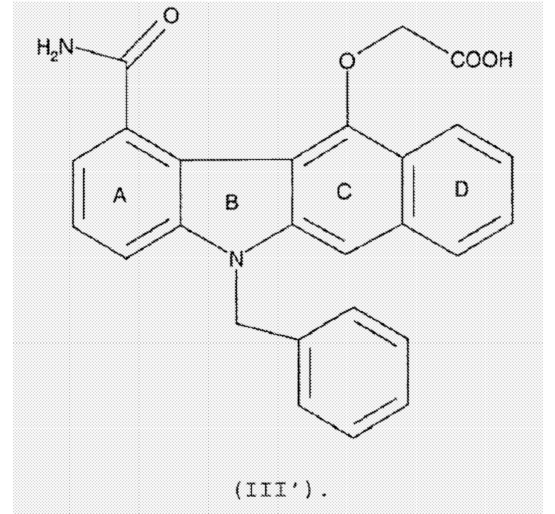

15. Verbindung der Formel I, die aus der Gruppe ausgewählt ist, welche besteht aus
(10-Benzyl-6-carbamoyl-1,2,3,10-tetrahydrocyclopenta[a]carbazol-5-yloxy)essigsäuremethylester,
(10-Benzyl-6-carbamoyl-1,2,3,10-tetrahydrocyclopenta[a]carbazol-5-yloxy)essigsäure,
(11-Benzyl-7-carbamoyl-2,3,4,11-tetrahydro-1H-benzo[a]carbazol-6-yloxy)essigsäuremethylester, und
(11-Benzyl-7-carbamoyl-2,3,4,11-tetrahydro-1H-benzo[a]carbazol-6-yloxy)essigsäure
oder ein pharmazeutisch annehmbares Salz, Solvat oder Methyl-, Ethyl-, Propyl-, Isopropyl-, n-Butyl-, Isobutyl-, tert-Butyl-, Morpholinoethyl- oder N,N-Diethylglycolamidoester hiervon.

16. Tetracyclische Verbindung, die durch die Formeln (C1), (C2), (C3), (C4) oder (C5) dargestellt wird

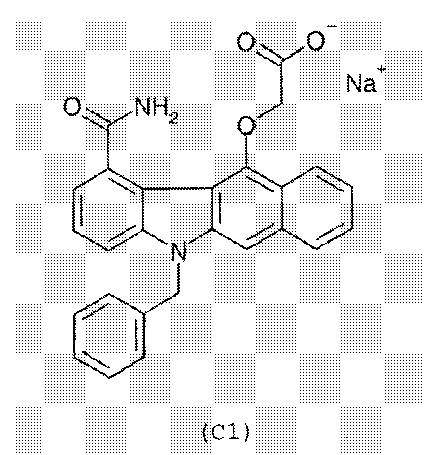

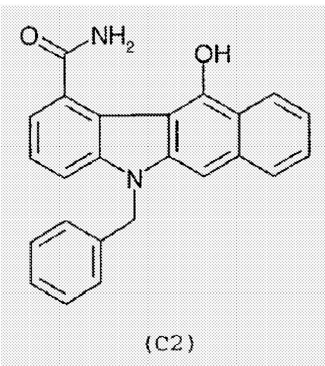

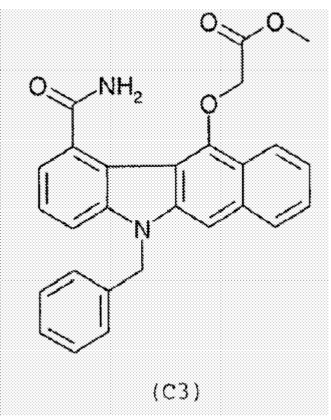

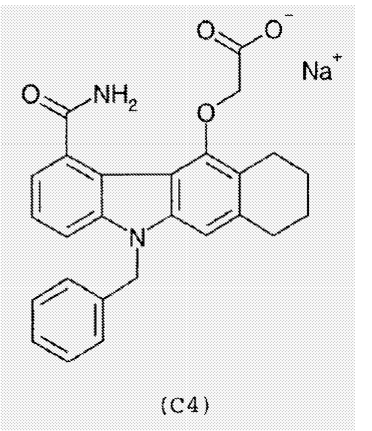

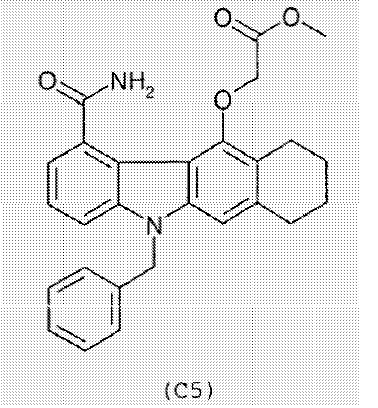

17. 9-Benzyl-9H-1-thia-9,10-diazacyclopenta[b]fluoren-4-carbonsäureamid oder ein pharmazeutisch annehmbares Salz hiervon.

18. Pharmazeutische Formulierung, die eine tetracyclische Verbindung nach Anspruch 1 bis 17 zusammen mit einem pharmazeutisch annehmbaren Träger oder Verdünnungsmittel enthält.

19. Tetracyclische Verbindung nach einem der Ansprüche 1 bis 17 zur Verwendung in der Therapie.

20. Pharmazeutische Formulierung, die eine therapeutisch wirksame Menge der Verbindung nach einem der Ansprüche 1 bis 17 enthält, die zur Hemmung der durch sPLA₂ vermittelten Freisetzung von Fettsäure brauchbar ist.

21. Verwendung einer Verbindung nach einem der Ansprüche 1 bis 17 und Gemischen hiervon zur Herstellung eines Arzneimittels zur Behandlung von entzündlichen Erkrankungen.

## Revendications

1. Composé tétracyclique répondant à la formule (I), ou un sel, un solvate ou un ester méthylique, éthylique, propylique, isopropylique, n-butylique, isobutylique, tert-butylique, morpholinoéthylique, ou N,N-diéthylglycolamido pharmaceutiquement acceptable de celui-ci ; 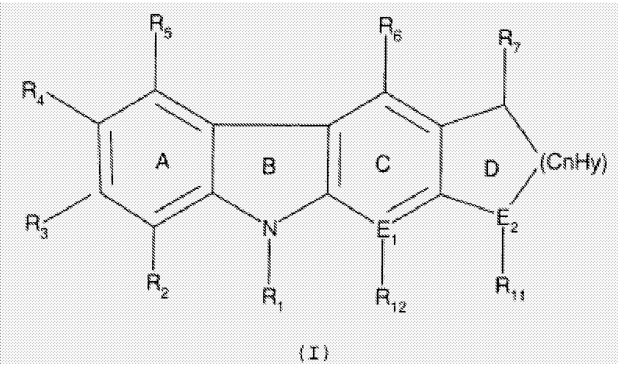 dans laquelle ;
E₁ représente C ou N et E₂ représente C, CH, N, O, ou S ;
n vaut 1, 2, ou 3 et y est un nombre approprié d'atomes d'hydrogène en fonction de la valeur de (n) et t également en fonction de l'insaturation du cycle ;
le cycle D possède zéro, une ou de multiples insaturations comme chimiquement possible en fonction de la taille du cycle et des effets conjugués ou électroniques ;
R₁ est le groupe -(L)-R₈₀ ; où -(L)- est un groupe représenté par n'importe laquelle des formules suivantes (Ia), (Ib), (Ic), (Id), (Ie), ou (If) : 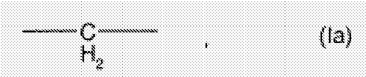
-O- , (Ib)
-S- , (Ic) 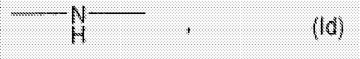 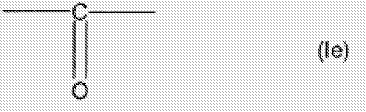
ou 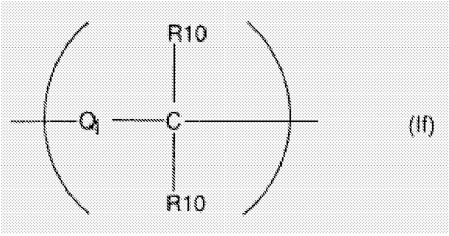 où Q₁ représente une liaison ou n'importe lequel des groupes divalents (Ia), (Ib), (Ic), (Id), et (Ie), et chaque R₁₀ représente indépendamment un atome d'hydrogène, un groupe alkyle en C₁-C₈; un groupe halogénoalkyle en C₁-C₈, ou un groupe alcoxy en C₁-C₈ ; et où R₈₀ représente un groupe substitué ou non substitué choisi dans le groupe constitué des groupes cycloalkyle en C₅ à C₁₄, cycloalcényle en C₅ à C₁₄, phényle, naphtyle, norbornanyle, bicycloheptadiényle, toluyle, xylényle, indényle, stilbényle, terphénylyle, diphényléthylényle, phénylcyclohexényle, acénaphtylényle, anthracényle, biphényle, bibenzylyle et des homologues bibenzylyle apparentés représentés par la formule (a) : 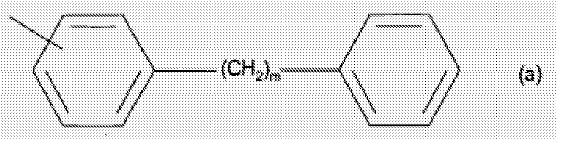 où m est un nombre de 1 à 8 ;
R₂ représente un atome d'hydrogène, un groupe méthyle, un groupe éthyle, un groupe propyle, un groupe isopropyle, un groupe cyclopropyle, -F, - CF₃, -Cl, -Br, ou -O-CH₃ ;
R₃ et R₄ sont indépendamment choisis dans le groupe constitué d'un atome d'hydrogène, d'un groupe alkyle en C₁-C₄, d'un groupe alcényle en C₂₋C₄, d'un groupe -O-(alkyle en C₁-C₄), d'un groupe -S-(alkyle en C₁-C₃), d'un groupe cycloalkyle en C₃-C₄, de -CF₃, d'un atome d'halogène, de -NO₂, de -CN, et de -SO₃ ;
R₅ représente -(L₅)-Z, où -(L₅)- représente un groupe liant divalent choisi parmi une liaison, ou un groupe divalent choisi parmi : 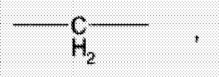
-O- ,
-S- , 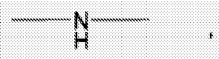
ou 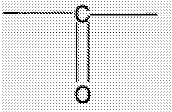 et Z est choisi parmi un groupe amide, thioamide, oxime amide, oxime thioamide, glyoxylamide, hydrazide, uréido ou acétamide représenté par les formules 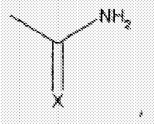 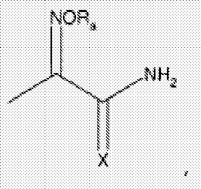 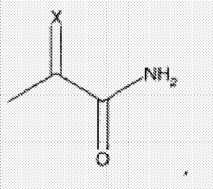 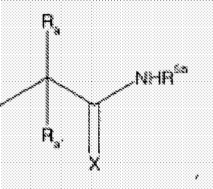 ou 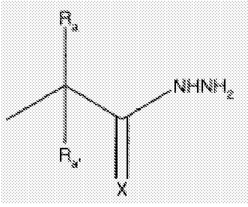 dans lesquelles X représente un atome d'oxygène ou un atome de soufre, Rₐ et Rₐ sont indépendamment choisis parmi un atome d'hydrogène, un groupe alkyle en C₁-C₈, un groupe aryle, un groupe alkaryle en C₁-C₈, un groupe alcoxy en C₁-C₈, un groupe aralkyle et -CN, et R^{5a} représente un atome d'hydrogène, un groupe méthyle ou un groupe éthyle ;
R₆ représente un atome d'hydrogène, CONH₂, CONHR^{6b}, -(Lₐ)-(groupe acide), -(Lₕ)-(groupe amide N-hydroxyfonctionnel) ou -(L_{c})-(groupe acide acylaminé)-, dans lequel
-(Lₐ)-, -(Lₕ)- ou -(L_{c})- est choisi dans un groupe représenté par la formule : 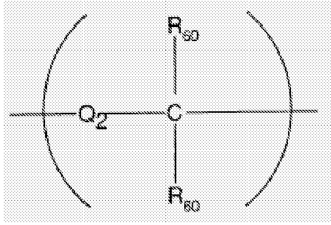 où Q₂ est choisi dans le groupe -(CH₂)-, -O-, -NR₆₀-, -C(O)-, et -S-, et chaque R₆₀ est indépendamment choisi parmi un atome d'hydrogène, un groupe alkyle en C₁-C₈, un groupe aryle, un groupe alkaryle en C₁-C₈, un groupe alcoxy en C₁-C₈, un groupe aralkyle et un atome d'halogène ;
le (groupe acide) est choisi parmi CO₂H, CO₂Na, CO₂K, COR^{6a}, SO₃H ou P(O)(OH)₂ ;
un (groupe amide N-hydroxyfonctionnel) est représenté par la formule : 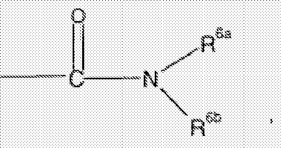 dans laquelle R^{6a} est choisi dans le groupe constitué de OH, d'un groupe alcoxy en C₁-C₆, et d'un groupe aryloxy ; et dans laquelle R^{6b} représente un atome d'hydrogène ou un substituant organique choisi dans le groupe constitué d'un groupe alkyle en C₁-C₈, d'un groupe aryle, d'un groupe aralkyle en C₇-C₁₄, d'un groupe alkaryle en C₇-C₁₄, d'un groupe cycloalkyle en C₃-C₈, d'un groupe alcoxyalkyle (en C₁-C₈), et de ces groupes substitués par un atome d'halogène, -CF₃, -OH, un groupe alkyle en C₁-C₈, un groupe amino, un groupe carbonyle, et -CN ; et
le (groupe acide acylaminé) est représenté par la formule : 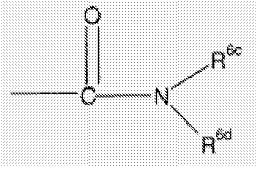 dans laquelle R^{6c} est choisi dans le groupe constitué de H, d'un groupe alkyle en C₁-C₆, d'un groupe alcoxy en C₁-C₆, d'un groupe hétéroaryle, d'un groupe aryle, et de -CF₃; et dans laquelle NR^{6d} est un résidu acide aminé d'un acide aminé ayant un atome d'azote qui fait partie du groupe amino de l'acide aminé ; et dans laquelle le résidu acide aminé est dérivé d'un acide aminé choisi dans le groupe comprenant l'isoleucine, la valine, la phénylalanine, l'acide aspartique, la leucine, la glycine, l'asparagine, la cystéine, la glutamine, l'acide glutamique, l'histidine, la lysine, la méthionine, la sérine, la thréonine, le tryptophane, la tyrosine, et des dérivés de ceux-ci ;
R₇ est choisi parmi un atome d'hydrogène, un groupe alkyle en C₁-C₈, un groupe alcényle en C₂-C₈, un groupe alcynyle en C₂-C₈, un groupe aralkyle en C₇-C₁₂, un groupe alkaryle en C₇-C₁₂, un groupe cycloalkyle en C₃-C₈, un groupe cycloalcényle en C₃-C₈, un groupe phényle, un groupe toluyle, un groupe xylényle, un groupe biphényle, un groupe alcoxy en C₁-C₈, un groupe alcényloxy en C₂-C₈, un groupe alcynyloxy en C₂-C₈, un groupe alcoxyalkyle (en C₂-C₁₂), un groupe alcoxyalkyloxy (en C₂-C₁₂), un groupe alkylcarbonyle (en C₂-C₁₂), un groupe alkylcarbonylamino (en C₂-C₁₂), le groupe -(Lₕ)-(groupe amide N-hydroxyfonctionnel) comme précédemment défini ou le groupe -(L_{c})-(groupe acide acylaminé) comme précédemment défini, ou le groupe -(Lₐ)-(groupe acide) comme précédemment défini ;
et à condition qu'au moins un de R₆ ou R₇ soit le groupe -(Lₕ)-(groupe amide N-hydroxyfonctionnel) ou le groupe -(L_{c})-(groupe acide acylaminé), ou le groupe -(Lₐ)-(groupe acide) ;
R₁₁ et R₁₂ selon le besoin par rapport à l'identité de E₁ et E₂, sont indépendamment choisis parmi un atome d'hydrogène, un groupe alkyle en C₁₋C₈, un groupe alcényle en C₂-C₈, un groupe alcynyle en C₂-C₈, un groupe aralkyle en C₇-C₁₂, un groupe alkaryle en C₇-C₁₂, un groupe cycloalkyle en C₃₋C₈, un groupe cycloalcényle en C₃-C₈, un groupe phényle, un groupe toluyle, un groupe xylényle, un groupe biphényle, un groupe alcoxy en C₁-C₈, un groupe alcényloxy en C₂-C₈, un groupe alcynyloxy en C₂-C₈, un groupe alcoxyalkyle (en C₂-C₁₂), un groupe alcoxyalkyloxy (en C₂-C₁₂), un groupe alkylcarbonyle en (C₂-C₁₂), et un groupe alkylcarbonylamino (en C₂-C₁₂).

2. Composé selon la revendication 1, dans lequel E₁ et E₂ représentent un atome de carbone, et R₃ et R₄ représentent indépendamment un atome d'hydrogène, un groupe alkyle en C₁-C₄, un groupe alcényle en C₂₋C₄, un groupe -O-(alkyle en C₁-C₃), un groupe -S-(alkyle en C₁-C₃), un groupe cycloalkyle en C₃-C₄, -CF₃, un atome d'halogène, -NO₂, -CN, ou -SO₃.

3. Composé selon la revendication 1, dans lequel le cycle D a 1, 2 ou 3 doubles liaisons en fonction de la taille du cycle.

4. Composé selon la revendication 1, dans lequel E₁ et E₂ représentent tous les deux un atome de carbone.

5. Composé selon la revendication 1, dans lequel -(Lₕ)-, -(Lₐ)- ou - (L_{c})-, pour R₆, est un groupe divalent indépendamment choisi parmi 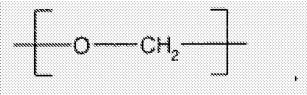 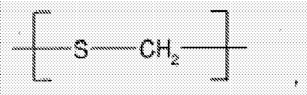 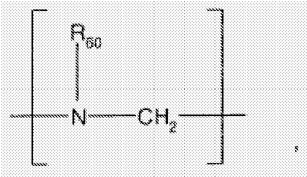 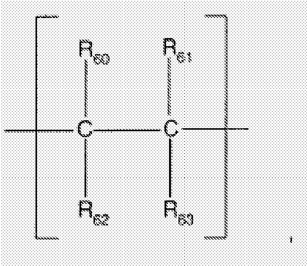 ou 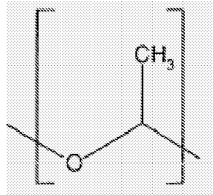 où R₆₀, R₆₁, R₆₂, et R₆₃ sont indépendamment choisis parmi un atome d'hydrogène, et un groupe alkyle en C₁-C₈.

6. Composé selon la revendication 1, dans lequel pour R₅, Z est le groupe représenté par la formule : 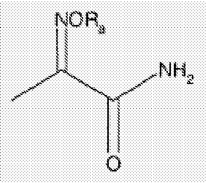 et le groupe liant -(L₅)- est une liaison ; et Rₐ représente un atome d'hydrogène, un groupe méthyle, un groupe éthyle, un groupe propyle, un groupe isopropyle, un groupe phényle, ou un groupe benzyle.

7. Composé selon la revendication 1, dans lequel pour R₅, Z est le groupe représenté par la formule : 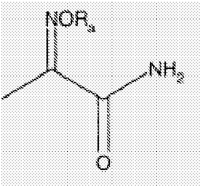 et le groupe liant -(L₅)- est une liaison ; et Rₐ représente un atome d'hydrogène.

8. Composé selon la revendication 1, dans lequel pour R₅, Z est le groupe représenté par la formule : 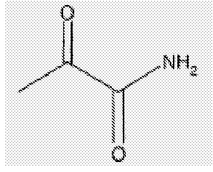 et le groupe liant -(L₅)- est une liaison.

9. Composé selon la revendication 1, dans lequel pour R₅, Z est le groupe représenté par la formule : 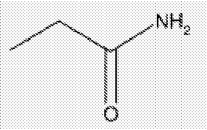 et le groupe liant -(L₅)- est une liaison.

10. Composé selon la revendication 1, dans lequel pour R₅, le groupe liant divalent -(L₅)- est une liaison.

11. Composé selon la revendication 1, dans lequel R₆ représente le groupe -(L_{c})-(groupe acide acylaminé) et dans lequel le (groupe acide aminé) est : 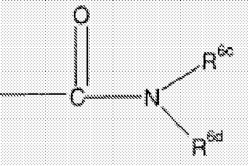 et R^{6c} est choisi dans le groupe constitué de H, d'un groupe alkyle en C₁-C₆, d'un groupe alcoxy en C₁-C₆, d'un groupe hétéroaryle, d'un groupe aryle, et de -CF₃; et dans lequel NR^{6d} est un résidu acide aminé d'un acide aminé ayant un atome d'azote qui fait partie du groupe amino de l'acide aminé ; et dans lequel le résidu acide aminé est dérivé d'un acide aminé choisi dans le groupe comprenant l'isoleucine, la valine, la phénylalanine, l'acide aspartique, la leucine, la glycine, l'asparagine, la cystéine, la glutamine, l'acide glutamique, l'histidine, la lysine, la méthionine, la sérine, la thréonine, le tryptophane, la tyrosine, et des dérivés de ceux-ci.

12. Composé selon la revendication 1, dans lequel R₆ représente le groupe -(Lₐ)-(groupe acide) et dans lequel le (groupe acide) est choisi dans le groupe constitué de -COOH, -COONa, et -COOK.

13. Composé tétracyclique représenté par la formule (II'), ou un sel, un solvate ou un ester méthylique, éthylique, propylique, isopropylique, n-butylique, isobutylique, tert-butylique, morpholinoéthylique, ou N,N-diéthylglycolamido pharmaceutiquement acceptable de celui-ci ; 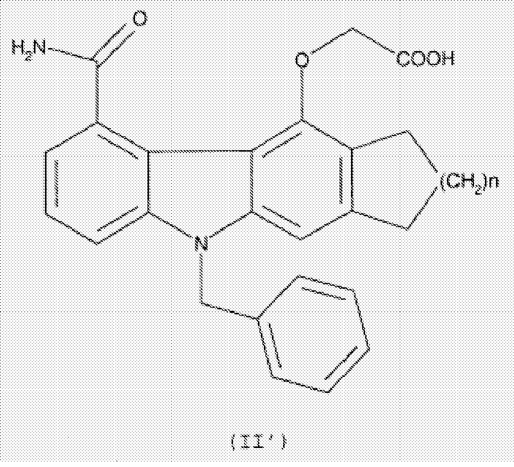 dans laquelle ;
n vaut 1 ou 2.

14. Composé tétracyclique représenté par la formule (III'), ou un sel, un solvate ou un ester méthylique, éthylique, propylique, isopropylique, n-butylique, isobutylique, tert-butylique, morpholinoéthylique, ou N,N-diéthylglycolamido pharmaceutiquement acceptable de celui-ci ;

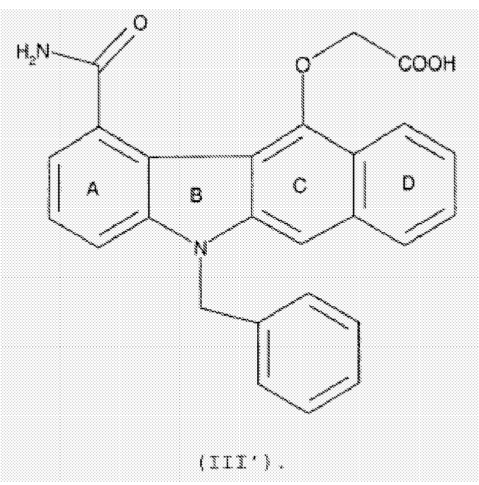

15. Composé selon la formule I choisi dans le groupe constitué de :
ester méthylique de l'acide (10-benzyl-6-carbamoyl-1,2,3,10-tétrahydrocyclopenta[a]carbazol-5-yloxy)acétique,
acide (10-benzyl-6-carbamoyl-1,2,3,10-tétrahydrocyclopenta[a]carbazol-5-yloxy)acétique,
ester méthylique de l'acide (11-benzyl-7-carbamoyl-2,3,4,11-tétrahydro-1 H-benzo[a]carbazol-6-yloxy)acétique, et
acide (11-benzyl-7-carbamoyl-2,3,4,11-tétrahydro-1H-benzo[a]carbazol-6-yloxy)acétique, ou un sel, un solvate ou un ester méthylique, éthylique, propylique, isopropylique, n-butylique, isobutylique, tert-butylique, morpholinoéthylique, ou N,N-diéthylglycolamido pharmaceutiquement acceptable de celui-ci.

16. Composé tétracyclique représenté par les formules (C1), (C2), (C3), (C4) ou (C5) :

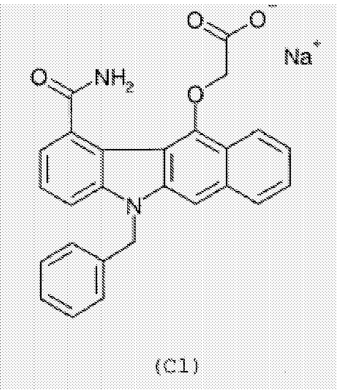

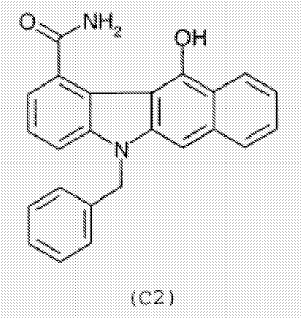

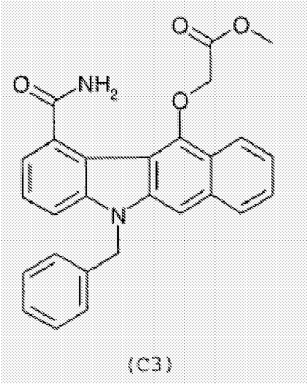

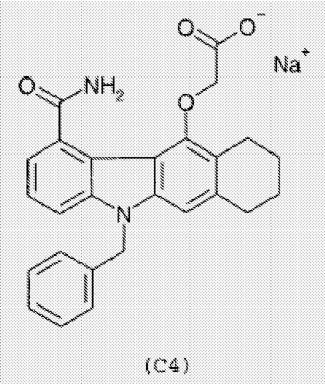

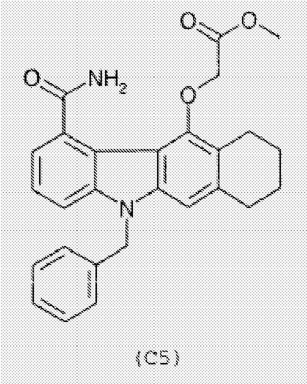

17. Amide de l'acide 9-benzyl-9H-1-thia-9,10-diazacyclopenta[b]fluorène-4-carboxylique ou un sel pharmaceutiquement acceptable de celui-ci.

18. Formulation pharmaceutique comprenant un composé tétracyclique selon l'une quelconque des revendications 1 à 17, avec un support ou un diluant pharmaceutiquement acceptable.

19. Composé tétracyclique selon l'une quelconque des revendications 1 à 17, pour une utilisation en thérapie.

20. Formulation pharmaceutique contenant une quantité thérapeutiquement efficace du composé selon l'une quelconque des revendications 1 à 17, utile pour l'inhibition de la libération d'acide gras médiée par sPLA₂.

21. Utilisation d'un composé selon l'une quelconque des revendications 1 à 17 et de mélanges de celui-ci, pour la fabrication d'un médicament destiné au traitement de maladies inflammatoires.
